# EUROPEAN PATENT APPLICATION

(11) **EP 3 293 260 A1**
(43) Date of publication of application: **14.03.2018**
(21) Application number: 16306150.0
(22) Date of filing: 12.09.2016
(51) Int. Cl.: C12N 9/26

(54) **ACID-ALPHA GLUCOSIDASE VARIANTS AND USES THEREOF**

(71) Applicant: GENETHON, 91000 Evry (FR); INSERM (Institut National de la Santé et de la Recherche Médicale), 75013 Paris (FR); Universite d'Evry Val d'Essonne, 91000 Evry (FR)
(72) Inventor: MINGOZZI, Federico, 75011 PARIS (FR); RONZITTI, Giuseppe, 75011 PARIS (FR)
(74) Representative: Cabinet Becker et Associés

(57) **Abstract**

The present invention relates to variants of acid-alpha glucosidase and uses thereof.

SEQUENCE LISTING

<110>  GENETHON et al.

<120>  ACID-ALPHA GLUCOSIDASE VARIANTS AND USES THEREOF

<130>  B2299EP00

<160>  33

<170>  PatentIn version 3.3

<210>  1
<211>  925
<212>  PRT
<213>  hGAAwt w/o sp

<400>  1

Gly His Ile Leu Leu His Asp Phe Leu Leu Val Pro Arg Glu Leu Ser
1               5                   10                  15

Gly Ser Ser Pro Val Leu Glu Glu Thr His Pro Ala His Gln Gln Gly
            20                  25                  30

Ala Ser Arg Pro Gly Pro Arg Asp Ala Gln Ala His Pro Gly Arg Pro
        35                  40                  45

Arg Ala Val Pro Thr Gln Cys Asp Val Pro Pro Asn Ser Arg Phe Asp
    50                  55                  60

Cys Ala Pro Asp Lys Ala Ile Thr Gln Glu Gln Cys Glu Ala Arg Gly
65                  70                  75                  80

Cys Cys Tyr Ile Pro Ala Lys Gln Gly Leu Gln Gly Ala Gln Met Gly
                85                  90                  95

Gln Pro Trp Cys Phe Phe Pro Pro Ser Tyr Pro Ser Tyr Lys Leu Glu
            100                 105                 110

Asn Leu Ser Ser Ser Glu Met Gly Tyr Thr Ala Thr Leu Thr Arg Thr
        115                 120                 125

Thr Pro Thr Phe Phe Pro Lys Asp Ile Leu Thr Leu Arg Leu Asp Val
    130                 135                 140

Met Met Glu Thr Glu Asn Arg Leu His Phe Thr Ile Lys Asp Pro Ala
145                 150                 155                 160

Asn Arg Arg Tyr Glu Val Pro Leu Glu Thr Pro His Val His Ser Arg
                165                 170                 175

Ala Pro Ser Pro Leu Tyr Ser Val Glu Phe Ser Glu Glu Pro Phe Gly
            180                 185                 190

Val Ile Val Arg Arg Gln Leu Asp Gly Arg Val Leu Leu Asn Thr Thr
        195                 200                 205

Val Ala Pro Leu Phe Phe Ala Asp Gln Phe Leu Gln Leu Ser Thr Ser
    210                 215                 220

Leu Pro Ser Gln Tyr Ile Thr Gly Leu Ala Glu His Leu Ser Pro Leu
225                 230                 235                 240

Met Leu Ser Thr Ser Trp Thr Arg Ile Thr Leu Trp Asn Arg Asp Leu
                245                 250                 255

Ala Pro Thr Pro Gly Ala Asn Leu Tyr Gly Ser His Pro Phe Tyr Leu
            260                 265                 270

Ala Leu Glu Asp Gly Gly Ser Ala His Gly Val Phe Leu Leu Asn Ser
        275                 280                 285

Asn Ala Met Asp Val Val Leu Gln Pro Ser Pro Ala Leu Ser Trp Arg
    290                 295                 300

Ser Thr Gly Gly Ile Leu Asp Val Tyr Ile Phe Leu Gly Pro Glu Pro
305                 310                 315                 320

Lys Ser Val Val Gln Gln Tyr Leu Asp Val Val Gly Tyr Pro Phe Met
                325                 330                 335

Pro Pro Tyr Trp Gly Leu Gly Phe His Leu Cys Arg Trp Gly Tyr Ser
            340                 345                 350

Ser Thr Ala Ile Thr Arg Gln Val Val Glu Asn Met Thr Arg Ala His
        355                 360                 365

Phe Pro Leu Asp Val Gln Trp Asn Asp Leu Asp Tyr Met Asp Ser Arg
    370                 375                 380

Arg Asp Phe Thr Phe Asn Lys Asp Gly Phe Arg Asp Phe Pro Ala Met
385                 390                 395                 400

Val Gln Glu Leu His Gln Gly Gly Arg Arg Tyr Met Met Ile Val Asp
                405                 410                 415

Pro Ala Ile Ser Ser Ser Gly Pro Ala Gly Ser Tyr Arg Pro Tyr Asp
            420                 425                 430

Glu Gly Leu Arg Arg Gly Val Phe Ile Thr Asn Glu Thr Gly Gln Pro
        435                 440                 445

Leu Ile Gly Lys Val Trp Pro Gly Ser Thr Ala Phe Pro Asp Phe Thr
    450                 455                 460

Asn Pro Thr Ala Leu Ala Trp Trp Glu Asp Met Val Ala Glu Phe His
465                 470                 475                 480

Asp Gln Val Pro Phe Asp Gly Met Trp Ile Asp Met Asn Glu Pro Ser
                485                 490                 495

Asn Phe Ile Arg Gly Ser Glu Asp Gly Cys Pro Asn Asn Glu Leu Glu
            500                 505                 510

Asn Pro Pro Tyr Val Pro Gly Val Val Gly Gly Thr Leu Gln Ala Ala
        515                 520                 525

Thr Ile Cys Ala Ser Ser His Gln Phe Leu Ser Thr His Tyr Asn Leu
    530                 535                 540

His Asn Leu Tyr Gly Leu Thr Glu Ala Ile Ala Ser His Arg Ala Leu
545                 550                 555                 560

Val Lys Ala Arg Gly Thr Arg Pro Phe Val Ile Ser Arg Ser Thr Phe
                565                 570                 575

Ala Gly His Gly Arg Tyr Ala Gly His Trp Thr Gly Asp Val Trp Ser
            580                 585                 590

Ser Trp Glu Gln Leu Ala Ser Ser Val Pro Glu Ile Leu Gln Phe Asn
        595                 600                 605

Leu Leu Gly Val Pro Leu Val Gly Ala Asp Val Cys Gly Phe Leu Gly
    610                 615                 620

Asn Thr Ser Glu Glu Leu Cys Val Arg Trp Thr Gln Leu Gly Ala Phe
625                 630                 635                 640

Tyr Pro Phe Met Arg Asn His Asn Ser Leu Leu Ser Leu Pro Gln Glu
                645                 650                 655

Pro Tyr Ser Phe Ser Glu Pro Ala Gln Gln Ala Met Arg Lys Ala Leu
            660                 665                 670

Thr Leu Arg Tyr Ala Leu Leu Pro His Leu Tyr Thr Leu Phe His Gln
        675                 680                 685

Ala His Val Ala Gly Glu Thr Val Ala Arg Pro Leu Phe Leu Glu Phe
    690                 695                 700

Pro Lys Asp Ser Ser Thr Trp Thr Val Asp His Gln Leu Leu Trp Gly
705                 710                 715                 720

Glu Ala Leu Leu Ile Thr Pro Val Leu Gln Ala Gly Lys Ala Glu Val
                725                 730                 735

Thr Gly Tyr Phe Pro Leu Gly Thr Trp Tyr Asp Leu Gln Thr Val Pro
            740                 745                 750

Val Glu Ala Leu Gly Ser Leu Pro Pro Pro Pro Ala Ala Pro Arg Glu
        755                 760                 765

Pro Ala Ile His Ser Glu Gly Gln Trp Val Thr Leu Pro Ala Pro Leu
    770                 775                 780

Asp Thr Ile Asn Val His Leu Arg Ala Gly Tyr Ile Ile Pro Leu Gln
785                 790                 795                 800

Gly Pro Gly Leu Thr Thr Thr Glu Ser Arg Gln Gln Pro Met Ala Leu
                805                 810                 815

Ala Val Ala Leu Thr Lys Gly Gly Glu Ala Arg Gly Glu Leu Phe Trp
            820                 825                 830

Asp Asp Gly Glu Ser Leu Glu Val Leu Glu Arg Gly Ala Tyr Thr Gln
        835                 840                 845

Val Ile Phe Leu Ala Arg Asn Asn Thr Ile Val Asn Glu Leu Val Arg
    850                 855                 860

Val Thr Ser Glu Gly Ala Gly Leu Gln Leu Gln Lys Val Thr Val Leu
865                 870                 875                 880

Gly Val Ala Thr Ala Pro Gln Gln Val Leu Ser Asn Gly Val Pro Val
                885                 890                 895

Ser Asn Phe Thr Tyr Ser Pro Asp Thr Lys Val Leu Asp Ile Cys Val
            900                 905                 910

Ser Leu Leu Met Gly Glu Gln Phe Leu Val Ser Trp Cys
        915                 920                 925

<210>  2

<211>  952
<212>  PRT
<213>  homo sapiens

<400>  2

<210>  3
<211>  18
<212>  PRT
<213>  artificial

<220>

<223>  sp7

<400>  3

Met Ala Phe Leu Trp Leu Leu Ser Cys Trp Ala Leu Leu Gly Thr Thr
1               5                   10                  15

Phe Gly

<210>  4
<211>  27
<212>  PRT
<213>  artificial

<220>
<223>  sp1

<400>  4

Met Gly Val Arg His Pro Pro Cys Ser His Arg Leu Leu Ala Val Cys
1               5                   10                  15

Ala Leu Val Ser Leu Ala Thr Ala Ala Leu Leu
            20                  25

<210>  5
<211>  24
<212>  PRT
<213>  artificial

<220>
<223>  sp2

<400>  5

Met Pro Ser Ser Val Ser Trp Gly Ile Leu Leu Leu Ala Gly Leu Cys
1               5                   10                  15

Cys Leu Val Pro Val Ser Leu Ala
            20

<210>  6
<211>  25
<212>  PRT
<213>  artificial

<220>
<223>  sp6

<400>  6

Met Pro Pro Pro Arg Thr Gly Arg Gly Leu Leu Trp Leu Gly Leu Val
1               5                   10                  15

Leu Ser Ser Val Cys Val Ala Leu Gly

20                  25

<210>  7
<211>  22
<212>  PRT
<213>  artificial

<220>
<223>  sp8

<400>  7

Met Ala Ser Arg Leu Thr Leu Leu Thr Leu Leu Leu Leu Leu Leu Ala
1               5                   10                  15

Gly Asp Arg Ala Ser Ser
            20

<210>  8
<211>  2859
<212>  DNA
<213>  homo sapiens

<400>  8
atgggagtga ggcacccgcc ctgctcccac cggctcctgg ccgtctgcgc cctcgtgtcc      60

ttggcaaccg cagcgctcct ggggcacatc ctactccatg atttcctgct ggttccccga     120

gagctgagtg gctcctcccc agtcctggag gagactcacc cagctcacca gcagggagcc     180

agcagaccag ggccccggga tgcccaggca caccccgggc ggccgcgagc agtgcccaca     240

cagtgcgacg tcccccccaa cagccgcttc gattgcgccc ctgacaaggc catcacccag     300

gaacagtgcg aggcccgcgg ctgttgctac atccctgcaa agcaggggct gcagggagcc     360

cagatggggc agccctggtg cttcttccca cccagctacc ccagctacaa gctggagaac     420

ctgagctcct ctgaaatggg ctacacggcc accctgaccc gtaccacccc caccttcttc     480

cccaaggaca tcctgaccct gcggctggac gtgatgatgg agactgagaa ccgcctccac     540

ttcacgatca aagatccagc taacaggcgc tacgaggtgc ccttggagac cccgcatgtc     600

cacagccggg caccgtcccc actctacagc gtggagttct ccgaggagcc cttcggggtg     660

atcgtgcgcc ggcagctgga cggccgcgtg ctgctgaaca cgacggtggc gcccctgttc     720

tttgcggacc agttccttca gctgtccacc tcgctgccct cgcagtatat cacaggcctc     780

gccgagcacc tcagtcccct gatgctcagc accagctgga ccaggatcac cctgtggaac     840

cgggaccttg cgcccacgcc cggtgcgaac ctctacgggt ctcacccttt ctacctggcg     900

ctggaggacg gcgggtcggc acacggggtg ttcctgctaa acagcaatgc catggatgtg     960

gtcctgcagc cgagccctgc ccttagctgg aggtcgacag gtgggatcct ggatgtctac    1020

atcttcctgg gcccagagcc caagagcgtg gtgcagcagt acctggacgt tgtgggatac    1080

ccgttcatgc cgccatactg gggcctgggc ttccacctgt gccgctgggg ctactcctcc 1140

accgctatca cccgccaggt ggtggagaac atgaccaggg cccacttccc cctggacgtc 1200

cagtggaacg acctggacta catggactcc cggagggact tcacgttcaa caaggatggc 1260

ttccgggact tcccggccat ggtgcaggag ctgcaccagg gcggccggcg ctacatgatg 1320

atcgtggatc ctgccatcag cagctcgggc cctgccggga gctacaggcc ctacgacgag 1380

ggtctgcgga gggggttttt catcaccaac gagaccggcc agccgctgat tgggaaggta 1440

tggcccgggt ccactgcctt ccccgacttc accaacccca cagccctggc ctggtgggag 1500

gacatggtgg ctgagttcca tgaccaggtg cccttcgacg gcatgtggat tgacatgaac 1560

gagccttcca acttcatcag gggctctgag gacggctgcc ccaacaatga gctggagaac 1620

ccaccctacg tgcctggggt ggttgggggg accctccagg cggccaccat ctgtgcctcc 1680

agccaccagt ttctctccac acactacaac ctgcacaacc tctacggcct gaccgaagcc 1740

atcgcctccc acagggcgct ggtgaaggct cgggggacac gcccatttgt gatctcccgc 1800

tcgacctttg ctggccacgg ccgatacgcc ggccactgga cgggggacgt gtggagctcc 1860

tgggagcagc tcgcctcctc cgtgccagaa atcctgcagt ttaacctgct gggggtgcct 1920

ctggtcgggg ccgacgtctg cggcttcctg ggcaacacct cagaggagct gtgtgtgcgc 1980

tggacccagc tgggggcctt ctaccccttc atgcggaacc acaacagcct gctcagtctg 2040

ccccaggagc cgtacagctt cagcgagccg gcccagcagg ccatgaggaa ggccctcacc 2100

ctgcgctacg cactcctccc ccacctctac acactgttcc accaggccca cgtcgcgggg 2160

gagaccgtgg cccggcccct cttcctggag ttccccaagg actctagcac ctggactgtg 2220

gaccaccagc tcctgtgggg ggaggccctg ctcatcaccc cagtgctcca ggccgggaag 2280

gccgaagtga ctggctactt ccccttgggc acatggtacg acctgcagac ggtgccagta 2340

gaggcccttg gcagcctccc acccccacct gcagctcccc gtgagccagc catccacagc 2400

gaggggcagt gggtgacgct gccggccccc ctggacacca tcaacgtcca cctccgggct 2460

gggtacatca tccccctgca gggccctggc ctcacaacca cagagtcccg ccagcagccc 2520

atggccctgg ctgtggccct gaccaagggt ggggaggccc gaggggagct gttctgggac 2580

gatggagaga gcctggaagt gctggagcga ggggcctaca cacaggtcat cttcctggcc 2640

aggaataaca cgatcgtgaa tgagctggta cgtgtgacca gtgagggagc tggcctgcag 2700

ctgcagaagg tgactgtcct gggcgtggcc acggcgcccc agcaggtcct ctccaacggt 2760

gtccctgtct ccaacttcac ctacagcccc gacaccaagg tcctggacat ctgtgtctcg 2820

ctgttgatgg gagagcagtt tctcgtcagc tggtgttag 2859

<210> 9
<211> 2778

<212>  DNA
<213>  artificial

<220>
<223>  hGAAwt w/o sp

<400>  9
gggcacatcc tactccatga tttcctgctg gttccccgag agctgagtgg ctcctcccca      60

gtcctggagg agactcaccc agctcaccag cagggagcca gcagaccagg gccccgggat     120

gcccaggcac accccgggcg gccgcgagca gtgcccacac agtgcgacgt cccccccaac     180

agccgcttcg attgcgcccc tgacaaggcc atcacccagg aacagtgcga ggcccgcggc     240

tgttgctaca tccctgcaaa gcaggggctg cagggagccc agatggggca gccctggtgc     300

ttcttcccac ccagctaccc cagctacaag ctggagaacc tgagctcctc tgaaatgggc     360

tacacggcca ccctgacccg taccaccccc accttcttcc ccaaggacat cctgaccctg     420

cggctggacg tgatgatgga gactgagaac cgcctccact tcacgatcaa agatccagct     480

aacaggcgct acgaggtgcc cttggagacc ccgcatgtcc acagccgggc accgtcccca     540

ctctacagcg tggagttctc cgaggagccc ttcggggtga tcgtgcgccg gcagctggac     600

ggccgcgtgc tgctgaacac gacggtggcg cccctgttct ttgcggacca gttccttcag     660

ctgtccacct cgctgccctc gcagtatatc acaggcctcg ccgagcacct cagtcccctg     720

atgctcagca ccagctggac caggatcacc ctgtggaacc gggaccttgc gcccacgccc     780

ggtgcgaacc tctacgggtc tcaccctttc tacctggcgc tggaggacgg cgggtcggca     840

cacggggtgt tcctgctaaa cagcaatgcc atggatgtgg tcctgcagcc gagccctgcc     900

cttagctgga ggtcgacagg tgggatcctg gatgtctaca tcttcctggg cccagagccc     960

aagagcgtgg tgcagcagta cctggacgtt gtgggatacc cgttcatgcc gccatactgg    1020

ggcctgggct tccacctgtg ccgctggggc tactcctcca ccgctatcac ccgccaggtg    1080

gtggagaaca tgaccagggc ccacttcccc ctggacgtcc agtggaacga cctggactac    1140

atggactccc ggagggactt cacgttcaac aaggatggct tccgggactt ccccgccatg    1200

gtgcaggagc tgcaccaggg cggccggcgc tacatgatga tcgtggatcc tgccatcagc    1260

agctcgggcc ctgccgggag ctacaggccc tacgacgagg gtctgcggag gggggttttc    1320

atcaccaacg agaccggcca gccgctgatt gggaaggtat ggcccgggtc cactgccttc    1380

cccgacttca ccaaccccac agccctggcc tggtgggagg acatggtggc tgagttccat    1440

gaccaggtgc ccttcgacgg catgtggatt gacatgaacg agccttccaa cttcatcagg    1500

ggctctgagg acggctgccc caacaatgag ctggagaacc caccctacgt gcctggggtg    1560

gttgggggga ccctccaggc ggccaccatc tgtgcctcca gccaccagtt tctctccaca    1620

cactacaacc tgcacaacct ctacggcctg accgaagcca tcgcctccca cagggcgctg    1680

gtgaaggctc gggggacacg cccatttgtg atctcccgct cgacctttgc tggccacggc 1740

cgatacgccg gccactggac gggggacgtg tggagctcct gggagcagct cgcctcctcc 1800

gtgccagaaa tcctgcagtt taacctgctg ggggtgcctc tggtcggggc cgacgtctgc 1860

ggcttcctgg gcaacacctc agaggagctg tgtgtgcgct ggacccagct gggggccttc 1920

taccccttca tgcggaacca caacagcctg ctcagtctgc cccaggagcc gtacagcttc 1980

agcgagccgg cccagcaggc catgaggaag gccctcaccc tgcgctacgc actcctcccc 2040

cacctctaca cactgttcca ccaggcccac gtcgcggggg agaccgtggc ccggcccctc 2100

ttcctggagt tccccaagga ctctagcacc tggactgtgg accaccagct cctgtggggg 2160

gaggccctgc tcatcacccc agtgctccag gccgggaagg ccgaagtgac tggctacttc 2220

cccttgggca catggtacga cctgcagacg gtgccagtag aggcccttgg cagcctccca 2280

cccccacctg cagctccccg tgagccagcc atccacagcg aggggcagtg ggtgacgctg 2340

ccggcccccc tggacaccat caacgtccac ctccgggctg ggtacatcat ccccctgcag 2400

ggccctggcc tcacaaccac agagtcccgc cagcagccca tggccctggc tgtggccctg 2460

accaagggtg gggaggcccg aggggagctg ttctgggacg atggagagag cctggaagtg 2520

ctggagcgag gggcctacac acaggtcatc ttcctggcca ggaataacac gatcgtgaat 2580

gagctggtac gtgtgaccag tgagggagct ggcctgcagc tgcagaaggt gactgtcctg 2640

ggcgtggcca cggcgcccca gcaggtcctc tccaacggtg tccctgtctc caacttcacc 2700

tacagccccg acaccaaggt cctggacatc tgtgtctcgc tgttgatggg agagcagttt 2760

ctcgtcagct ggtgttag 2778

<210> 10
<211> 2778
<212> DNA
<213> artificial

<220>
<223> hGAAco1 w/o sp

<400> 10
   ggccatatcc tgctgcacga ctttctacta gtgcccagag agctgagcgg cagctctccc 60
   gtgctggaag aaacacaccc tgcccatcag cagggcgcct ctagacctgg acctagagat 120
   gcccaggccc accccggcag acctagagct gtgcctaccc agtgtgacgt gccccccaac 180
   agcagattcg actgcgcccc tgacaaggcc atcacccagg aacagtgcga ggccagaggc 240
   tgctgctaca tccctgccaa gcagggactg cagggcgctc agatgggaca gccctggtgc 300
   ttcttcccac cctcctaccc cagctacaag ctggaaaacc tgagcagcag cgagatgggc 360
   tacaccgcca ccctgaccag aaccaccccc acattcttcc caaaggacat cctgaccctg 420
   cggctggacg tgatgatgga aaccgagaac cggctgcact tcaccatcaa ggaccccgcc 480
   aatcggagat acgaggtgcc cctggaaacc ccccacgtgc actctagagc ccccagccct 540
   ctgtacagcg tggaattcag cgaggaaccc ttcggcgtga tcgtgcggag acagctggat 600
   ggcagagtgc tgctgaacac caccgtggcc cctctgttct tcgccgacca gttcctgcag 660
   ctgagcacca gcctgcccag ccagtacatc acaggactgg ccgagcacct gagccccctg 720
   atgctgagca catcctggac ccggatcacc ctgtggaaca gggatctggc ccctacccct 780
   ggcgccaatc tgtacggcag ccaccctttc tacctggccc tggaagatgg cggatctgcc 840
   cacggagtgt ttctgctgaa ctccaacgcc atggacgtgg tgctgcagcc tagccctgcc 900
   ctgtcttgga gaagcacagg cggcatcctg gatgtgtaca tctttctggg ccccgagccc 960
   aagagcgtgg tgcagcagta tctggatgtc gtgggctacc ccttcatgcc cccttactgg 1020
   ggcctgggat tccacctgtg cagatggggc tactccagca ccgccatcac cagacaggtg 1080
   gtggaaaaca tgaccagagc ccacttccca ctggatgtgc agtggaacga cctggactac 1140
   atggacagca gacgggactt caccttcaac aaggacggct tccgggactt ccccgccatg 1200
   gtgcaggaac tgcatcaggg cggcagacgg tacatgatga tcgtggatcc cgccatcagc 1260
   tcctctggcc ctgccggctc ttacagaccc tacgacgagg gcctgcggag aggcgtgttc 1320
   atcaccaacg agacaggcca gcccctgatc ggcaaagtgt ggcctggcag cacagccttc 1380
   cccgacttca ccaatcctac cgccctggct tggtgggagg acatggtggc cgagttccac 1440
   gaccaggtgc ccttcgacgg catgtggatc gacatgaacg agcccagcaa cttcatccgg 1500
   ggcagcgagg atggctgccc caacaacgaa ctggaaaatc ccccttacgt gcccggcgtc 1560
   gtgggcggaa cactgcaggc cgctacaatc tgtgccagca gccaccagtt tctgagcacc 1620
   cactacaacc tgcacaacct gtacggcctg accgaggcca ttgccagcca ccgcgctctc 1680
   gtgaaagcca gaggcacacg gcccttcgtg atcagcagaa gcacctttgc cggccacggc 1740
   agatacgccg gacattggac tggcgacgtg tggtcctctt gggagcagct ggcctctagc 1800
   gtgcccgaga tcctgcagtt caatctgctg ggcgtgccac tcgtgggcgc cgatgtgtgt 1860
   ggcttcctgg gcaacacctc cgaggaactg tgtgtgcggt ggacacagct gggcgccttc 1920
   taccctttca tgagaaacca caacagcctg ctgagcctgc cccaggaacc ctacagcttt 1980
   agcgagcctg cacagcaggc catgcggaag gccctgacac tgagatacgc tctgctgccc 2040
   cacctgtaca ccctgtttca ccaggcccat gtggccggcg agacagtggc cagacctctg 2100
   tttctggaat tccccaagga cagcagcacc tggaccgtgg accatcagct gctgtgggga 2160
   gaggctctgc tgattacccc agtgctgcag gcaggcaagg ccgaagtgac cggctacttt 2220
   cccctgggca cttggtacga cctgcagacc gtgcctgtgg aagccctggg atctctgcct 2280
   ccacctcctg ccgctcctag agagcctgcc attcactctg agggccagtg ggtcacactg 2340
   cctgcccccc tggataccat caacgtgcac ctgagggccg gctacatcat accactgcag 2400
   ggacctggcc tgaccaccac cgagtctaga cagcagccaa tggccctggc cgtggccctg 2460
   accaaaggcg gagaagctag gggcgagctg ttctgggacg atggcgagag cctggaagtg 2520
   ctggaaagag gcgcctatac ccaagtgatc ttcctggccc ggaacaacac catcgtgaac 2580
   gagctggtgc gcgtgacctc tgaaggcgct ggactgcagc tgcagaaagt gaccgtgctg 2640
   ggagtggcca cagcccctca gcaggtgctg tctaatggcg tgcccgtgtc caacttcacc 2700
   tacagccccg acaccaaggt gctggacatc tgcgtgtcac tgctgatggg agagcagttt 2760
   ctggtgtcct ggtgctga 2778

<210> 11
<211> 2778
<212> DNA
<213> artificial

<220>
<223> hGAAco2 w/o sp

<400> 11
ggacacatcc tgctgcacga cttcctgttg gtgcctagag agctgagcgg atcatcccca 60

gtgctggagg agactcatcc tgctcaccaa cagggagctt ccagaccagg accgagagac 120

gcccaagccc atcctggtag accaagagct gtgcctaccc aatgcgacgt gccacccaac 180

tcccgattcg actgcgcgcc agataaggct attacccaag agcagtgtga agccagaggt 240

tgctgctaca tcccagcgaa gcaaggattg caaggcgccc aaatgggaca accttggtgt 300

ttcttccccc cttcgtaccc atcatataaa ctcgaaaacc tgtcctcttc ggaaatgggt 360

tatactgcca ccctcaccag aactactcct actttcttcc cgaaagacat cttgaccttg 420

aggctggacg tgatgatgga gactgaaaac cggctgcatt tcactatcaa agatcctgcc 480

aatcggcgat acgaggtccc tctggaaacc cctcacgtgc actcacgggc tccttctccg 540

ctttactccg tcgaattctc tgaggaaccc ttcggagtga tcgttagacg ccagctggat 600

ggtagagtgc tgttgaacac tactgtggcc ccacttttct tcgctgacca gtttctgcaa 660

ctgtccactt ccctgccatc ccagtacatt actggactcg ccgaacacct gtcgccactg 720

atgctctcga cctcttggac tagaatcact ttgtggaaca gagacttggc ccctactccg 780

ggagcaaatc tgtacggaag ccaccctttt tacctggcgc tcgaagatgg cggatccgct 840

cacggagtgt tcctgctgaa tagcaacgca atggacgtgg tgctgcaacc ttcccctgca 900

ctcagttgga gaagtaccgg gggtattctg gacgtgtaca tcttcctcgg accagaaccc 960

aagagcgtgg tgcagcaata tctggacgtg gtcggatacc cttttatgcc tccttactgg 1020

ggactgggat tccacctttg ccgttggggc tactcatcca ccgccattac cagacaggtg 1080

gtggagaata tgaccagagc ccacttccct ctcgacgtgc agtggaacga tctggactat 1140

atggactccc ggagagattt caccttcaac aaggacgggt tccgcgattt tcccgcgatg 1200

gttcaagagc tccaccaggg tggtcgaaga tatatgatga tcgtcgaccc agccatttcg 1260

agcagcggac ccgctggatc ttatagacct tacgacgaag gccttaggag aggagtgttc 1320

atcacaaacg agactggaca gcctttgatc ggtaaagtgt ggcctggatc aaccgccttt 1380

cctgacttta ccaatcccac tgccttggct tggtgggagg acatggtggc cgaattccac 1440

gaccaagtcc cctttgatgg aatgtggatc gatatgaacg aaccaagcaa ttttatcaga 1500

ggttccgaag acggttgccc caacaacgaa ctggaaaacc ctccttatgt gcccggagtc 1560

gtgggcggaa cattacaggc cgcgactatt tgcgccagca gccaccaatt cctgtccact 1620

cactacaacc tccacaacct ttatggatta accgaagcta ttgcaagtca cagggctctg 1680

gtgaaggcta gagggactag gccctttgtg atctcccgat ccacctttgc cggacacggg 1740

agatacgccg gtcactggac tggtgacgtg tggagctcat gggaacaact ggcctcctcc 1800

gtgccggaaa tcttacagtt caaccttctg ggtgtccctc ttgtcggagc agacgtgtgt 1860

gggtttcttg gtaacacctc cgaggaactg tgtgtgcgct ggactcaact gggtgcattc 1920

tacccattca tgagaaacca caactccttg ctgtccctgc cacaagagcc ctactcgttc 1980

agcgagcctg cacaacaggc tatgcggaag gcactgaccc tgagatacgc cctgcttcca 2040

cacttataca ctctcttcca tcaagcgcat gtggcaggag aaaccgttgc aaggcctctt 2100

ttccttgaat tccccaagga ttcctcgact tggacggtgg atcatcagct gctgtgggga 2160

gaagctctgc tgattactcc agtgttgcaa gccggaaaag ctgaggtgac cggatacttt 2220

ccgctgggaa cctggtacga cctccagact gtccctgttg aagcccttgg atcactgcct 2280

ccgcctccgg cagctccacg cgaaccagct atacattccg agggacagtg ggttacatta 2340

ccagctcctc tggacacaat caacgtccac ttaagagctg gctacattat ccctctgcaa 2400

ggaccaggac tgactacgac cgagagcaga cagcagccaa tggcactggc tgtggctctg 2460

accaagggag gggaagctag aggagaactc ttctgggatg atggggagtc ccttgaagtg 2520

ctggaaagag gcgcttacac tcaagtcatt ttccttgcac ggaacaacac cattgtgaac 2580

gaattggtgc gagtgaccag cgaaggagct ggacttcaac tgcagaaggt cactgtgctc 2640

ggagtggcta ccgctcctca gcaagtgctg tcgaatggag tccccgtgtc aaactttacc 2700

tactcccctg acactaaggt gctcgacatt tgcgtgtccc tcctgatggg agagcagttc 2760

cttgtgtcct ggtgttga 2778

<210> 12
<211> 2754
<212> DNA
<213> artificial

<220>
<223>  hGAAco1-delta-8 w/o sp

<400>  12
ctactagtgc ccagagagct gagcggcagc tctcccgtgc tggaagaaac acaccctgcc      60

catcagcagg gcgcctctag acctggacct agagatgccc aggcccaccc cggcagacct     120

agagctgtgc ctacccagtg tgacgtgccc cccaacagca gattcgactg cgcccctgac     180

aaggccatca cccaggaaca gtgcgaggcc agaggctgct gctacatccc tgccaagcag     240

ggactgcagg gcgctcagat gggacagccc tggtgcttct tcccaccctc ctaccccagc     300

tacaagctgg aaaacctgag cagcagcgag atgggctaca ccgccaccct gaccagaacc     360

acccccacat tcttcccaaa ggacatcctg accctgcggc tggacgtgat gatggaaacc     420

gagaaccggc tgcacttcac catcaaggac cccgccaatc ggagatacga ggtgcccctg     480

gaaacccccc acgtgcactc tagagccccc agccctctgt acagcgtgga attcagcgag     540

gaacccttcg gcgtgatcgt gcggagacag ctggatggca gagtgctgct gaacaccacc     600

gtggcccctc tgttcttcgc cgaccagttc ctgcagctga gcaccagcct gcccagccag     660

tacatcacag gactggccga gcacctgagc cccctgatgc tgagcacatc ctggacccgg     720

atcaccctgt ggaacaggga tctggcccct acccctggcg ccaatctgta cggcagccac     780

cctttctacc tggccctgga agatggcgga tctgcccacg gagtgtttct gctgaactcc     840

aacgccatgg acgtggtgct gcagcctagc cctgccctgt cttggagaag cacaggcggc     900

atcctggatg tgtacatctt tctgggcccc gagcccaaga gcgtggtgca gcagtatctg     960

gatgtcgtgg gctacccctt catgccccct tactggggcc tgggattcca cctgtgcaga    1020

tggggctact ccagcaccgc catcaccaga caggtggtgg aaaacatgac cagagcccac    1080

ttcccactgg atgtgcagtg gaacgacctg gactacatgg acagcagacg ggacttcacc    1140

ttcaacaagg acggcttccg ggacttcccc gccatggtgc aggaactgca tcagggcggc    1200

agacggtaca tgatgatcgt ggatcccgcc atcagctcct ctggccctgc cggctcttac    1260

agaccctacg acgagggcct gcggagaggc gtgttcatca ccaacgagac aggccagccc    1320

ctgatcggca aagtgtggcc tggcagcaca gccttccccg acttcaccaa tcctaccgcc    1380

ctggcttggt gggaggacat ggtggccgag ttccacgacc aggtgccctt cgacggcatg    1440

tggatcgaca tgaacgagcc cagcaacttc atccggggca gcgaggatgg ctgccccaac    1500

aacgaactgg aaaatccccc ttacgtgccc ggcgtcgtgg gcggaacact gcaggccgct    1560

acaatctgtg ccagcagcca ccagtttctg agcacccact acaacctgca caacctgtac    1620

ggcctgaccg aggccattgc cagccaccgc gctctcgtga aagccagagg cacacggccc    1680

ttcgtgatca gcagaagcac ctttgccggc cacggcagat acgccggaca ttggactggc    1740

gacgtgtggt cctcttggga gcagctggcc tctagcgtgc ccgagatcct gcagttcaat    1800

ctgctgggcg tgccactcgt gggcgccgat gtgtgtggct tcctgggcaa cacctccgag 1860

gaactgtgtg tgcggtggac acagctgggc gccttctacc ctttcatgag aaaccacaac 1920

agcctgctga gcctgcccca ggaaccctac agctttagcg agcctgcaca gcaggccatg 1980

cggaaggccc tgacactgag atacgctctg ctgccccacc tgtacaccct gtttcaccag 2040

gcccatgtgg ccggcgagac agtggccaga cctctgtttc tggaattccc caaggacagc 2100

agcacctgga ccgtggacca tcagctgctg tggggagagg ctctgctgat taccccagtg 2160

ctgcaggcag gcaaggccga agtgaccggc tactttcccc tgggcacttg gtacgacctg 2220

cagaccgtgc ctgtggaagc cctgggatct ctgcctccac ctcctgccgc tcctagagag 2280

cctgccattc actctgaggg ccagtgggtc acactgcctg cccccctgga taccatcaac 2340

gtgcacctga gggccggcta catcatacca ctgcagggac ctggcctgac caccaccgag 2400

tctagacagc agccaatggc cctggccgtg gccctgacca aaggcggaga agctaggggc 2460

gagctgttct gggacgatgg cgagagcctg gaagtgctgg aaagaggcgc ctatacccaa 2520

gtgatcttcc tggcccggaa caacaccatc gtgaacgagc tggtgcgcgt gacctctgaa 2580

ggcgctggac tgcagctgca gaaagtgacc gtgctgggag tggccacagc ccctcagcag 2640

gtgctgtcta atggcgtgcc cgtgtccaac ttcacctaca gccccgacac caaggtgctg 2700

gacatctgcg tgtcactgct gatgggagag cagtttctgg tgtcctggtg ctga 2754

<210> 13
<211> 2754
<212> DNA
<213> artificial

<220>
<223> hGAAco2-delta8 w/o sp

<400> 13
ctgttggtgc ctagagagct gagcggatca tccccagtgc tggaggagac tcatcctgct 60

caccaacagg gagcttccag accaggaccg agagacgccc aagcccatcc tggtagacca 120

agagctgtgc ctacccaatg cgacgtgcca cccaactccc gattcgactg cgcgccagat 180

aaggctatta cccaagagca gtgtgaagcc agaggttgct gctacatccc agcgaagcaa 240

ggattgcaag gcgcccaaat gggacaacct tggtgtttct tccccccttc gtacccatca 300

tataaactcg aaaacctgtc ctcttcggaa atgggttata ctgccaccct caccagaact 360

actcctactt tcttcccgaa agacatcttg accttgaggc tggacgtgat gatggagact 420

gaaaaccggc tgcatttcac tatcaaagat cctgccaatc ggcgatacga ggtccctctg 480

gaaacccctc acgtgcactc acgggctcct tctccgcttt actccgtcga attctctgag 540

gaacccttcg gagtgatcgt tagacgccag ctggatggta gagtgctgtt gaacactact 600

gtggccccac ttttcttcgc tgaccagttt ctgcaactgt ccacttccct gccatcccag 660
tacattactg gactcgccga acacctgtcg ccactgatgc tctcgacctc ttggactaga 720
atcactttgt ggaacagaga cttggcccct actccgggag caaatctgta cggaagccac 780
cctttttacc tggcgctcga agatggcgga tccgctcacg gagtgttcct gctgaatagc 840
aacgcaatgg acgtggtgct gcaaccttcc cctgcactca gttggagaag taccgggggt 900
attctggacg tgtacatctt cctcggacca gaacccaaga gcgtggtgca gcaatatctg 960
gacgtggtcg gatacccttt tatgcctcct tactggggac tgggattcca cctttgccgt 1020
tggggctact catccaccgc cattaccaga caggtggtgg agaatatgac cagagcccac 1080
ttccctctcg acgtgcagtg gaacgatctg gactatatgg actcccggag agatttcacc 1140
ttcaacaagg acgggttccg cgattttccc gcgatggttc aagagctcca ccagggtggt 1200
cgaagatata tgatgatcgt cgacccagcc atttcgagca gcggacccgc tggatcttat 1260
agaccttacg acgaaggcct taggagagga gtgttcatca caaacgagac tggacagcct 1320
ttgatcggta aagtgtggcc tggatcaacc gcctttcctg actttaccaa tcccactgcc 1380
ttggcttggt gggaggacat ggtggccgaa ttccacgacc aagtcccctt tgatggaatg 1440
tggatcgata tgaacgaacc aagcaatttt atcagaggtt ccgaagacgg ttgccccaac 1500
aacgaactgg aaaaccctcc ttatgtgccc ggagtcgtgg gcggaacatt acaggccgcg 1560
actatttgcg ccagcagcca ccaattcctg tccactcact acaacctcca caacctttat 1620
ggattaaccg aagctattgc aagtcacagg gctctggtga aggctagagg gactaggccc 1680
tttgtgatct cccgatccac ctttgccgga cacgggagat acgccggtca ctggactggt 1740
gacgtgtgga gctcatggga acaactggcc tcctccgtgc cggaaatctt acagttcaac 1800
cttctgggtg tccctcttgt cggagcagac gtgtgtgggt ttcttggtaa cacctccgag 1860
gaactgtgtg tgcgctggac tcaactgggt gcattctacc cattcatgag aaaccacaac 1920
tccttgctgt ccctgccaca agagccctac tcgttcagcg agcctgcaca acaggctatg 1980
cggaaggcac tgaccctgag atacgccctg cttccacact tatacactct cttccatcaa 2040
gcgcatgtgg caggagaaac cgttgcaagg cctcttttcc ttgaattccc caaggattcc 2100
tcgacttgga cggtggatca tcagctgctg tggggagaag ctctgctgat tactccagtg 2160
ttgcaagccg gaaaagctga ggtgaccgga tactttccgc tgggaacctg gtacgacctc 2220
cagactgtcc ctgttgaagc ccttggatca ctgcctccgc ctccggcagc tccacgcgaa 2280
ccagctatac attccgaggg acagtgggtt acattaccag ctcctctgga cacaatcaac 2340
gtccacttaa gagctggcta cattatccct ctgcaaggac caggactgac tacgaccgag 2400
agcagacagc agccaatggc actggctgtg gctctgacca agggagggga agctagagga 2460
gaactcttct gggatgatgg ggagtccctt gaagtgctgg aaagaggcgc ttacactcaa 2520

gtcattttcc ttgcacggaa caacaccatt gtgaacgaat tggtgcgagt gaccagcgaa 2580

ggagctggac ttcaactgca gaaggtcact gtgctcggag tggctaccgc tcctcagcaa 2640

gtgctgtcga atggagtccc cgtgtcaaac tttacctact cccctgacac taaggtgctc 2700

gacatttgcg tgtccctcct gatgggagag cagttccttg tgtcctggtg ttga 2754

<210> 14
<211> 397
<212> DNA
<213> artificial

<220>
<223> hAAT promoter

<400> 14
gatcttgcta ccagtggaac agccactaag gattctgcag tgagagcaga gggccagcta 60

agtggtactc tcccagagac tgtctgactc acgccacccc ctccaccttg gacacaggac 120

gctgtggttt ctgagccagg tacaatgact cctttcggta agtgcagtgg aagctgtaca 180

ctgcccaggc aaagcgtccg ggcagcgtag gcgggcgact cagatcccag ccagtggact 240

tagcccctgt ttgctcctcc gataactggg gtgaccttgg ttaatattca ccagcagcct 300

cccccgttgc ccctctggat ccactgctta aatacggacg aggacagggc cctgtctcct 360

cagcttcagg caccaccact gacctgggac agtgaat 397

<210> 15
<211> 321
<212> DNA
<213> artificial

<220>
<223> ApoE control region

<400> 15
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg g 321

<210> 16
<211> 441
<212> DNA
<213> artificial

<220>
<223> HBB2 intron

<400>  16
gtacacatat tgaccaaatc agggtaattt tgcatttgta attttaaaaa atgctttctt      60

cttttaatat acttttttgt ttatcttatt tctaatactt tccctaatct ctttctttca     120

gggcaataat gatacaatgt atcatgcctc tttgcaccat tctaaagaat aacagtgata     180

atttctgggt taaggcaata gcaatatttc tgcatataaa tatttctgca tataaattgt     240

aactgatgta agaggtttca tattgctaat agcagctaca atccagctac cattctgctt     300

ttattttatg gttgggataa ggctggatta ttctgagtcc aagctaggcc cttttgctaa     360

tcatgttcat acctcttatc ttcctcccac agctcctggg caacgtgctg gtctgtgtgc     420

tggcccatca ctttggcaaa g                                                441

<210>  17
<211>  441
<212>  DNA
<213>  artificial

<220>
<223>  modified HBB2 intron

<400>  17
gtacacatat tgaccaaatc agggtaattt tgcatttgta attttaaaaa atgctttctt      60

cttttaatat acttttttgt ttatcttatt tctaatactt tccctaatct ctttctttca     120

gggcaataat gatacaatgt atcatgcctc tttgcaccat tctaaagaat aacagtgata     180

atttctgggt taaggcaata gcaatatttc tgcatataaa tatttctgca tataaattgt     240

aactgatgta agaggtttca tattgctaat agcagctaca atccagctac cattctgctt     300

ttattttctg gttgggataa ggctggatta ttctgagtcc aagctaggcc cttttgctaa     360

tcttgttcat acctcttatc ttcctcccac agctcctggg caacctgctg gtctctctgc     420

tggcccatca ctttggcaaa g                                                441

<210>  18
<211>  1438
<212>  DNA
<213>  artificial

<220>
<223>  FIX intron

<400>  18
ggtttgtttc cttttttaaa atacattgag tatgcttgcc ttttagatat agaaatatct      60

gatgctgtct tcttcactaa attttgatta catgatttga cagcaatatt gaagagtcta     120

acagccagca cgcaggttgg taagtactgg ttctttgtta gctaggtttt cttcttcttc     180

atttttaaaa ctaaatagat cgacaatgct tatgatgcat ttatgtttaa taaacactgt     240

tcagttcatg atttggtcat gtaattcctg ttagaaaaca ttcatctcct tggtttaaaa     300

aaattaaaag tgggaaaaca aagaaatagc agaatatagt gaaaaaaaat aaccacatta 360

tttttgtttg gacttaccac tttgaaatca aaatgggaaa caaaagcaca aacaatggcc 420

ttatttacac aaaaagtctg attttaagat atatgacatt tcaaggtttc agaagtatgt 480

aatgaggtgt gtctctaatt ttttaaatta tatatcttca atttaaagtt ttagttaaaa 540

cataaagatt aacctttcat tagcaagctg ttagttatca ccaacgcttt tcatggatta 600

ggaaaaaatc attttgtctc tatgtcaaac atcttggagt tgatatttgg ggaaacacaa 660

tactcagttg agttccctag gggagaaaag cacgcttaag aattgacata aagagtagga 720

agttagctaa tgcaacatat atcactttgt tttttcacaa ctacagtgac tttatgtatt 780

tcccagagga aggcatacag ggaagaaatt atcccatttg gacaaacagc atgttctcac 840

aggaagcatt tatcacactt acttgtcaac tttctagaat caaatctagt agctgacagt 900

accaggatca ggggtgccaa ccctaagcac ccccagaaag ctgactggcc ctgtggttcc 960

cactccagac atgatgtcag ctgtgaaatc gacgtcgctg gaccataatt aggcttctgt 1020

tcttcaggag acatttgttc aaagtcattt gggcaaccat attctgaaaa cagcccagcc 1080

agggtgatgg atcactttgc aaagatcctc aatgagctat tttcaagtga tgacaaagtg 1140

tgaagttaac cgctcatttg agaactttct ttttcatcca aagtaaattc aaatatgatt 1200

agaaatctga ccttttatta ctggaattct cttgactaaa agtaaaattg aattttaatt 1260

cctaaatctc catgtgtata cagtactgtg ggaacatcac agattttggc tccatgccct 1320

aaagagaaat tggctttcag attatttgga ttaaaaacaa agactttctt aagagatgta 1380

aaattttcat gatgttttct tttttgctaa aactaaagaa ttattctttt acatttca 1438

<210> 19
<211> 1438
<212> DNA
<213> artificial

<220>
<223> modified FIX intron

<400> 19
ggtttgtttc cttttttaaa atacattgag tatgcttgcc ttttagatat agaaatatct 60

gatgctgtct tcttcactaa attttgatta catgatttga cagcaatatt gaagagtcta 120

acagccagca cgcaggttgg taagtactgg ttctttgtta gctaggtttt cttcttcttc 180

atttttaaaa ctaaatagat cgacattgct tttgttgcat ttatgtttaa taaacactgt 240

tcagttcatg atttggtcat gtaattcctg ttagaaaaca ttcatctcct tggtttaaaa 300

aaattaaaag tgggaaaaca aagaaatagc agaatatagt gaaaaaaaat aaccacatta 360

tttttgtttg gacttaccac tttgaaatca aattgggaaa caaaagcaca aacaatggcc 420

ttatttacac aaaaagtctg attttaagat atatgacatt tcaaggtttc agaagtatgt 480

aatgaggtgt gtctctaatt ttttaaatta tatatcttca atttaaagtt ttagttaaaa 540

cataaagatt aacctttcat tagcaagctg ttagttatca ccaacgcttt tcatggatta 600

ggaaaaaatc attttgtctc tttgtcaaac atcttggagt tgatatttgg ggaaacacaa 660

tactcagttg agttccctag gggagaaaag cacgcttaag aattgacata aagagtagga 720

agttagctat tgcaacatat atcactttgt tttttcacaa ctacagtgac tttttgtatt 780

tcccagagga aggcatacag ggaagaaatt atcccatttg gacaaacagc ttgttctcac 840

aggaagcatt tatcacactt acttgtcaac tttctagaat caaatctagt agctgacagt 900

accaggatca ggggtgccaa ccctaagcac ccccagaaag ctgactggcc ctgtggttcc 960

cactccagac atgatgtcag ctgtgaaatc gacgtcgctg gaccataatt aggcttctgt 1020

tcttcaggag acatttgttc aaagtcattt gggcaaccat attctgaaaa cagcccagcc 1080

agggtgttgg atcactttgc aaagatcctc attgagctat tttcaagtgt tgacaaagtg 1140

tgaagttaac cgctcatttg agaactttct ttttcatcca aagtaaattc aaatatgatt 1200

agaaatctga ccttttatta ctggaattct cttgactaaa agtaaaattg aattttaatt 1260

cctaaatctc catgtgtata cagtactgtg ggaacatcac agattttggc tccatgccct 1320

aaagagaaat tggctttcag attatttgga ttaaaaacaa agactttctt aagagatgta 1380

aaatttcttt gttgttttct tttttgctaa aactaaagaa ttattctttt acatttca 1438

<210> 20
<211> 881
<212> DNA
<213> artificial

<220>
<223> chicken beta-globin intron

<400> 20
gcgggagtcg ctgcgttgcc ttcgccccgt gccccgctcc gccgccgcct cgcgccgccc 60

gccccggctc tgactgaccg cgttactccc acaggtgagc gggcgggacg gcccttctcc 120

tccgggctgt aattagcgct tggtttaatg acggcttgtt tcttttctgt ggctgcgtga 180

aagccttgag gggctccggg agggcccttt gtgcgggggg agcggctcgg ggggtgcgtg 240

cgtgtgtgtg tgcgtgggga gcgccgcgtg cggctccgcg ctgcccggcg gctgtgagcg 300

ctgcgggcgc ggcgcggggc tttgtgcgct ccgcagtgtg cgcgagggga gcgcggccgg 360

gggcggtgcc ccgcggtgcg gggggggctg cgaggggaac aaaggctgcg tgcggggtgt 420

gtgcgtgggg gggtgagcag ggggtgtggg cgcgtcggtc gggctgcaac cccccctgca 480

cccccctccc cgagttgctg agcacggccc ggcttcgggt gcggggctcc gtacggggcg 540

tggcgcgggg ctcgccgtgc cgggcggggg gtggcggcag gtgggggtgc cgggcggggc 600

ggggccgcct cgggccgggg agggctcggg ggaggggcgc ggcggccccc ggagcgccgg 660

cggctgtcga ggcgcggcga gccgcagcca ttgcctttta tggtaatcgt gcgagagggc 720

gcagggactt cctttgtccc aaatctgtgc ggagccgaaa tctgggaggc gccgccgcac 780

cccctctagc gggcgcgggg cgaagcggtg cggcgccggc aggaaggaaa tgggcgggga 840

gggccttcgt gcgtcgccgc gccgccgtcc ccttctccct c 881

<210> 21
<211> 881
<212> DNA
<213> artificial

<220>
<223> modified chicken beta-globin intron

<400> 21
gcgggagtcg ctgcgttgcc ttcgccccgt gccccgctcc gccgccgcct cgcgccgccc 60

gccccggctc tgactgaccg cgttactccc acaggtgagc gggcgggacg gcccttctcc 120

tccgggctgt aattagcgct tggtttaatg acggcttgtt tcttttctgt ggctgcgtga 180

aagccttgag gggctccggg agggcccttt gtgcgggggg agcggctcgg ggggtgcgtg 240

cgtgtgtgtg tgcgtgggga gcgccgcgtg cggctccgcg ctgcccggcg gctgtgagcg 300

ctgcgggcgc ggcgcggggc tttgtgcgct ccgcagtgtg cgcgagggga gcgcggccgg 360

gggcggtgcc ccgcggtgcg gggggggctg cgaggggaac aaaggctgcg tgcggggtgt 420

gtgcgtgggg gggtgagcag ggggtgtggg cgcgtcggtc gggctgcaac cccccctgca 480

cccccctccc cgagttgctg agcacggccc ggcttcgggt gcggggctcc gtacggggcg 540

tggcgcgggg ctcgccgtgc cgggcggggg gtggcggcag gtgggggtgc cgggcggggc 600

ggggccgcct cgggccgggg agggctcggg ggaggggcgc ggcggccccc ggagcgccgg 660

cggctgtcga ggcgcggcga gccgcagcca ttgccttttt tggtaatcgt gcgagagggc 720

gcagggactt cctttgtccc aaatctgtgc ggagccgaaa tctgggaggc gccgccgcac 780

cccctctagc gggcgcgggg cgaagcggtg cggcgccggc aggaaggaat tgggcgggga 840

gggccttcgt gcgtcgccgc gccgccgtcc ccttctccct c 881

<210> 22
<211> 4318
<212> DNA
<213> artificial

<220>
<223> construct: sp2+hGAAwt-delta-8

<400> 22
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120
tactcatgtc cctaaaatgg gcaaacattg caagcagcaa acagcaaaca cacagccctc 180
cctgcctgct gaccttggag ctggggcaga ggtcagagac ctctctgggc ccatgccacc 240
tccaacatcc actcgacccc ttggaatttc ggtggagagg agcagaggtt gtcctggcgt 300
ggtttaggta gtgtgagagg ggtacccggg gatcttgcta ccagtggaac agccactaag 360
gattctgcag tgagagcaga gggccagcta agtggtactc tcccagagac tgtctgactc 420
acgccacccc ctccaccttg gacacaggac gctgtggttt ctgagccagg tacaatgact 480
cctttcggta agtgcagtgg aagctgtaca ctgcccaggc aaagcgtccg ggcagcgtag 540
gcgggcgact cagatcccag ccagtggact tagcccctgt ttgctcctcc gataactggg 600
gtgaccttgg ttaatattca ccagcagcct cccccgttgc ccctctggat ccactgctta 660
aatacggacg aggacagggc cctgtctcct cagcttcagg caccaccact gacctgggac 720
agtgaataga tcctgagaac ttcagggtga gtctatggga cccttgatgt tttctttccc 780
cttcttttct atggttaagt tcatgtcata ggaaggggag aagtaacagg gtacacatat 840
tgaccaaatc agggtaattt tgcatttgta attttaaaaa atgctttctt cttttaatat 900
acttttttgt ttatcttatt tctaatactt tccctaatct ctttctttca gggcaataat 960
gatacaatgt atcatgcctc tttgcaccat tctaaagaat aacagtgata atttctgggt 1020
taaggcaata gcaatatttc tgcatataaa tatttctgca tataaattgt aactgatgta 1080
agaggtttca tattgctaat agcagctaca atccagctac cattctgctt ttattttctg 1140
gttgggataa ggctggatta ttctgagtcc aagctaggcc cttttgctaa tcttgttcat 1200
acctcttatc ttcctcccac agctcctggg caacctgctg gtctctctgc tggcccatca 1260
ctttggcaaa gcacgcgtgc caccatgccg tcttctgtct cgtggggcat cctcctgctg 1320
gcaggcctgt gctgcctggt ccctgtctcc ctggctctgc tggttccccg agagctgagt 1380
ggctcctccc cagtcctgga ggagactcac ccagctcacc agcagggagc cagcagacca 1440
gggccccggg atgcccaggc acaccccggg cggccgcgag cagtgcccac acagtgcgac 1500
gtccccccca acagccgctt cgattgcgcc cctgacaagg ccatcaccca ggaacagtgc 1560
gaggcccgcg gctgttgcta catccctgca aagcaggggc tgcagggagc ccagatgggg 1620
cagccctggt gcttcttccc acccagctac cccagctaca agctggagaa cctgagctcc 1680
tctgaaatgg gctacacggc caccctgacc cgtaccaccc ccaccttctt ccccaaggac 1740
atcctgaccc tgcggctgga cgtgatgatg gagactgaga accgcctcca cttcacgatc 1800
aaagatccag ctaacaggcg ctacgaggtg cccttggaga ccccgcatgt ccacagccgg 1860
gcaccgtccc cactctacag cgtggagttc tccgaggagc ccttcggggt gatcgtgcgc 1920
cggcagctgg acggccgcgt gctgctgaac acgacggtgg cgcccctgtt ctttgcggac 1980

cagttccttc agctgtccac ctcgctgccc tcgcagtata tcacaggcct cgccgagcac 2040

ctcagtcccc tgatgctcag caccagctgg accaggatca ccctgtggaa ccgggacctt 2100

gcgcccacgc ccggtgcgaa cctctacggg tctcaccctt tctacctggc gctggaggac 2160

ggcgggtcgg cacacggggt gttcctgcta aacagcaatg ccatggatgt ggtcctgcag 2220

ccgagccctg cccttagctg gaggtcgaca ggtgggatcc tggatgtcta catcttcctg 2280

ggcccagagc ccaagagcgt ggtgcagcag tacctggacg ttgtgggata cccgttcatg 2340

ccgccatact ggggcctggg cttccacctg tgccgctggg gctactcctc caccgctatc 2400

acccgccagg tggtggagaa catgaccagg gcccacttcc ccctggacgt ccagtggaac 2460

gacctggact acatggactc ccggagggac ttcacgttca acaaggatgg cttccgggac 2520

ttcccggcca tggtgcagga gctgcaccag ggcggccggc gctacatgat gatcgtggat 2580

cctgccatca gcagctcggg ccctgccggg agctacaggc cctacgacga gggtctgcgg 2640

aggggggttt tcatcaccaa cgagaccggc cagccgctga ttgggaaggt atggcccggg 2700

tccactgcct tccccgactt caccaacccc acagccctgg cctggtggga ggacatggtg 2760

gctgagttcc atgaccaggt gcccttcgac ggcatgtgga ttgacatgaa cgagccttcc 2820

aacttcatca ggggctctga ggacggctgc cccaacaatg agctggagaa cccaccctac 2880

gtgcctgggg tggttggggg gaccctccag gcggccacca tctgtgcctc cagccaccag 2940

tttctctcca cacactacaa cctgcacaac ctctacggcc tgaccgaagc catcgcctcc 3000

cacagggcgc tggtgaaggc tcgggggaca cgcccatttg tgatctcccg ctcgaccttt 3060

gctggccacg gccgatacgc cggccactgg acgggggacg tgtggagctc ctgggagcag 3120

ctcgcctcct ccgtgccaga aatcctgcag tttaacctgc tgggggtgcc tctggtcggg 3180

gccgacgtct gcggcttcct gggcaacacc tcagaggagc tgtgtgtgcg ctggacccag 3240

ctgggggcct tctacccctt catgcggaac cacaacagcc tgctcagtct gccccaggag 3300

ccgtacagct tcagcgagcc ggcccagcag gccatgagga aggccctcac cctgcgctac 3360

gcactcctcc cccacctcta cacactgttc caccaggccc acgtcgcggg ggagaccgtg 3420

gcccggcccc tcttcctgga gttccccaag gactctagca cctggactgt ggaccaccag 3480

ctcctgtggg gggaggccct gctcatcacc ccagtgctcc aggccgggaa ggccgaagtg 3540

actggctact tccccttggg cacatggtac gacctgcaga cggtgccagt agaggccctt 3600

ggcagcctcc cacccccacc tgcagctccc cgtgagccag ccatccacag cgaggggcag 3660

tgggtgacgc tgccggcccc cctggacacc atcaacgtcc acctccgggc tgggtacatc 3720

atccccctgc agggccctgg cctcacaacc acagagtccc gccagcagcc catggccctg 3780

gctgtggccc tgaccaaggg tggggaggcc cgaggggagc tgttctggga cgatggagag 3840

agcctggaag tgctggagcg aggggcctac acacaggtca tcttcctggc caggaataac 3900

acgatcgtga atgagctggt acgtgtgacc agtgagggag ctggcctgca gctgcagaag 3960

gtgactgtcc tgggcgtggc cacggcgccc cagcaggtcc tctccaacgg tgtccctgtc 4020

tccaacttca cctacagccc cgacaccaag gtcctggaca tctgtgtctc gctgttgatg 4080

ggagagcagt ttctcgtcag ctggtgttag ctcgagagat ctaccggtga attcaccgcg 4140

ggtttaaact gtgccttcta gttgccagcc atctgttgtt tgcccctccc ccgtgccttc 4200

cttgaccctg gaaggtgcca ctcccactgt cctttcctaa taaaatgagg aaattgcatc 4260

gcattgtctg agtaggtgtc attctattct ggggggtggg gtgggggcta gctctaga 4318

<210> 23
<211> 4318
<212> DNA
<213> artificial

<220>
<223> construct: sp2+hGAAco1-delta-8

<400> 23
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg ggtacccggg gatcttgcta ccagtggaac agccactaag 360

gattctgcag tgagagcaga gggccagcta agtggtactc tcccagagac tgtctgactc 420

acgccacccc ctccaccttg gacacaggac gctgtggttt ctgagccagg tacaatgact 480

cctttcggta agtgcagtgg aagctgtaca ctgcccaggc aaagcgtccg ggcagcgtag 540

gcgggcgact cagatcccag ccagtggact tagcccctgt ttgctcctcc gataactggg 600

gtgaccttgg ttaatattca ccagcagcct cccccgttgc ccctctggat ccactgctta 660

aatacggacg aggacagggc cctgtctcct cagcttcagg caccaccact gacctgggac 720

agtgaataga tcctgagaac ttcagggtga gtctatggga cccttgatgt tttctttccc 780

cttcttttct atggttaagt tcatgtcata ggaaggggag aagtaacagg gtacacatat 840

tgaccaaatc agggtaattt tgcatttgta attttaaaaa atgctttctt cttttaatat 900

acttttttgt ttatcttatt tctaatactt tccctaatct ctttctttca gggcaataat 960

gatacaatgt atcatgcctc tttgcaccat tctaaagaat aacagtgata atttctgggt 1020

taaggcaata gcaatatttc tgcatataaa tatttctgca tataaattgt aactgatgta 1080

agaggtttca tattgctaat agcagctaca atccagctac cattctgctt ttattttctg 1140

gttgggataa ggctggatta ttctgagtcc aagctaggcc cttttgctaa tcttgttcat 1200
acctcttatc ttcctcccac agctcctggg caacctgctg gtctctctgc tggcccatca 1260
ctttggcaaa gcacgcgtgc caccatgcct agctctgtgt cctggggcat tctgctgctg 1320
gccggcctgt gttgtctggt gcctgtgtct ctggccctac tagtgcccag agagctgagc 1380
ggcagctctc ccgtgctgga agaaacacac cctgcccatc agcagggcgc ctctagacct 1440
ggacctagag atgcccaggc ccaccccggc agacctagag ctgtgcctac ccagtgtgac 1500
gtgcccccca acagcagatt cgactgcgcc cctgacaagg ccatcaccca ggaacagtgc 1560
gaggccagag gctgctgcta catccctgcc aagcagggac tgcagggcgc tcagatggga 1620
cagccctggt gcttcttccc accctcctac cccagctaca agctggaaaa cctgagcagc 1680
agcgagatgg gctacaccgc caccctgacc agaaccaccc ccacattctt cccaaaggac 1740
atcctgaccc tgcggctgga cgtgatgatg gaaaccgaga accggctgca cttcaccatc 1800
aaggaccccg ccaatcggag atacgaggtg cccctggaaa ccccccacgt gcactctaga 1860
gcccccagcc ctctgtacag cgtggaattc agcgaggaac ccttcggcgt gatcgtgcgg 1920
agacagctgg atggcagagt gctgctgaac accaccgtgg cccctctgtt cttcgccgac 1980
cagttcctgc agctgagcac cagcctgccc agccagtaca tcacaggact ggccgagcac 2040
ctgagccccc tgatgctgag cacatcctgg acccggatca ccctgtggaa cagggatctg 2100
gcccctaccc ctggcgccaa tctgtacggc agccaccctt tctacctggc cctggaagat 2160
ggcggatctg cccacggagt gtttctgctg aactccaacg ccatggacgt ggtgctgcag 2220
cctagccctg ccctgtcttg gagaagcaca ggcggcatcc tggatgtgta catctttctg 2280
ggccccgagc ccaagagcgt ggtgcagcag tatctggatg tcgtgggcta ccccttcatg 2340
cccccttact ggggcctggg attccacctg tgcagatggg gctactccag caccgccatc 2400
accagacagg tggtggaaaa catgaccaga gcccacttcc cactggatgt gcagtggaac 2460
gacctggact acatggacag cagacgggac ttcaccttca acaaggacgg cttccgggac 2520
ttccccgcca tggtgcagga actgcatcag ggcggcagac ggtacatgat gatcgtggat 2580
cccgccatca gctcctctgg ccctgccggc tcttacagac cctacgacga gggcctgcgg 2640
agaggcgtgt tcatcaccaa cgagacaggc cagcccctga tcggcaaagt gtggcctggc 2700
agcacagcct tccccgactt caccaatcct accgccctgg cttggtggga ggacatggtg 2760
gccgagttcc acgaccaggt gcccttcgac ggcatgtgga tcgacatgaa cgagcccagc 2820
aacttcatcc ggggcagcga ggatggctgc cccaacaacg aactggaaaa tcccccttac 2880
gtgcccggcg tcgtgggcgg aacactgcag gccgctacaa tctgtgccag cagccaccag 2940
tttctgagca cccactacaa cctgcacaac ctgtacggcc tgaccgaggc cattgccagc 3000

caccgcgctc tcgtgaaagc cagaggcaca cggcccttcg tgatcagcag aagcaccttt 3060

gccggccacg gcagatacgc cggacattgg actggcgacg tgtggtcctc ttgggagcag 3120

ctggcctcta gcgtgcccga gatcctgcag ttcaatctgc tgggcgtgcc actcgtgggc 3180

gccgatgtgt gtggcttcct gggcaacacc tccgaggaac tgtgtgtgcg gtggacacag 3240

ctgggcgcct tctacccttt catgagaaac cacaacagcc tgctgagcct gccccaggaa 3300

ccctacagct ttagcgagcc tgcacagcag gccatgcgga aggccctgac actgagatac 3360

gctctgctgc cccacctgta caccctgttt caccaggccc atgtggccgg cgagacagtg 3420

gccagacctc tgtttctgga attccccaag gacagcagca cctggaccgt ggaccatcag 3480

ctgctgtggg gagaggctct gctgattacc ccagtgctgc aggcaggcaa ggccgaagtg 3540

accggctact ttcccctggg cacttggtac gacctgcaga ccgtgcctgt ggaagccctg 3600

ggatctctgc ctccacctcc tgccgctcct agagagcctg ccattcactc tgagggccag 3660

tgggtcacac tgcctgcccc cctggatacc atcaacgtgc acctgagggc cggctacatc 3720

ataccactgc agggacctgg cctgaccacc accgagtcta gacagcagcc aatggccctg 3780

gccgtggccc tgaccaaagg cggagaagct aggggcgagc tgttctggga cgatggcgag 3840

agcctggaag tgctggaaag aggcgcctat acccaagtga tcttcctggc ccggaacaac 3900

accatcgtga acgagctggt gcgcgtgacc tctgaaggcg ctggactgca gctgcagaaa 3960

gtgaccgtgc tgggagtggc cacagcccct cagcaggtgc tgtctaatgg cgtgcccgtg 4020

tccaacttca cctacagccc cgacaccaag gtgctggaca tctgcgtgtc actgctgatg 4080

ggagagcagt ttctggtgtc ctggtgctga ctcgagagat ctaccggtga attcaccgcg 4140

ggtttaaact gtgccttcta gttgccagcc atctgttgtt tgcccctccc ccgtgccttc 4200

cttgaccctg gaaggtgcca ctcccactgt cctttcctaa taaaatgagg aaattgcatc 4260

gcattgtctg agtaggtgtc attctattct ggggggtggg gtgggggcta gctctaga 4318

<210> 24
<211> 4318
<212> DNA
<213> artificial

<220>
<223> construct: sp2+hGAAco2-delta-8

<400> 24
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg ggtacccggg gatcttgcta ccagtggaac agccactaag 360

gattctgcag tgagagcaga gggccagcta agtggtactc tcccagagac tgtctgactc 420

acgccacccc ctccaccttg gacacaggac gctgtggttt ctgagccagg tacaatgact 480

cctttcggta agtgcagtgg aagctgtaca ctgcccaggc aaagcgtccg ggcagcgtag 540

gcgggcgact cagatcccag ccagtggact tagcccctgt ttgctcctcc gataactggg 600

gtgaccttgg ttaatattca ccagcagcct cccccgttgc ccctctggat ccactgctta 660

aatacggacg aggacagggc cctgtctcct cagcttcagg caccaccact gacctgggac 720

agtgaataga tcctgagaac ttcagggtga gtctatggga cccttgatgt tttctttccc 780

cttcttttct atggttaagt tcatgtcata ggaaggggag aagtaacagg gtacacatat 840

tgaccaaatc agggtaattt tgcatttgta attttaaaaa atgctttctt cttttaatat 900

acttttttgt ttatcttatt tctaatactt tccctaatct ctttctttca gggcaataat 960

gatacaatgt atcatgcctc tttgcaccat tctaaagaat aacagtgata atttctgggt 1020

taaggcaata gcaatatttc tgcatataaa tatttctgca tataaattgt aactgatgta 1080

agaggtttca tattgctaat agcagctaca atccagctac cattctgctt ttattttctg 1140

gttgggataa ggctggatta ttctgagtcc aagctaggcc cttttgctaa tcttgttcat 1200

acctcttatc ttcctcccac agctcctggg caacctgctg gtctctctgc tggcccatca 1260

ctttggcaaa gcacgcgtgc caccatgcca tcgtcagtgt cttggggcat tcttctgctc 1320

gccggattgt gttgcctggt gcctgtctca ttggccctgt tggtgcctag agagctgagc 1380

ggatcatccc cagtgctgga ggagactcat cctgctcacc aacagggagc ttccagacca 1440

ggaccgagag acgcccaagc ccatcctggt agaccaagag ctgtgcctac ccaatgcgac 1500

gtgccaccca actcccgatt cgactgcgcg ccagataagg ctattaccca agagcagtgt 1560

gaagccagag gttgctgcta catcccagcg aagcaaggat tgcaaggcgc ccaaatggga 1620

caaccttggt gtttcttccc cccttcgtac ccatcatata aactcgaaaa cctgtcctct 1680

tcggaaatgg gttatactgc caccctcacc agaactactc ctactttctt cccgaaagac 1740

atcttgacct tgaggctgga cgtgatgatg gagactgaaa accggctgca tttcactatc 1800

aaagatcctg ccaatcggcg atacgaggtc cctctggaaa cccctcacgt gcactcacgg 1860

gctccttctc cgctttactc cgtcgaattc tctgaggaac ccttcggagt gatcgttaga 1920

cgccagctgg atggtagagt gctgttgaac actactgtgg ccccactttt cttcgctgac 1980

cagtttctgc aactgtccac ttccctgcca tcccagtaca ttactggact cgccgaacac 2040

ctgtcgccac tgatgctctc gacctcttgg actagaatca ctttgtggaa cagagacttg 2100

gcccctactc cgggagcaaa tctgtacgga agccaccctt tttacctggc gctcgaagat 2160

ggcggatccg ctcacggagt gttcctgctg aatagcaacg caatggacgt ggtgctgcaa 2220
ccttcccctg cactcagttg gagaagtacc gggggtattc tggacgtgta catcttcctc 2280
ggaccagaac ccaagagcgt ggtgcagcaa tatctggacg tggtcggata cccttttatg 2340
cctccttact ggggactggg attccacctt tgccgttggg gctactcatc caccgccatt 2400
accagacagg tggtggagaa tatgaccaga gcccacttcc ctctcgacgt gcagtggaac 2460
gatctggact atatggactc ccggagagat ttcaccttca acaaggacgg gttccgcgat 2520
tttcccgcga tggttcaaga gctccaccag ggtggtcgaa gatatatgat gatcgtcgac 2580
ccagccattt cgagcagcgg acccgctgga tcttatagac cttacgacga aggccttagg 2640
agaggagtgt tcatcacaaa cgagactgga cagcctttga tcggtaaagt gtggcctgga 2700
tcaaccgcct ttcctgactt taccaatccc actgccttgg cttggtggga ggacatggtg 2760
gccgaattcc acgaccaagt cccctttgat ggaatgtgga tcgatatgaa cgaaccaagc 2820
aattttatca gaggttccga agacggttgc cccaacaacg aactggaaaa ccctccttat 2880
gtgcccggag tcgtgggcgg aacattacag gccgcgacta tttgcgccag cagccaccaa 2940
ttcctgtcca ctcactacaa cctccacaac ctttatggat taaccgaagc tattgcaagt 3000
cacagggctc tggtgaaggc tagagggact aggccctttg tgatctcccg atccaccttt 3060
gccggacacg ggagatacgc cggtcactgg actggtgacg tgtggagctc atgggaacaa 3120
ctggcctcct ccgtgccgga aatcttacag ttcaaccttc tgggtgtccc tcttgtcgga 3180
gcagacgtgt gtgggtttct tggtaacacc tccgaggaac tgtgtgtgcg ctggactcaa 3240
ctgggtgcat tctacccatt catgagaaac cacaactcct tgctgtccct gccacaagag 3300
ccctactcgt tcagcgagcc tgcacaacag gctatgcgga aggcactgac cctgagatac 3360
gccctgcttc cacacttata cactctcttc catcaagcgc atgtggcagg agaaaccgtt 3420
gcaaggcctc ttttccttga attccccaag gattcctcga cttggacggt ggatcatcag 3480
ctgctgtggg gagaagctct gctgattact ccagtgttgc aagccggaaa agctgaggtg 3540
accggatact ttccgctggg aacctggtac gacctccaga ctgtccctgt tgaagccctt 3600
ggatcactgc ctccgcctcc ggcagctcca cgcgaaccag ctatacattc cgagggacag 3660
tgggttacat taccagctcc tctggacaca atcaacgtcc acttaagagc tggctacatt 3720
atccctctgc aaggaccagg actgactacg accgagagca gacagcagcc aatggcactg 3780
gctgtggctc tgaccaaggg aggggaagct agaggagaac tcttctggga tgatggggag 3840
tcccttgaag tgctggaaag aggcgcttac actcaagtca ttttccttgc acggaacaac 3900
accattgtga acgaattggt gcgagtgacc agcgaaggag ctggacttca actgcagaag 3960
gtcactgtgc tcggagtggc taccgctcct cagcaagtgc tgtcgaatgg agtccccgtg 4020
tcaaacttta cctactcccc tgacactaag gtgctcgaca tttgcgtgtc cctcctgatg 4080

ggagagcagt tccttgtgtc ctggtgttga ctcgagagat ctaccggtga attcaccgcg 4140

ggtttaaact gtgccttcta gttgccagcc atctgttgtt tgcccctccc ccgtgccttc 4200

cttgaccctg gaaggtgcca ctcccactgt cctttcctaa taaaatgagg aaattgcatc 4260

gcattgtctg agtaggtgtc attctattct ggggggtggg gtgggggcta gctctaga 4318

<210> 25
<211> 4300
<212> DNA
<213> artificial

<220>
<223> construct: sp7+hGAAco1-delta-8

<400> 25
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg ggtacccggg gatcttgcta ccagtggaac agccactaag 360

gattctgcag tgagagcaga gggccagcta agtggtactc tcccagagac tgtctgactc 420

acgccacccc ctccaccttg gacacaggac gctgtggttt ctgagccagg tacaatgact 480

cctttcggta agtgcagtgg aagctgtaca ctgcccaggc aaagcgtccg ggcagcgtag 540

gcgggcgact cagatcccag ccagtggact tagcccctgt ttgctcctcc gataactggg 600

gtgaccttgg ttaatattca ccagcagcct cccccgttgc ccctctggat ccactgctta 660

aatacggacg aggacagggc cctgtctcct cagcttcagg caccaccact gacctgggac 720

agtgaataga tcctgagaac ttcagggtga gtctatggga cccttgatgt tttctttccc 780

cttcttttct atggttaagt tcatgtcata ggaaggggag aagtaacagg gtacacatat 840

tgaccaaatc agggtaattt tgcatttgta attttaaaaa atgctttctt cttttaatat 900

acttttttgt ttatcttatt tctaatactt tccctaatct ctttctttca gggcaataat 960

gatacaatgt atcatgcctc tttgcaccat tctaaagaat aacagtgata atttctgggt 1020

taaggcaata gcaatatttc tgcatataaa tatttctgca tataaattgt aactgatgta 1080

agaggtttca tattgctaat agcagctaca atccagctac cattctgctt ttattttctg 1140

gttgggataa ggctggatta ttctgagtcc aagctaggcc cttttgctaa tcttgttcat 1200

acctcttatc ttcctcccac agctcctggg caacctgctg gtctctctgc tggcccatca 1260

ctttggcaaa gcacgcgtgc caccatggcc tttctgtggc tgctgagctg ttgggccctg 1320

ctgggcacca ccttcggcct actagtgccc agagagctga gcggcagctc tcccgtgctg 1380
gaagaaacac accctgccca tcagcagggc gcctctagac ctggacctag agatgcccag 1440
gcccaccccg gcagacctag agctgtgcct acccagtgtg acgtgccccc caacagcaga 1500
ttcgactgcg cccctgacaa ggccatcacc caggaacagt gcgaggccag aggctgctgc 1560
tacatccctg ccaagcaggg actgcagggc gctcagatgg gacagccctg gtgcttcttc 1620
ccaccctcct accccagcta caagctggaa aacctgagca gcagcgagat gggctacacc 1680
gccaccctga ccagaaccac ccccacattc ttcccaaagg acatcctgac cctgcggctg 1740
gacgtgatga tggaaaccga gaaccggctg cacttcacca tcaaggaccc cgccaatcgg 1800
agatacgagg tgcccctgga aaccccccac gtgcactcta gagcccccag ccctctgtac 1860
agcgtggaat tcagcgagga acccttcggc gtgatcgtgc ggagacagct ggatggcaga 1920
gtgctgctga acaccaccgt ggcccctctg ttcttcgccg accagttcct gcagctgagc 1980
accagcctgc ccagccagta catcacagga ctggccgagc acctgagccc cctgatgctg 2040
agcacatcct ggacccggat caccctgtgg aacagggatc tggcccctac ccctggcgcc 2100
aatctgtacg gcagccaccc tttctacctg gccctggaag atggcggatc tgcccacgga 2160
gtgtttctgc tgaactccaa cgccatggac gtggtgctgc agcctagccc tgccctgtct 2220
tggagaagca caggcggcat cctggatgtg tacatctttc tgggccccga gcccaagagc 2280
gtggtgcagc agtatctgga tgtcgtgggc taccccttca tgccccctta ctggggcctg 2340
ggattccacc tgtgcagatg gggctactcc agcaccgcca tcaccagaca ggtggtggaa 2400
aacatgacca gagcccactt cccactggat gtgcagtgga acgacctgga ctacatggac 2460
agcagacggg acttcacctt caacaaggac ggcttccggg acttccccgc catggtgcag 2520
gaactgcatc agggcggcag acggtacatg atgatcgtgg atcccgccat cagctcctct 2580
ggccctgccg gctcttacag accctacgac gagggcctgc ggagaggcgt gttcatcacc 2640
aacgagacag gccagcccct gatcggcaaa gtgtggcctg gcagcacagc cttccccgac 2700
ttcaccaatc ctaccgccct ggcttggtgg gaggacatgg tggccgagtt ccacgaccag 2760
gtgcccttcg acggcatgtg gatcgacatg aacgagccca gcaacttcat ccggggcagc 2820
gaggatggct gccccaacaa cgaactggaa aatccccctt acgtgcccgg cgtcgtgggc 2880
ggaacactgc aggccgctac aatctgtgcc agcagccacc agtttctgag cacccactac 2940
aacctgcaca acctgtacgg cctgaccgag gccattgcca gccaccgcgc tctcgtgaaa 3000
gccagaggca cacggccctt cgtgatcagc agaagcacct ttgccggcca cggcagatac 3060
gccggacatt ggactggcga cgtgtggtcc tcttgggagc agctggcctc tagcgtgccc 3120
gagatcctgc agttcaatct gctgggcgtg ccactcgtgg gcgccgatgt gtgtggcttc 3180
ctgggcaaca cctccgagga actgtgtgtg cggtggacac agctgggcgc cttctaccct 3240

ttcatgagaa accacaacag cctgctgagc ctgccccagg aaccctacag ctttagcgag 3300

cctgcacagc aggccatgcg gaaggccctg acactgagat acgctctgct gccccacctg 3360

tacaccctgt ttcaccaggc ccatgtggcc ggcgagacag tggccagacc tctgtttctg 3420

gaattcccca aggacagcag cacctggacc gtggaccatc agctgctgtg gggagaggct 3480

ctgctgatta ccccagtgct gcaggcaggc aaggccgaag tgaccggcta ctttcccctg 3540

ggcacttggt acgacctgca gaccgtgcct gtggaagccc tgggatctct gcctccacct 3600

cctgccgctc ctagagagcc tgccattcac tctgagggcc agtgggtcac actgcctgcc 3660

cccctggata ccatcaacgt gcacctgagg gccggctaca tcataccact gcagggacct 3720

ggcctgacca ccaccgagtc tagacagcag ccaatggccc tggccgtggc cctgaccaaa 3780

ggcggagaag ctaggggcga gctgttctgg gacgatggcg agagcctgga agtgctggaa 3840

agaggcgcct atacccaagt gatcttcctg gcccggaaca acaccatcgt gaacgagctg 3900

gtgcgcgtga cctctgaagg cgctggactg cagctgcaga aagtgaccgt gctgggagtg 3960

gccacagccc ctcagcaggt gctgtctaat ggcgtgcccg tgtccaactt cacctacagc 4020

cccgacacca aggtgctgga catctgcgtg tcactgctga tgggagagca gtttctggtg 4080

tcctggtgct gactcgagag atctaccggt gaattcaccg cgggtttaaa ctgtgccttc 4140

tagttgccag ccatctgttg tttgcccctc ccccgtgcct tccttgaccc tggaaggtgc 4200

cactcccact gtcctttcct aataaaatga ggaaattgca tcgcattgtc tgagtaggtg 4260

tcattctatt ctggggggtg gggtgggggc tagctctaga 4300

<210> 26
<211> 4198
<212> DNA
<213> artificial

<220>
<223> sp7+hGAAco1-delta-42

<400> 26
   aggctcagag gcacacagga gtttctgggc tcaccctgcc cccttccaac ccctcagttc 60
   ccatcctcca gcagctgttt gtgtgctgcc tctgaagtcc acactgaaca aacttcagcc 120
   tactcatgtc cctaaaatgg gcaaacattg caagcagcaa acagcaaaca cacagccctc 180
   cctgcctgct gaccttggag ctggggcaga ggtcagagac ctctctgggc ccatgccacc 240
   tccaacatcc actcgacccc ttggaatttc ggtggagagg agcagaggtt gtcctggcgt 300
   ggtttaggta gtgtgagagg ggtacccggg gatcttgcta ccagtggaac agccactaag 360
   gattctgcag tgagagcaga gggccagcta agtggtactc tcccagagac tgtctgactc 420
   acgccacccc ctccaccttg gacacaggac gctgtggttt ctgagccagg tacaatgact 480
   cctttcggta agtgcagtgg aagctgtaca ctgcccaggc aaagcgtccg ggcagcgtag 540
   gcgggcgact cagatcccag ccagtggact tagcccctgt ttgctcctcc gataactggg 600
   gtgaccttgg ttaatattca ccagcagcct cccccgttgc ccctctggat ccactgctta 660
   aatacggacg aggacagggc cctgtctcct cagcttcagg caccaccact gacctgggac 720
   agtgaataga tcctgagaac ttcagggtga gtctatggga cccttgatgt tttctttccc 780
   cttcttttct atggttaagt tcatgtcata ggaaggggag aagtaacagg gtacacatat 840
   tgaccaaatc agggtaattt tgcatttgta attttaaaaa atgctttctt cttttaatat 900
   acttttttgt ttatcttatt tctaatactt tccctaatct ctttctttca gggcaataat 960
   gatacaatgt atcatgcctc tttgcaccat tctaaagaat aacagtgata atttctgggt 1020
   taaggcaata gcaatatttc tgcatataaa tatttctgca tataaattgt aactgatgta 1080
   agaggtttca tattgctaat agcagctaca atccagctac cattctgctt ttattttctg 1140
   gttgggataa ggctggatta ttctgagtcc aagctaggcc cttttgctaa tcttgttcat 1200
   acctcttatc ttcctcccac agctcctggg caacctgctg gtctctctgc tggcccatca 1260
   ctttggcaaa gcacgcgtgc caccatggcc tttctgtggc tgctgagctg ttgggccctg 1320
   ctgggcacca ccttcggcgc ccaccccggc agacctagag ctgtgcctac ccagtgtgac 1380
   gtgcccccca acagcagatt cgactgcgcc cctgacaagg ccatcaccca ggaacagtgc 1440
   gaggccagag gctgctgcta catccctgcc aagcagggac tgcagggcgc tcagatggga 1500
   cagccctggt gcttcttccc accctcctac cccagctaca agctggaaaa cctgagcagc 1560
   agcgagatgg gctacaccgc caccctgacc agaaccaccc ccacattctt cccaaaggac 1620
   atcctgaccc tgcggctgga cgtgatgatg gaaaccgaga accggctgca cttcaccatc 1680
   aaggaccccg ccaatcggag atacgaggtg cccctggaaa ccccccacgt gcactctaga 1740
   gcccccagcc ctctgtacag cgtggaattc agcgaggaac ccttcggcgt gatcgtgcgg 1800
   agacagctgg atggcagagt gctgctgaac accaccgtgg cccctctgtt cttcgccgac 1860
   cagttcctgc agctgagcac cagcctgccc agccagtaca tcacaggact ggccgagcac 1920
   ctgagccccc tgatgctgag cacatcctgg acccggatca ccctgtggaa cagggatctg 1980
   gcccctaccc ctggcgccaa tctgtacggc agccaccctt tctacctggc cctggaagat 2040
   ggcggatctg cccacggagt gtttctgctg aactccaacg ccatggacgt ggtgctgcag 2100
   cctagccctg ccctgtcttg gagaagcaca ggcggcatcc tggatgtgta catctttctg 2160
   ggccccgagc ccaagagcgt ggtgcagcag tatctggatg tcgtgggcta ccccttcatg 2220
   cccccttact ggggcctggg attccacctg tgcagatggg gctactccag caccgccatc 2280
   accagacagg tggtggaaaa catgaccaga gcccacttcc cactggatgt gcagtggaac 2340
   gacctggact acatggacag cagacgggac ttcaccttca acaaggacgg cttccgggac 2400
   ttccccgcca tggtgcagga actgcatcag ggcggcagac ggtacatgat gatcgtggat 2460
   cccgccatca gctcctctgg ccctgccggc tcttacagac cctacgacga gggcctgcgg 2520
   agaggcgtgt tcatcaccaa cgagacaggc cagcccctga tcggcaaagt gtggcctggc 2580
   agcacagcct tccccgactt caccaatcct accgccctgg cttggtggga ggacatggtg 2640
   gccgagttcc acgaccaggt gcccttcgac ggcatgtgga tcgacatgaa cgagcccagc 2700
   aacttcatcc ggggcagcga ggatggctgc cccaacaacg aactggaaaa tcccccttac 2760
   gtgcccggcg tcgtgggcgg aacactgcag gccgctacaa tctgtgccag cagccaccag 2820
   tttctgagca cccactacaa cctgcacaac ctgtacggcc tgaccgaggc cattgccagc 2880
   caccgcgctc tcgtgaaagc cagaggcaca cggcccttcg tgatcagcag aagcaccttt 2940
   gccggccacg gcagatacgc cggacattgg actggcgacg tgtggtcctc ttgggagcag 3000
   ctggcctcta gcgtgcccga gatcctgcag ttcaatctgc tgggcgtgcc actcgtgggc 3060
   gccgatgtgt gtggcttcct gggcaacacc tccgaggaac tgtgtgtgcg gtggacacag 3120
   ctgggcgcct tctacccttt catgagaaac cacaacagcc tgctgagcct gccccaggaa 3180
   ccctacagct ttagcgagcc tgcacagcag gccatgcgga aggccctgac actgagatac 3240
   gctctgctgc cccacctgta caccctgttt caccaggccc atgtggccgg cgagacagtg 3300
   gccagacctc tgtttctgga attccccaag gacagcagca cctggaccgt ggaccatcag 3360
   ctgctgtggg gagaggctct gctgattacc ccagtgctgc aggcaggcaa ggccgaagtg 3420
   accggctact ttcccctggg cacttggtac gacctgcaga ccgtgcctgt ggaagccctg 3480
   ggatctctgc ctccacctcc tgccgctcct agagagcctg ccattcactc tgagggccag 3540
   tgggtcacac tgcctgcccc cctggatacc atcaacgtgc acctgagggc cggctacatc 3600
   ataccactgc agggacctgg cctgaccacc accgagtcta gacagcagcc aatggccctg 3660
   gccgtggccc tgaccaaagg cggagaagct aggggcgagc tgttctggga cgatggcgag 3720
   agcctggaag tgctggaaag aggcgcctat acccaagtga tcttcctggc ccggaacaac 3780
   accatcgtga acgagctggt gcgcgtgacc tctgaaggcg ctggactgca gctgcagaaa 3840
   gtgaccgtgc tgggagtggc cacagcccct cagcaggtgc tgtctaatgg cgtgcccgtg 3900
   tccaacttca cctacagccc cgacaccaag gtgctggaca tctgcgtgtc actgctgatg 3960
   ggagagcagt ttctggtgtc ctggtgctga ctcgagagat ctaccggtga attcaccgcg 4020
   ggtttaaact gtgccttcta gttgccagcc atctgttgtt tgcccctccc ccgtgccttc 4080
   cttgaccctg gaaggtgcca ctcccactgt cctttcctaa taaaatgagg aaattgcatc 4140
   gcattgtctg agtaggtgtc attctattct ggggggtggg gtgggggcta gctctaga 4198

<210> 27

<211>  917
<212>  PRT
<213>  artificial

<220>
<223>  hGAA-delta-8

<400>  27

Leu Leu Val Pro Arg Glu Leu Ser Gly Ser Ser Pro Val Leu Glu Glu
1               5                   10                  15

Thr His Pro Ala His Gln Gln Gly Ala Ser Arg Pro Gly Pro Arg Asp
            20                  25                  30

Ala Gln Ala His Pro Gly Arg Pro Arg Ala Val Pro Thr Gln Cys Asp
        35                  40                  45

Val Pro Pro Asn Ser Arg Phe Asp Cys Ala Pro Asp Lys Ala Ile Thr
    50                  55                  60

Gln Glu Gln Cys Glu Ala Arg Gly Cys Cys Tyr Ile Pro Ala Lys Gln
65                  70                  75                  80

Gly Leu Gln Gly Ala Gln Met Gly Gln Pro Trp Cys Phe Phe Pro Pro
                85                  90                  95

Ser Tyr Pro Ser Tyr Lys Leu Glu Asn Leu Ser Ser Ser Glu Met Gly
            100                 105                 110

Tyr Thr Ala Thr Leu Thr Arg Thr Thr Pro Thr Phe Phe Pro Lys Asp
        115                 120                 125

Ile Leu Thr Leu Arg Leu Asp Val Met Met Glu Thr Glu Asn Arg Leu
    130                 135                 140

His Phe Thr Ile Lys Asp Pro Ala Asn Arg Arg Tyr Glu Val Pro Leu
145                 150                 155                 160

Glu Thr Pro His Val His Ser Arg Ala Pro Ser Pro Leu Tyr Ser Val
                165                 170                 175

Glu Phe Ser Glu Glu Pro Phe Gly Val Ile Val Arg Arg Gln Leu Asp
            180                 185                 190

Gly Arg Val Leu Leu Asn Thr Thr Val Ala Pro Leu Phe Phe Ala Asp
        195                 200                 205

Gln Phe Leu Gln Leu Ser Thr Ser Leu Pro Ser Gln Tyr Ile Thr Gly
    210                 215                 220

Leu Ala Glu His Leu Ser Pro Leu Met Leu Ser Thr Ser Trp Thr Arg
225                 230                 235                 240

Ile Thr Leu Trp Asn Arg Asp Leu Ala Pro Thr Pro Gly Ala Asn Leu
                245                 250                 255

Tyr Gly Ser His Pro Phe Tyr Leu Ala Leu Glu Asp Gly Gly Ser Ala
            260                 265                 270

His Gly Val Phe Leu Leu Asn Ser Asn Ala Met Asp Val Val Leu Gln
        275                 280                 285

Pro Ser Pro Ala Leu Ser Trp Arg Ser Thr Gly Gly Ile Leu Asp Val
    290                 295                 300

Tyr Ile Phe Leu Gly Pro Glu Pro Lys Ser Val Val Gln Gln Tyr Leu
305                 310                 315                 320

Asp Val Val Gly Tyr Pro Phe Met Pro Pro Tyr Trp Gly Leu Gly Phe
                325                 330                 335

His Leu Cys Arg Trp Gly Tyr Ser Ser Thr Ala Ile Thr Arg Gln Val
            340                 345                 350

Val Glu Asn Met Thr Arg Ala His Phe Pro Leu Asp Val Gln Trp Asn
        355                 360                 365

Asp Leu Asp Tyr Met Asp Ser Arg Arg Asp Phe Thr Phe Asn Lys Asp
    370                 375                 380

Gly Phe Arg Asp Phe Pro Ala Met Val Gln Glu Leu His Gln Gly Gly
385                 390                 395                 400

Arg Arg Tyr Met Met Ile Val Asp Pro Ala Ile Ser Ser Ser Gly Pro
                405                 410                 415

Ala Gly Ser Tyr Arg Pro Tyr Asp Glu Gly Leu Arg Arg Gly Val Phe
            420                 425                 430

Ile Thr Asn Glu Thr Gly Gln Pro Leu Ile Gly Lys Val Trp Pro Gly
        435                 440                 445

Ser Thr Ala Phe Pro Asp Phe Thr Asn Pro Thr Ala Leu Ala Trp Trp
    450                 455                 460

Glu Asp Met Val Ala Glu Phe His Asp Gln Val Pro Phe Asp Gly Met

465                 470                 475                 480

Trp Ile Asp Met Asn Glu Pro Ser Asn Phe Ile Arg Gly Ser Glu Asp
                485                 490                 495

Gly Cys Pro Asn Asn Glu Leu Glu Asn Pro Pro Tyr Val Pro Gly Val
            500                 505                 510

Val Gly Gly Thr Leu Gln Ala Ala Thr Ile Cys Ala Ser Ser His Gln
        515                 520                 525

Phe Leu Ser Thr His Tyr Asn Leu His Asn Leu Tyr Gly Leu Thr Glu
    530                 535                 540

Ala Ile Ala Ser His Arg Ala Leu Val Lys Ala Arg Gly Thr Arg Pro
545                 550                 555                 560

Phe Val Ile Ser Arg Ser Thr Phe Ala Gly His Gly Arg Tyr Ala Gly
                565                 570                 575

His Trp Thr Gly Asp Val Trp Ser Ser Trp Glu Gln Leu Ala Ser Ser
            580                 585                 590

Val Pro Glu Ile Leu Gln Phe Asn Leu Leu Gly Val Pro Leu Val Gly
        595                 600                 605

Ala Asp Val Cys Gly Phe Leu Gly Asn Thr Ser Glu Glu Leu Cys Val
    610                 615                 620

Arg Trp Thr Gln Leu Gly Ala Phe Tyr Pro Phe Met Arg Asn His Asn
625                 630                 635                 640

Ser Leu Leu Ser Leu Pro Gln Glu Pro Tyr Ser Phe Ser Glu Pro Ala
                645                 650                 655

Gln Gln Ala Met Arg Lys Ala Leu Thr Leu Arg Tyr Ala Leu Leu Pro
            660                 665                 670

His Leu Tyr Thr Leu Phe His Gln Ala His Val Ala Gly Glu Thr Val
        675                 680                 685

Ala Arg Pro Leu Phe Leu Glu Phe Pro Lys Asp Ser Ser Thr Trp Thr
    690                 695                 700

Val Asp His Gln Leu Leu Trp Gly Glu Ala Leu Leu Ile Thr Pro Val
705                 710                 715                 720

Leu Gln Ala Gly Lys Ala Glu Val Thr Gly Tyr Phe Pro Leu Gly Thr
                725                 730                 735

Trp Tyr Asp Leu Gln Thr Val Pro Val Glu Ala Leu Gly Ser Leu Pro
            740                 745                 750

Pro Pro Pro Ala Ala Pro Arg Glu Pro Ala Ile His Ser Glu Gly Gln
        755                 760                 765

Trp Val Thr Leu Pro Ala Pro Leu Asp Thr Ile Asn Val His Leu Arg
    770                 775                 780

Ala Gly Tyr Ile Ile Pro Leu Gln Gly Pro Gly Leu Thr Thr Thr Glu
785                 790                 795                 800

Ser Arg Gln Gln Pro Met Ala Leu Ala Val Ala Leu Thr Lys Gly Gly
                805                 810                 815

Glu Ala Arg Gly Glu Leu Phe Trp Asp Asp Gly Glu Ser Leu Glu Val
            820                 825                 830

Leu Glu Arg Gly Ala Tyr Thr Gln Val Ile Phe Leu Ala Arg Asn Asn
        835                 840                 845

Thr Ile Val Asn Glu Leu Val Arg Val Thr Ser Glu Gly Ala Gly Leu
    850                 855                 860

Gln Leu Gln Lys Val Thr Val Leu Gly Val Ala Thr Ala Pro Gln Gln
865                 870                 875                 880

Val Leu Ser Asn Gly Val Pro Val Ser Asn Phe Thr Tyr Ser Pro Asp
                885                 890                 895

Thr Lys Val Leu Asp Ile Cys Val Ser Leu Leu Met Gly Glu Gln Phe
            900                 905                 910

Leu Val Ser Trp Cys
        915

<210>  28
<211>  883
<212>  PRT
<213>  artificial

<220>
<223>  hGAA-delta-42

<400>  28

Ala His Pro Gly Arg Pro Arg Ala Val Pro Thr Gln Cys Asp Val Pro

1               5                   10                  15

Pro Asn Ser Arg Phe Asp Cys Ala Pro Asp Lys Ala Ile Thr Gln Glu
            20                  25                  30

Gln Cys Glu Ala Arg Gly Cys Cys Tyr Ile Pro Ala Lys Gln Gly Leu
        35                  40                  45

Gln Gly Ala Gln Met Gly Gln Pro Trp Cys Phe Phe Pro Pro Ser Tyr
    50                  55                  60

Pro Ser Tyr Lys Leu Glu Asn Leu Ser Ser Ser Glu Met Gly Tyr Thr
65                  70                  75                  80

Ala Thr Leu Thr Arg Thr Thr Pro Thr Phe Phe Pro Lys Asp Ile Leu
                85                  90                  95

Thr Leu Arg Leu Asp Val Met Met Glu Thr Glu Asn Arg Leu His Phe
            100                 105                 110

Thr Ile Lys Asp Pro Ala Asn Arg Arg Tyr Glu Val Pro Leu Glu Thr
        115                 120                 125

Pro His Val His Ser Arg Ala Pro Ser Pro Leu Tyr Ser Val Glu Phe
    130                 135                 140

Ser Glu Glu Pro Phe Gly Val Ile Val Arg Arg Gln Leu Asp Gly Arg
145                 150                 155                 160

Val Leu Leu Asn Thr Thr Val Ala Pro Leu Phe Phe Ala Asp Gln Phe
                165                 170                 175

Leu Gln Leu Ser Thr Ser Leu Pro Ser Gln Tyr Ile Thr Gly Leu Ala
            180                 185                 190

Glu His Leu Ser Pro Leu Met Leu Ser Thr Ser Trp Thr Arg Ile Thr
        195                 200                 205

Leu Trp Asn Arg Asp Leu Ala Pro Thr Pro Gly Ala Asn Leu Tyr Gly
    210                 215                 220

Ser His Pro Phe Tyr Leu Ala Leu Glu Asp Gly Gly Ser Ala His Gly
225                 230                 235                 240

Val Phe Leu Leu Asn Ser Asn Ala Met Asp Val Val Leu Gln Pro Ser
                245                 250                 255

Pro Ala Leu Ser Trp Arg Ser Thr Gly Gly Ile Leu Asp Val Tyr Ile
            260                 265                 270

Phe Leu Gly Pro Glu Pro Lys Ser Val Val Gln Gln Tyr Leu Asp Val
        275                 280                 285

Val Gly Tyr Pro Phe Met Pro Pro Tyr Trp Gly Leu Gly Phe His Leu
    290                 295                 300

Cys Arg Trp Gly Tyr Ser Ser Thr Ala Ile Thr Arg Gln Val Val Glu
305                 310                 315                 320

Asn Met Thr Arg Ala His Phe Pro Leu Asp Val Gln Trp Asn Asp Leu
                325                 330                 335

Asp Tyr Met Asp Ser Arg Arg Asp Phe Thr Phe Asn Lys Asp Gly Phe
            340                 345                 350

Arg Asp Phe Pro Ala Met Val Gln Glu Leu His Gln Gly Gly Arg Arg
        355                 360                 365

Tyr Met Met Ile Val Asp Pro Ala Ile Ser Ser Ser Gly Pro Ala Gly
    370                 375                 380

Ser Tyr Arg Pro Tyr Asp Glu Gly Leu Arg Arg Gly Val Phe Ile Thr
385                 390                 395                 400

Asn Glu Thr Gly Gln Pro Leu Ile Gly Lys Val Trp Pro Gly Ser Thr
                405                 410                 415

Ala Phe Pro Asp Phe Thr Asn Pro Thr Ala Leu Ala Trp Trp Glu Asp
            420                 425                 430

Met Val Ala Glu Phe His Asp Gln Val Pro Phe Asp Gly Met Trp Ile
        435                 440                 445

Asp Met Asn Glu Pro Ser Asn Phe Ile Arg Gly Ser Glu Asp Gly Cys
    450                 455                 460

Pro Asn Asn Glu Leu Glu Asn Pro Pro Tyr Val Pro Gly Val Val Gly
465                 470                 475                 480

Gly Thr Leu Gln Ala Ala Thr Ile Cys Ala Ser Ser His Gln Phe Leu
                485                 490                 495

Ser Thr His Tyr Asn Leu His Asn Leu Tyr Gly Leu Thr Glu Ala Ile
            500                 505                 510

Ala Ser His Arg Ala Leu Val Lys Ala Arg Gly Thr Arg Pro Phe Val
        515                 520                 525

Ile Ser Arg Ser Thr Phe Ala Gly His Gly Arg Tyr Ala Gly His Trp
    530                 535                 540

Thr Gly Asp Val Trp Ser Ser Trp Glu Gln Leu Ala Ser Ser Val Pro
545                 550                 555                 560

Glu Ile Leu Gln Phe Asn Leu Leu Gly Val Pro Leu Val Gly Ala Asp
                565                 570                 575

Val Cys Gly Phe Leu Gly Asn Thr Ser Glu Glu Leu Cys Val Arg Trp
            580                 585                 590

Thr Gln Leu Gly Ala Phe Tyr Pro Phe Met Arg Asn His Asn Ser Leu
        595                 600                 605

Leu Ser Leu Pro Gln Glu Pro Tyr Ser Phe Ser Glu Pro Ala Gln Gln
    610                 615                 620

Ala Met Arg Lys Ala Leu Thr Leu Arg Tyr Ala Leu Leu Pro His Leu
625                 630                 635                 640

Tyr Thr Leu Phe His Gln Ala His Val Ala Gly Glu Thr Val Ala Arg
                645                 650                 655

Pro Leu Phe Leu Glu Phe Pro Lys Asp Ser Ser Thr Trp Thr Val Asp
            660                 665                 670

His Gln Leu Leu Trp Gly Glu Ala Leu Leu Ile Thr Pro Val Leu Gln
        675                 680                 685

Ala Gly Lys Ala Glu Val Thr Gly Tyr Phe Pro Leu Gly Thr Trp Tyr
    690                 695                 700

Asp Leu Gln Thr Val Pro Val Glu Ala Leu Gly Ser Leu Pro Pro Pro
705                 710                 715                 720

Pro Ala Ala Pro Arg Glu Pro Ala Ile His Ser Glu Gly Gln Trp Val
                725                 730                 735

Thr Leu Pro Ala Pro Leu Asp Thr Ile Asn Val His Leu Arg Ala Gly
            740                 745                 750

Tyr Ile Ile Pro Leu Gln Gly Pro Gly Leu Thr Thr Thr Glu Ser Arg
        755                 760                 765

Gln Gln Pro Met Ala Leu Ala Val Ala Leu Thr Lys Gly Gly Glu Ala
    770                 775                 780

Arg Gly Glu Leu Phe Trp Asp Asp Gly Glu Ser Leu Glu Val Leu Glu
785                 790                 795                 800

Arg Gly Ala Tyr Thr Gln Val Ile Phe Leu Ala Arg Asn Asn Thr Ile
                805                 810                 815

Val Asn Glu Leu Val Arg Val Thr Ser Glu Gly Ala Gly Leu Gln Leu
            820                 825                 830

Gln Lys Val Thr Val Leu Gly Val Ala Thr Ala Pro Gln Gln Val Leu
        835                 840                 845

Ser Asn Gly Val Pro Val Ser Asn Phe Thr Tyr Ser Pro Asp Thr Lys
    850                 855                 860

Val Leu Asp Ile Cys Val Ser Leu Leu Met Gly Glu Gln Phe Leu Val
865                 870                 875                 880

Ser Trp Cys

<210>  29
<211>  952
<212>  PRT
<213>  homo sapiens

<400>  29

<210>  30
<211>  952
<212>  PRT
<213>  homo sapiens

<400>  30

Met Gly Val Arg His Pro Pro Cys Ser His Arg Leu Leu Ala Val Cys
1               5                   10                  15

Ala Leu Val Ser Leu Ala Thr Ala Ala Leu Leu Gly His Ile Leu Leu
            20                  25                  30

His Asp Phe Leu Leu Val Pro Arg Glu Leu Ser Gly Ser Ser Pro Val
        35                  40                  45

Leu Glu Glu Thr His Pro Ala His Gln Gln Gly Ala Ser Arg Pro Gly
    50                  55                  60

Pro Arg Asp Ala Gln Ala His Pro Gly Arg Pro Arg Ala Val Pro Thr
65                  70                  75                  80

Gln Cys Asp Val Pro Pro Asn Ser Arg Phe Asp Cys Ala Pro Asp Lys
                85                  90                  95

Ala Ile Thr Gln Glu Gln Cys Glu Ala Arg Gly Cys Cys Tyr Ile Pro
            100                 105                 110

Ala Lys Gln Gly Leu Gln Gly Ala Gln Met Gly Gln Pro Trp Cys Phe
        115                 120                 125

Phe Pro Pro Ser Tyr Pro Ser Tyr Lys Leu Glu Asn Leu Ser Ser Ser
    130                 135                 140

Glu Met Gly Tyr Thr Ala Thr Leu Thr Arg Thr Thr Pro Thr Phe Phe
145                 150                 155                 160

Pro Lys Asp Ile Leu Thr Leu Arg Leu Asp Val Met Met Glu Thr Glu
                165                 170                 175

Asn Arg Leu His Phe Thr Ile Lys Asp Pro Ala Asn Arg Arg Tyr Glu
            180                 185                 190

Val Pro Leu Glu Thr Pro Arg Val His Ser Arg Ala Pro Ser Pro Leu
        195                 200                 205

Tyr Ser Val Glu Phe Ser Glu Glu Pro Phe Gly Val Ile Val His Arg
    210                 215                 220

Gln Leu Asp Gly Arg Val Leu Leu Asn Thr Thr Val Ala Pro Leu Phe
225                 230                 235                 240

Phe Ala Asp Gln Phe Leu Gln Leu Ser Thr Ser Leu Pro Ser Gln Tyr
                245                 250                 255

Ile Thr Gly Leu Ala Glu His Leu Ser Pro Leu Met Leu Ser Thr Ser
            260                 265                 270

Trp Thr Arg Ile Thr Leu Trp Asn Arg Asp Leu Ala Pro Thr Pro Gly
        275                 280                 285

Ala Asn Leu Tyr Gly Ser His Pro Phe Tyr Leu Ala Leu Glu Asp Gly
    290                 295                 300

Gly Ser Ala His Gly Val Phe Leu Leu Asn Ser Asn Ala Met Asp Val
305                 310                 315                 320

Val Leu Gln Pro Ser Pro Ala Leu Ser Trp Arg Ser Thr Gly Gly Ile
                325                 330                 335

Leu Asp Val Tyr Ile Phe Leu Gly Pro Glu Pro Lys Ser Val Val Gln
            340                 345                 350

Gln Tyr Leu Asp Val Val Gly Tyr Pro Phe Met Pro Pro Tyr Trp Gly
        355                 360                 365

Leu Gly Phe His Leu Cys Arg Trp Gly Tyr Ser Ser Thr Ala Ile Thr
    370                 375                 380

Arg Gln Val Val Glu Asn Met Thr Arg Ala His Phe Pro Leu Asp Val
385                 390                 395                 400

Gln Trp Asn Asp Leu Asp Tyr Met Asp Ser Arg Arg Asp Phe Thr Phe
                405                 410                 415

Asn Lys Asp Gly Phe Arg Asp Phe Pro Ala Met Val Gln Glu Leu His
            420                 425                 430

Gln Gly Gly Arg Arg Tyr Met Met Ile Val Asp Pro Ala Ile Ser Ser
        435                 440                 445

Ser Gly Pro Ala Gly Ser Tyr Arg Pro Tyr Asp Glu Gly Leu Arg Arg
    450                 455                 460

Gly Val Phe Ile Thr Asn Glu Thr Gly Gln Pro Leu Ile Gly Lys Val
465                 470                 475                 480

Trp Pro Gly Ser Thr Ala Phe Pro Asp Phe Thr Asn Pro Thr Ala Leu
                485                 490                 495

Ala Trp Trp Glu Asp Met Val Ala Glu Phe His Asp Gln Val Pro Phe
            500                 505                 510

Asp Gly Met Trp Ile Asp Met Asn Glu Pro Ser Asn Phe Ile Arg Gly
        515                 520                 525

Ser Glu Asp Gly Cys Pro Asn Asn Glu Leu Glu Asn Pro Pro Tyr Val
    530                 535                 540

Pro Gly Val Val Gly Gly Thr Leu Gln Ala Ala Thr Ile Cys Ala Ser
545                 550                 555                 560

Ser His Gln Phe Leu Ser Thr His Tyr Asn Leu His Asn Leu Tyr Gly
                565                 570                 575

Leu Thr Glu Ala Ile Ala Ser His Arg Ala Leu Val Lys Ala Arg Gly
            580                 585                 590

Thr Arg Pro Phe Val Ile Ser Arg Ser Thr Phe Ala Gly His Gly Arg
        595                 600                 605

Tyr Ala Gly His Trp Thr Gly Asp Val Trp Ser Ser Trp Glu Gln Leu

610                 615                 620

Ala Ser Ser Val Pro Glu Ile Leu Gln Phe Asn Leu Leu Gly Val Pro
625                 630                 635                 640

Leu Val Gly Ala Asp Val Cys Gly Phe Leu Gly Asn Thr Ser Glu Glu
                645                 650                 655

Leu Cys Val Arg Trp Thr Gln Leu Gly Ala Phe Tyr Pro Phe Met Arg
            660                 665                 670

Asn His Asn Ser Leu Leu Ser Leu Pro Gln Glu Pro Tyr Ser Phe Ser
        675                 680                 685

Glu Pro Ala Gln Gln Ala Met Arg Lys Ala Leu Thr Leu Arg Tyr Ala
    690                 695                 700

Leu Leu Pro His Leu Tyr Thr Leu Phe His Gln Ala His Val Ala Gly
705                 710                 715                 720

Glu Thr Val Ala Arg Pro Leu Phe Leu Glu Phe Pro Lys Asp Ser Ser
                725                 730                 735

Thr Trp Thr Val Asp His Gln Leu Leu Trp Gly Glu Ala Leu Leu Ile
            740                 745                 750

Thr Pro Val Leu Gln Ala Gly Lys Ala Glu Val Thr Gly Tyr Phe Pro
        755                 760                 765

Leu Gly Thr Trp Tyr Asp Leu Gln Thr Val Pro Ile Glu Ala Leu Gly
    770                 775                 780

Ser Leu Pro Pro Pro Pro Ala Ala Pro Arg Glu Pro Ala Ile His Ser
785                 790                 795                 800

Glu Gly Gln Trp Val Thr Leu Pro Ala Pro Leu Asp Thr Ile Asn Val
                805                 810                 815

His Leu Arg Ala Gly Tyr Ile Ile Pro Leu Gln Gly Pro Gly Leu Thr
            820                 825                 830

Thr Thr Glu Ser Arg Gln Gln Pro Met Ala Leu Ala Val Ala Leu Thr
        835                 840                 845

Lys Gly Gly Glu Ala Arg Gly Glu Leu Phe Trp Asp Asp Gly Glu Ser
    850                 855                 860

Leu Glu Val Leu Glu Arg Gly Ala Tyr Thr Gln Val Ile Phe Leu Ala
865                 870                 875                 880

Arg Asn Asn Thr Ile Val Asn Glu Leu Val Arg Val Thr Ser Glu Gly
                885                 890                 895

Ala Gly Leu Gln Leu Gln Lys Val Thr Val Leu Gly Val Ala Thr Ala
            900                 905                 910

Pro Gln Gln Val Leu Ser Asn Gly Val Pro Val Ser Asn Phe Thr Tyr
        915                 920                 925

Ser Pro Asp Thr Lys Val Leu Asp Ile Cys Val Ser Leu Leu Met Gly
    930                 935                 940

Glu Gln Phe Leu Val Ser Trp Cys
945                 950

<210>  31
<211>  957
<212>  PRT
<213>  homo sapiens

<400>  31

<210>  32
<211>  952
<212>  PRT
<213>  homo sapiens

<400>  32

<210>  33
<211>  925
<212>  PRT
<213>  artificial

<220>
<223>  variant hGAAwt w/o sp

<400>  33

Gly His Ile Leu Leu His Asp Phe Leu Leu Val Pro Arg Glu Leu Ser
1               5                   10                  15

Gly Ser Ser Pro Val Leu Glu Glu Thr His Pro Ala His Gln Gln Gly
            20                  25                  30

Ala Ser Arg Pro Gly Pro Arg Asp Ala Gln Ala His Pro Gly Arg Pro
        35                  40                  45

Arg Ala Val Pro Thr Gln Cys Asp Val Pro Pro Asn Ser Arg Phe Asp
    50                  55                  60

Cys Ala Pro Asp Lys Ala Ile Thr Gln Glu Gln Cys Glu Ala Arg Gly
65                  70                  75                  80

Cys Cys Tyr Ile Pro Ala Lys Gln Gly Leu Gln Gly Ala Gln Met Gly
                85                  90                  95

Gln Pro Trp Cys Phe Phe Pro Pro Ser Tyr Pro Ser Tyr Lys Leu Glu
            100                 105                 110

Asn Leu Ser Ser Ser Glu Met Gly Tyr Thr Ala Thr Leu Thr Arg Thr
        115                 120                 125

Thr Pro Thr Phe Phe Pro Lys Asp Ile Leu Thr Leu Arg Leu Asp Val
    130                 135                 140

Met Met Glu Thr Glu Asn Arg Leu His Phe Thr Ile Lys Asp Pro Ala
145                 150                 155                 160

Asn Arg Arg Tyr Glu Val Pro Leu Glu Thr Pro Arg Val His Ser Arg
                165                 170                 175

Ala Pro Ser Pro Leu Tyr Ser Val Glu Phe Ser Glu Glu Pro Phe Gly
            180                 185                 190

Val Ile Val His Arg Gln Leu Asp Gly Arg Val Leu Leu Asn Thr Thr
        195                 200                 205

Val Ala Pro Leu Phe Phe Ala Asp Gln Phe Leu Gln Leu Ser Thr Ser
    210                 215                 220

Leu Pro Ser Gln Tyr Ile Thr Gly Leu Ala Glu His Leu Ser Pro Leu
225                 230                 235                 240

Met Leu Ser Thr Ser Trp Thr Arg Ile Thr Leu Trp Asn Arg Asp Leu
                245                 250                 255

Ala Pro Thr Pro Gly Ala Asn Leu Tyr Gly Ser His Pro Phe Tyr Leu
            260                 265                 270

Ala Leu Glu Asp Gly Gly Ser Ala His Gly Val Phe Leu Leu Asn Ser
        275                 280                 285

Asn Ala Met Asp Val Val Leu Gln Pro Ser Pro Ala Leu Ser Trp Arg
    290                 295                 300

Ser Thr Gly Gly Ile Leu Asp Val Tyr Ile Phe Leu Gly Pro Glu Pro
305                 310                 315                 320

Lys Ser Val Val Gln Gln Tyr Leu Asp Val Val Gly Tyr Pro Phe Met
                325                 330                 335

Pro Pro Tyr Trp Gly Leu Gly Phe His Leu Cys Arg Trp Gly Tyr Ser
            340                 345                 350

Ser Thr Ala Ile Thr Arg Gln Val Val Glu Asn Met Thr Arg Ala His
        355                 360                 365

Phe Pro Leu Asp Val Gln Trp Asn Asp Leu Asp Tyr Met Asp Ser Arg
    370                 375                 380

Arg Asp Phe Thr Phe Asn Lys Asp Gly Phe Arg Asp Phe Pro Ala Met
385                 390                 395                 400

Val Gln Glu Leu His Gln Gly Gly Arg Arg Tyr Met Met Ile Val Asp
                405                 410                 415

Pro Ala Ile Ser Ser Ser Gly Pro Ala Gly Ser Tyr Arg Pro Tyr Asp
            420                 425                 430

Glu Gly Leu Arg Arg Gly Val Phe Ile Thr Asn Glu Thr Gly Gln Pro
        435                 440                 445

Leu Ile Gly Lys Val Trp Pro Gly Ser Thr Ala Phe Pro Asp Phe Thr
    450                 455                 460

Asn Pro Thr Ala Leu Ala Trp Trp Glu Asp Met Val Ala Glu Phe His
465                 470                 475                 480

Asp Gln Val Pro Phe Asp Gly Met Trp Ile Asp Met Asn Glu Pro Ser
                485                 490                 495

Asn Phe Ile Arg Gly Ser Glu Asp Gly Cys Pro Asn Asn Glu Leu Glu
            500                 505                 510

Asn Pro Pro Tyr Val Pro Gly Val Val Gly Gly Thr Leu Gln Ala Ala
        515                 520                 525

Thr Ile Cys Ala Ser Ser His Gln Phe Leu Ser Thr His Tyr Asn Leu
    530                 535                 540

His Asn Leu Tyr Gly Leu Thr Glu Ala Ile Ala Ser His Arg Ala Leu
545                 550                 555                 560

Val Lys Ala Arg Gly Thr Arg Pro Phe Val Ile Ser Arg Ser Thr Phe
                565                 570                 575

Ala Gly His Gly Arg Tyr Ala Gly His Trp Thr Gly Asp Val Trp Ser
            580                 585                 590

Ser Trp Glu Gln Leu Ala Ser Ser Val Pro Glu Ile Leu Gln Phe Asn
        595                 600                 605

Leu Leu Gly Val Pro Leu Val Gly Ala Asp Val Cys Gly Phe Leu Gly
    610                 615                 620

Asn Thr Ser Glu Glu Leu Cys Val Arg Trp Thr Gln Leu Gly Ala Phe
625                 630                 635                 640

Tyr Pro Phe Met Arg Asn His Asn Ser Leu Leu Ser Leu Pro Gln Glu
                645                 650                 655

Pro Tyr Ser Phe Ser Glu Pro Ala Gln Gln Ala Met Arg Lys Ala Leu

660                 665                 670

Thr Leu Arg Tyr Ala Leu Leu Pro His Leu Tyr Thr Leu Phe His Gln
        675                 680                 685

Ala His Val Ala Gly Glu Thr Val Ala Arg Pro Leu Phe Leu Glu Phe
    690                 695                 700

Pro Lys Asp Ser Ser Thr Trp Thr Val Asp His Gln Leu Leu Trp Gly
705                 710                 715                 720

Glu Ala Leu Leu Ile Thr Pro Val Leu Gln Ala Gly Lys Ala Glu Val
                725                 730                 735

Thr Gly Tyr Phe Pro Leu Gly Thr Trp Tyr Asp Leu Gln Thr Val Pro
            740                 745                 750

Ile Glu Ala Leu Gly Ser Leu Pro Pro Pro Pro Ala Ala Pro Arg Glu
        755                 760                 765

Pro Ala Ile His Ser Glu Gly Gln Trp Val Thr Leu Pro Ala Pro Leu
    770                 775                 780

Asp Thr Ile Asn Val His Leu Arg Ala Gly Tyr Ile Ile Pro Leu Gln
785                 790                 795                 800

Gly Pro Gly Leu Thr Thr Thr Glu Ser Arg Gln Gln Pro Met Ala Leu
                805                 810                 815

Ala Val Ala Leu Thr Lys Gly Gly Glu Ala Arg Gly Glu Leu Phe Trp
            820                 825                 830

Asp Asp Gly Glu Ser Leu Glu Val Leu Glu Arg Gly Ala Tyr Thr Gln
        835                 840                 845

Val Ile Phe Leu Ala Arg Asn Asn Thr Ile Val Asn Glu Leu Val Arg
    850                 855                 860

Val Thr Ser Glu Gly Ala Gly Leu Gln Leu Gln Lys Val Thr Val Leu
865                 870                 875                 880

Gly Val Ala Thr Ala Pro Gln Gln Val Leu Ser Asn Gly Val Pro Val
                885                 890                 895

Ser Asn Phe Thr Tyr Ser Pro Asp Thr Lys Val Leu Asp Ile Cys Val
            900                 905                 910

Ser Leu Leu Met Gly Glu Gln Phe Leu Val Ser Trp Cys
        915                 920                 925

## Description

The present invention relates to variants of acid-alpha glucosidase and uses thereof.

Pompe disease, also known as glycogen storage disease (GSD) type II and acid maltase deficiency, is an autosomal recessive metabolic myopathy caused by a deficiency of the lysosomal enzyme acid alpha-glucosidase (GAA). GAA is an exo-1,4 and 1,6-α-glucosidase that hydrolyzes glycogen to glucose in the lysosome. Deficiency of GAA leads to glycogen accumulation in lysosomes and causes progressive damage to respiratory, cardiac, and skeletal muscle. The disease ranges from a rapidly progressive infantile course that is usually fatal by 1-2 years of age to a more slowly progressive and heterogeneous course that causes significant morbidity and early mortality in children and adults. Hirschhorn RR, The Metabolic and Molecular Bases of Inherited Disease, 3: 3389-3420 (2001, McGraw-Hill); Van der Ploeg and Reuser, Lancet 372: 1342-1351 (2008).

Current human therapy for treating Pompe disease involves administration of recombinant human GAA, otherwise termed enzyme-replacement therapy (ERT). ERT has demonstrated efficacy for severe, infantile GSD II. However the benefit of enzyme therapy is limited by the need for frequent infusions and the development of inhibitor antibodies against recombinant hGAA (Amalfitano, A., et al. (2001) Genet. In Med. 3:132-138). Furthermore, ERT does not correct efficiently the entire body, probably because of a combination of poor biodistribution of the protein following peripheral vein delivery, lack of uptake from several tissues, and high immunogenicity. As an alternative or adjunct to ERT, the feasibility of gene therapy approaches to treat GSD-II have been investigated (Amalfitano, A., et al. (1999) Proc. Natl. Acad. Sci. USA 96:8861-8866, Ding, E., et al. (2002) Mol. Ther. 5:436-446, Fraites, T. J., et al. (2002) Mol. Ther. 5:571-578, Tsujino, S., et al. (1998) Hum. Gene Ther. 9:1609-1616). However, muscle-directed gene transfer to correct the genetic defect has to face the limitation of the systemic nature of the disease and the fact that muscle expression of a transgene tends to be more immunogenic compared with other tissues.

Doerfler et al., 2016 describe the combined administration of two constructs encoding a human codon-optimized GAA, one under the control of a liver specific promoter and the other one under the control of a muscle-specific promoter. Liver-specific promoter driven expression of GAA is employed to promote immune tolerance to GAA in a Gaa^{-/-} mouse model, while muscle-specific promoter driven expression of GAA provides expression of the therapeutic protein in part of the tissues targeted for therapy. However, this strategy is not entirely satisfactory in that it requires the use of multiple constructs and it does not result in body wide expression of GAA.

Modified GAA proteins have been proposed in the past to improve lysosomal storage disease treatment. In particular, application WO2004064750 and Sun et al. 2006, disclose a chimeric GAA polypeptide comprising a signal peptide operably linked to GAA as a way to enhance targeting of the protein to the secretory pathway.

However, therapies available to the patient are not entirely satisfactory and improved GAA polypeptides and GAA production is still a need in the art. In particular, a need still exists of a long term efficacy of the treatment with GAA, of high level GAA production, of improved immunological tolerance to the produced GAA polypeptide, and of improved uptake of GAA by the cells and tissues in need thereof. In addition, in WO2004064750 and Sun et al., 2006, tissue distribution of the chimeric GAA polypeptide disclosed therein is not entirely satisfactory. Therefore, a need still exists for a GAA polypeptide that would be fully therapeutic, by allowing a correction of glycogen accumulation in most if not all tissues of interest.

### SUMMARY OF THE INVENTION

The present invention relates to GAA variants that are expressed and secreted at higher levels compared to the wild type GAA protein and that elicit improved correction of the pathological accumulation of glycogen body-wide and results in the induction of immunological tolerance to GAA.

According to one aspect, the invention relates to a truncated GAA polypeptide, comprising a deletion of at least one amino acid from the N-terminal end of a parent GAA polypeptide, wherein the parent polypeptide corresponds to a precursor form of a GAA polypeptide devoid of its signal peptide. In a particular embodiment, said truncated GAA polypeptide has 1 to 75 consecutive amino acids deleted at its N-terminal end as compared to the parent GAA polypeptide. In a more particular embodiment said truncated GAA polypeptide has 6, 7, 8, 9, 10, 40, 41, 42, 43 or 44 consecutive amino acids deleted at its N-terminal end as compared to a parent GAA polypeptide, in particular 8 or 42 consecutive amino acids truncated at its N-terminal end as compared to a parent GAA polypeptide. In a further particular embodiment, the parent polypeptide is a human GAA (hGAA), in particular a hGAA having the amino acid sequence shown in SEQ ID NO:1 or SEQ ID NO:33, in particular SEQ ID NO:1.

In a particular embodiment, the truncated GAA polypeptide of the invention has the sequence shown in SEQ ID NO:27 or SEQ ID NO:28.

Moreover, the truncated GAA polypeptide of the invention may further comprise a signal peptide fused to its N-terminal end, in particular a signal peptide selected in the group consisting of SEQ ID NO:3 to 7, in particular the signal peptide of SEQ ID NO:3.

In another aspect, the invention relates to a nucleic acid molecule encoding a truncated GAA polypeptide as described above, optionally fused to a signal peptide via its N-terminal end. In some embodiments, the nucleic acid molecule has a nucleotide sequence optimized to improve the expression of and/or improve immune tolerance to the truncated GAA polypeptide *in vivo,* in particular in a human subject.

In yet another aspect, the invention relates to a nucleic acid construct, comprising the nucleic acid molecule of the invention operably linked to one or more regulatory sequences such as a promoter, an intron, a polyadenylation signal and/or an enhancer (for example a cis-regulatory module, or CRM). In a particular embodiment, the promoter is a liver-specific promoter preferably selected in the group consisting of the alpha-1 antitrypsin promoter (hAAT), the transthyretin promoter, the albumin promoter and the thyroxine-binding globulin (TBG) promoter. In another particular embodiment, the promoter is a muscle-specific promoter, such as the Spc5-12, MCK and desmin promoters. In another embodiment, the promoter is an ubiquitous promoter such as the CMV, CAG and PGK promoters. The nucleic acid construct may further optionally comprises an intron, in particular an intron selected in the group consisting of a human beta globin b2 (or HBB2) intron, a FIX intron, a chicken beta-globin intron and a SV40 intron, wherein said intron is optionally a modified intron such as a modified HBB2 intron of SEQ ID NO:17, a modified FIX intron of SEQ ID NO:19, or a modified chicken beta-globin intron of SEQ ID NO:21. In a particular embodiment of the nucleic acid construct of the invention, said construct comprises, preferably in this order,: an enhancer; an intron; a promoter, in particular a liver-specific promoter; the nucleic acid sequence encoding the GAA protein; and a polyadenylation signal, the construct comprising preferably, in this order: an ApoE control region; a HBB2 intron, in particular a modified HBB2 intron; a hAAT promoter; the nucleic acid sequence encoding the truncated GAA polypeptide; and a bovine growth hormone polyadenylation signal. In specific embodiments, said nucleic acid construct more particularly comprises the nucleotide sequence of any one of SEQ ID NO:22 to 26.

In another aspect, the invention relates to a vector comprising the nucleic acid molecule or the nucleic acid construct herein disclosed. The vector of the invention may be in particular a viral vector, preferably a retroviral vector, such as a lentiviral vector, or an AAV vector. Preferably, the vector is a single-stranded or double-stranded self-complementary AAV vector, preferably an AAV vector with an AAV-derived capsid, such as an AAV1, AAV2, variant AAV2, AAV3, variant AAV3, AAV3B, variant AAV3B, AAV4, AAV5, AAV6, variant AAV6, AAV7, AAV8, AAV9, AAV10 such as AAVcy10 and AAVrh10, AAVrh74, AAVdj, AAV-Anc80, AAV-LK03, AAV2i8, a porcine AAV capsid, such as AAVpo4 and AAVpo6 capsid, or with a chimeric capsid. In a specific embodiment, the vector is an AAV vector with an AAV8, AAV9, AAVrh74 or AAV2i8 capsid, in particular an AAV8, AAV9 or AAVrh74 capsid, more particularly an AAV8 capsid.

In yet another aspect, the invention provides a cell transformed with the nucleic acid molecule, the nucleic acid construct or the vector of the invention. More particularly, the cell is a liver cell or a muscle cell.

In a particular aspect, the invention provides a pharmaceutical composition, comprising, in a pharmaceutically acceptable carrier, the truncated GAA polypeptide, the nucleic acid molecule, the nucleic acid construct, the vector, or the cell of the invention.

The invention further relates to the truncated GAA polypeptide, the nucleic acid molecule, the nucleic acid construct, the vector, or the cell of the invention, for use as a medicament.

The invention further provides the truncated GAA polypeptide, the nucleic acid molecule, the nucleic acid construct, the vector, or the cell of the invention, for use in a method for treating a glycogen storage disease. In a particular embodiment, the glycogen storage disease is GSDI, GSDII, GSDIII, GSDIV, GSDV, GSDVI, GSDVII, GSDVIII or lethal congenital glycogen storage disease of the heart. In a more particular embodiment, the glycogen storage disease is selected in the group consisting of GSDI, GSDII and GSDIII, more particularly in the group consisting of GSDII and GSDIII. In an even more particular embodiment, the glycogen storage disease is GSDII.

### LEGENDS TO THE FIGURES

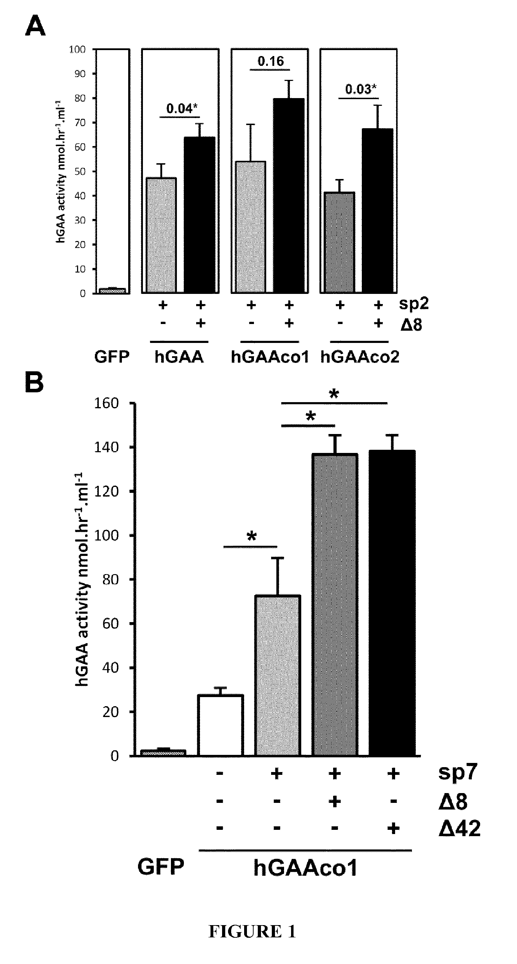

**Figure 1****.** Deletion of portions of hGAA increase its secretion in vitro. **Panel A.** Human hepatoma cells (Huh7) were transfected using Lipofectamine™ with a control plasmid expressing green fluorescent protein (GFP), or plasmids expressing wild-type hGAA (hGAA) or hGAA sequence optimized according to two distinct algorithms (hGAAco1 and co2, respectively). The different hGAA constructs contained the wild-type or the human alpha-1-antitrypsin signal peptide (sp2). Truncated hGAA has been obtained by deletion of 8 amino acids after the signal peptide (Δ8). 48 hours after transfection the activity of hGAA in the culture media was measured by a fluorogenic enzymatic assay and GAA activity evaluated against a standard curve of the product of the reaction as indicated in Materials and Methods. The histogram plot shows the average ± SE of the levels of secreted hGAA deriving from three different experiments. Statistical analysis has been performed by paired t-test, in the histogram are reported the p-values obtained (* = p<0.05 as indicated). **Panel B.** Human hepatoma cells (Huh7) were transfected using lipofectamine with a control plasmid expressing GFP, or plasmids expressing hGAAco1 with wild-type or chymotrypsinogen B1 signal peptide (sp7). hGAA protein has been truncated by removing 8 or 42 amino acids after the signal peptide (Δ8 and Δ42, respectively). 48 hours after transfection the activity of hGAA in the culture media was measured by a fluorogenic enzymatic assay as indicated above. The histogram plot shows the average ± SE of the levels of secreted hGAA deriving from three different experiments. Statistical analysis has been performed by ANOVA (* = p<0.05 as indicated).

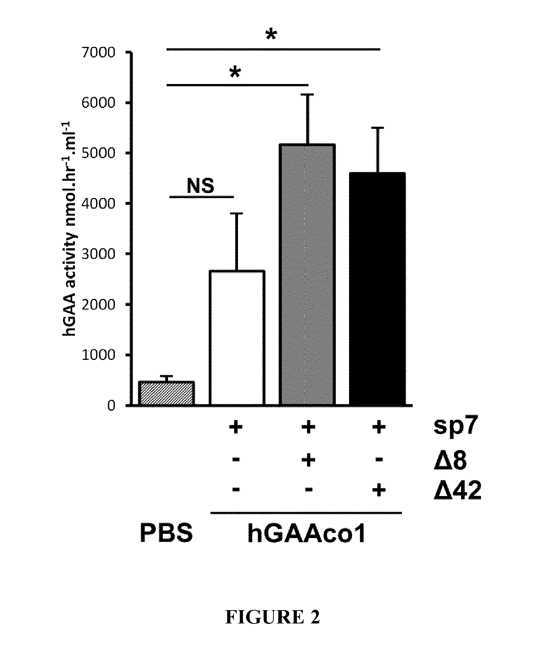

**Figure 2****.** Deletion of portions of hGAA increases its secretion in the bloodstream in a Pompe disease mouse model. 3 months-old GAA^{-/-} mice (n=4-5 mice/group) were intravenously injected with PBS or with 2E12 vg/kg of AAV8 vectors expressing sequence optimized hGAA (hGAAco1) under the transcriptional control of a liver specific promoter. Wild-type signal peptide of hGAA has been substituted with chymotrypsinogen B1 signal peptide (sp7) and the sequence of hGAA has been either used as the full-length native sequence or truncated by removing 8 or 42 amino acids after the signal peptide (Δ8 and Δ42, respectively). One month after the injection, mice were bled and hGAA activity was measured using a fluorogenic assay in serum. Statistical analysis was performed by ANOVA (* = p<0.05 as indicated).

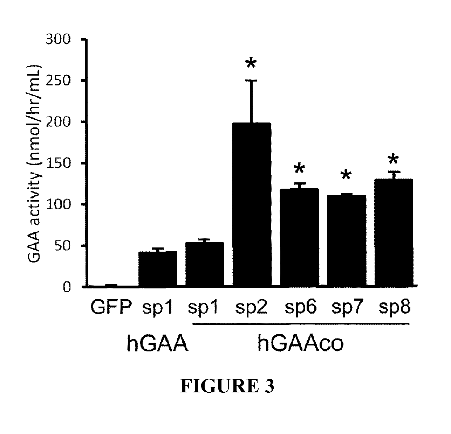

**Figure 3****.** Signal peptides enhance secretion of hGAA. Human hepatoma cells (Huh7) were transfected by Lipofectamine™ with a control plasmid (GFP), a plasmid expressing wild-type hGAA under the transcriptional control of a liver specific promoter (noted as sp1), or plasmids expressing sequence optimized Δ8 hGAA (hGAAco) fused with signal peptides 6-8 (sp6-8). 48 hours after transfection the activity of hGAA in the culture media was measured by a fluorogenic enzymatic assay and GAA activity evaluated against a standard curve of 4-methylumbelliferone. The histogram plot shows the average ± SE of the levels of secreted hGAA deriving from three different experiments. Statistical analysis has been performed by ANOVA (* = p<0.05 vs mock transfected cells).

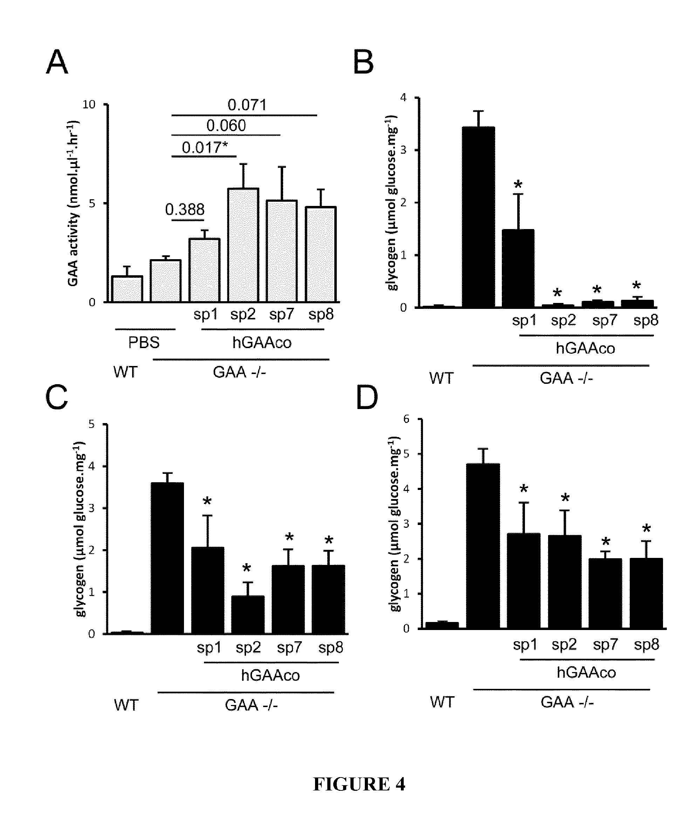

**Figure 4****.** Truncated Δ8 hGAA efficiently correct glycogen accumulation in a Pompe disease mouse model. 4 months-old wild type (WT) and GAA^{-/-} mice (n=4-5 mice/group) were intravenously injected with PBS or 6E11 vg/kg of AAV8 vectors expressing sequence optimized Δ8 hGAA (hGAAco) under the transcriptional control of human alpha-1-antytripsin promoter and fused with signal peptide 1, 2, 7 and 8 (sp1,2,7,8). **Panel A.** The histogram shows the hGAA activity measured by fluorogenic assay in blood three months after vectors injection. Statistical analysis has been performed by ANOVA, in the histogram are reported the p-values obtained vs PBS treated GAA -/- animals (* = p<0.05). **Panel B-D.** Biochemical correction of glycogen content in heart, diaphragm and quadriceps. 4 months-old GAA^{-/-} mice were treated as described above. Three months after the injections, mice were sacrificed and the glycogen content has been evaluated. Histograms show the glycogen content expressed as glucose released after enzymatic digestion of glycogen, measured in the heart **(panel B),** diaphragm **(panel C)** and quadriceps **(panel D).** Statistical analysis has been performed by ANOVA (*=p<0.05 vs PBS injected GAA -/- mice).

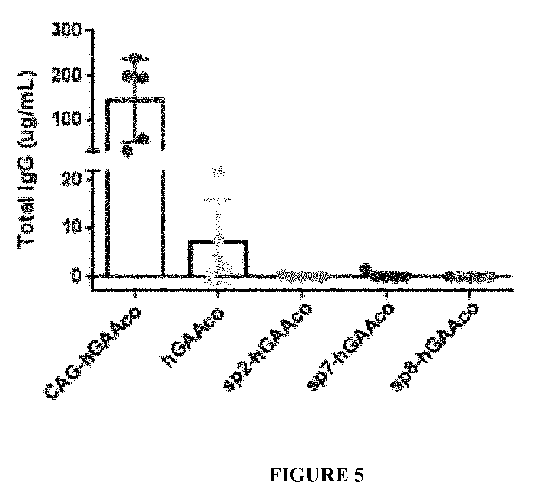

**Figure 5****.** Highly secreted hGAA reduces humoral response in in a Pompe disease mouse model. 4 months-old GAA-/- mice were intravenously injected with PBS or 2E12 vg/kg of AAV8 vectors expressing sequence optimized Δ8 hGAA (hGAAco) under the transcriptional control of human alpha-1-antytripsin promoter and fused with signal peptide 1, 2, 7 and 8 (sp1,2,7,8). 1 month after the injections, sera were analyzed for the presence of anti-hGAA antibodies by ELISA. The quantification has been performed using purified mouse IgG as standard. Statistical analysis has been performed by ANOVA (* = p<0.05)

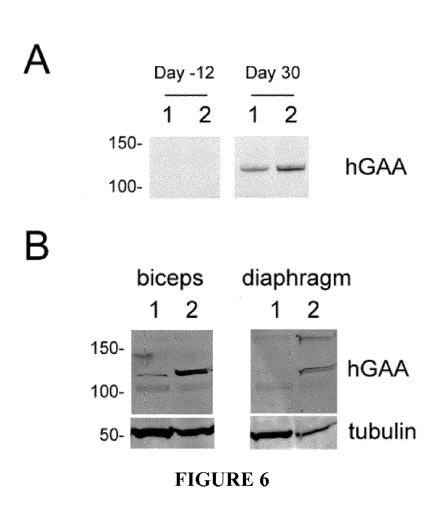

**Figure 6****.** AAV8-hAAT-sp7-Δ8-hGAAco1 injection leads to efficacious secretion of hGAA in the blood and uptake in muscle in NHP. Two *Macaca Fascicularis* monkeys were injected at day 0 with 2E12 vg/kg of AAV8-hAAT-sp7-Δ8-hGAAco1.**Panel A** hGAA western blot performed on serum from the two monkeys obtained twelve days before and 30 days after vector administration. On the left are indicated the positions of the bands of the molecular weight marker running in parallel with the samples. **Panel B** Three months after vector injection the monkeys were sacrificed and tissues harvested for biochemical evaluation of hGAA uptake. A hGAA Western blot was performed on tissue extracts obtained from biceps and diaphragm. An anti-tubulin antibody was used as loading control. On the left are indicated the positions of the bands of the molecular weight marker running in parallel with the samples.

### DETAILED DESCRIPTION OF THE INVENTION

The present invention relates to a truncated GAA polypeptide, to a nucleic acid molecule encoding such a truncated GAA polypeptide, to a nucleic acid construct comprising said nucleic acid, to a vector comprising said nucleic acid construct, to a cell comprising said nucleic acid molecule or construct or vector, and to a pharmaceutical composition comprising a polypeptide, a nucleic acid molecule, a nucleic acid construct, a vector or a cell according to the invention. The inventors have surprisingly shown that a truncated form of GAA according to the invention greatly improves GAA secretion while reducing its immunogenicity.

Lysosomal acid α-glucosidase or "GAA" (E.C. 3.2. 1.20) (1,4-α-D-glucan glucohydrolase), is an exo-1,4-α-D-glucosidase that hydrolyses both α-1,4 and α-1,6 linkages of oligosaccharides to liberate glucose. A deficiency in GAA results in glycogen storage disease type II (GSDII), also referred to as Pompe disease (although this term formally refers to the infantile onset form of the disease). It catalyzes the complete degradation of glycogen with slowing at branching points. The 28 kb human acid α-glucosidase gene on chromosome 17 encodes a 3.6 kb mRNA which produces a 951 amino acid polypeptide (Hoefsloot et al., (1988) EMBO J. 7: 1697; Martiniuk et al., (1990) DNA and Cell Biology 9: 85). The enzyme receives co-translational N-linked glycosylation in the endoplasmic reticulum. It is synthesized as a 110-kDa precursor form, which matures by extensive glycosylation modification, phosphorylation and by proteolytic processing through an approximately 90-kDa endosomal intermediate into the final lysosomal 76 and 67 kDa forms (Hoefsloot, (1988) EMBO J. 7: 1697; Hoefsloot et al., (1990) Biochem. J. 272: 485; Wisselaar et al., (1993) J. Biol. Chem. 268: 2223; Hermans et al., (1993) Biochem. J. 289: 681).

In patients with GSD II, a deficiency of acid α-glucosidase causes massive accumulation of glycogen in lysosomes, disrupting cellular function (Hirschhorn, R. and Reuser, A. J. (2001), in The Metabolic and Molecular Basis for Inherited Disease, (eds, Scriver, C. R. et al.) pages 3389-3419 (McGraw-Hill, New York). In the most common infantile form, patients exhibit progressive muscle degeneration and cardiomyopathy and die before two years of age. Severe debilitation is present in the juvenile and adult onset forms.

Furthermore, patients having other GSDs may benefit from the administration of an optimized form of GAA. For example, it has been shown (Sun et al. (2013) Mol Genet Metab 108(2): 145; WO2010/005565) that administration of GAA reduces glycogen in primary myoblasts from glycogen storage disease type III (GSD III) patients.

In particular, in the context of the present invention, a "precursor form of GAA" is a form of the GAA polypeptide that comprises its natural signal peptide. For example, the sequence of SEQ ID NO:2 is the precursor form of human GAA (hGAA). Within SEQ ID NO:2, amino acid residues 1-27 correspond to the signal peptide of the hGAA polypeptide. This sequence of the signal peptide of hGAA is also represented in SEQ ID NO:4.

In the context of the present invention, the truncated GAA polypeptide of the invention is derived from a parent GAA polypeptide. According to the present invention a "parent GAA polypeptide" is a functional, precursor GAA sequence as defined above, but devoid of its signal peptide. For example, with reference to the typical wild-type human GAA polypeptide, a complete wild-type GAA polypeptide (i.e. a precursor form of GAA) is represented in SEQ ID NO:2 or in SEQ ID NO:30 and has a signal peptide (corresponding to amino acids 1-27 of SEQ ID NO:2 or SEQ ID NO:30), whereas the parent GAA polypeptide serving as basis for the truncated GAA forms of these wild-type human GAA polypeptides are represented in SEQ ID NO:1 and SEQ ID NO:33, respectively and have no signal peptide. In this example, the latter, corresponding to amino acids 28-952 of SEQ ID NO:2 and to amino acids 28-952 of SEQ ID NO:30, is referred to as a parent GAA polypeptide.

According to the invention, the truncated GAA polypeptide of the invention is a functional GAA polypeptide, i.e. it has the functionality of wild-type GAA polypeptide. As defined above, the functionality of wild-type GAA is to hydrolyse both α-1,4 and α-1,6 linkages of oligosaccharides and polysaccharides, more particularly of glycogen, to liberate glucose. The functional GAA protein encoded by the nucleic acid of the invention may have a hydrolysing activity on glycogen of at least 50 %, 60 %, 70 %, 80 %, 90 %, 95 %, 99 %, or at least 100 % as compared to the wild-type GAA polypeptide of SEQ ID NO:1 or SEQ ID NO:33. The activity of the GAA protein encoded by the nucleic acid of the invention may even be of more than 100 %, such as of more than 110 %, 120 %, 130 %, 140 %, or even more than 150 % of the activity of the wild-type GAA protein of SEQ ID NO:1 or of SEQ ID NO:33.

The amino acid sequence of the parent GAA polypeptide or its coding sequence can be derived from any source, including avian and mammalian species. The term "avian" as used herein includes, but is not limited to, chickens, ducks, geese, quail, turkeys and pheasants. The term "mammal" as used herein includes, but is not limited to, humans, simians and other non-human primates, bovines, ovines, caprines, equines, felines, canines, lagomorphs, etc. In embodiments of the invention, the parent GAA polypeptide is a human, mouse or quail, in particular a human, GAA polypeptide.

In addition, the parent GAA polypeptide may be a functional variant of a GAA polypeptide, comprising one or more amino acid modifications such as amino acid insertion, deletion and/or substitution as compared to a GAA polypeptide. For example, the parent polypeptide may be a functional derivative of a human GAA polypeptide, such as the polypeptide of SEQ ID NO:1 or SEQ ID NO:33, in particular SEQ ID NO:1, having at least 80, 85, 90, 95, 96, 97, 98 or at least 99 percent sequence identity to this human GAA polypeptide. For example, in addition to the truncation defined above, the functional variant of a GAA polypeptide may have between 0 and 50, between 0 and 30, between 0 and 20, between 0 and 15, between 0 and 10, or between 0 and 5 amino acid changes to the parent GAA polypeptide, such as the parent GAA polypeptide shown in SEQ ID NO: 1 or SEQ ID NO:33, in particular SEQ ID NO:1. In particular, the parent GAA polypeptide may consist of the human GAA polypeptide having the amino acid sequence shown in SEQ ID NO:1 or SEQ ID NO:33, in particular in SEQ ID NO: 1.

The parent GAA polypeptide may also be a GAA variant such as GAA II as described by Kunita et al., (1997) Biochemica et Biophysica Acta 1362: 269; GAA polymorphisms and SNPs are described by Hirschhorn, R. and Reuser, A. J. (2001) In The Metabolic and Molecular Basis for Inherited Disease (Scriver, C. R. , Beaudet, A. L., Sly, W. S. & Valle, D. Eds. ), pp. 3389- 3419. McGraw-Hill, New York, see pages 3403-3405. Any variant GAA polypeptide known in the art may be used as a basis for defining a parent GAA polypeptide. Illustrative variant GAA polypeptides include SEQ ID NO:2 (NCBI reference sequence NP_000143.2); SEQ ID NO:29 (GenBank AAA52506.1); SEQ ID NO:30 (GenBank CAA68763.1); SEQ ID NO:31 (GenBank: EAW89583.1) and SEQ ID NO:32 (GenBank ABI53718.1). Other useful variants include those described in Hoefsloot et al., (1988) EMBO J. 7: 1697; and Van Hove et al., (1996) Proc. Natl. Acad. Sci. USA 93: 65 (human) and GenBank Accession number NM_008064 (mouse). Other variant GAA polypeptides include those described in WO2012/145644, WO00/34451 and US6,858,425. In a particular embodiment, the parent GAA polypeptide is derived from the amino acid sequence shown in SEQ ID NO:2 or SEQ ID NO:30.

The truncated form of GAA according to the invention is a N-terminally truncated form of a parent GAA polypeptide, wherein at least one amino acid is deleted from the N-terminal end of said parent GAA polypeptide.

By "truncated form", it is meant a GAA polypeptide that comprises one or several consecutive amino acids deleted from the N-terminal part of a parent GAA polypeptide. For example, the GAA moiety may have 1 to 75 consecutive amino acids or more than 75 consecutive amino acids truncated from its N-terminal end as compared to the parent GAA polypeptide. Specifically, the truncated GAA polypeptide may have 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74 or 75 consecutive amino acids truncated from its N-terminal end as compared to the parent GAA protein (in particular a truncated form of the parent hGAA protein shown in SEQ ID NO: 1). Using an alternative nomenclature, the GAA polypeptide resulting from the truncation of 1 amino acid in the parent GAA polypeptide is referred to as Δ1 GAA truncated form, the GAA polypeptide resulting from the truncation of 2 consecutive amino acids from the N-terminal end is referred to as Δ2 GAA truncated form, the GAA polypeptide resulting from the truncation of 3 consecutive amino acids in the parent GAA polypeptide is referred to as Δ3 GAA truncated form), etc. In a particular embodiment, the truncated GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ9, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form (in particular a truncated form of the parent hGAA protein shown in SEQ ID NO: 1). In a further particular embodiment, the truncated GAA polypeptide of the invention is a Δ6, Δ7, Δ8, Δ9 or Δ10 truncated form of GAA (in particular of the hGAA protein shown in SEQ ID NO: 1), in particular a Δ8 truncated form of GAA (in particular of the hGAA protein shown in SEQ ID NO: 1). In another particular embodiment, the truncated GAA polypeptide of the invention is a Δ40, Δ41, Δ42, Δ43 or Δ44 truncated form of GAA (in particular of the hGAA protein shown in SEQ ID NO: 1), in particular a Δ42 truncated form of GAA (in particular of the hGAA protein shown in SEQ ID NO: 1).

In a particular embodiment, of the invention, the truncated GAA polypeptide of the invention is a truncated form of a functional human GAA polypeptide. In a further particular embodiment, the parent hGAA polypeptide is the hGAA polypeptide shown in SEQ ID NO:1 or SEQ ID NO:33, in particular in SEQ ID NO:1. In a variant of this embodiment, the truncated GAA polypeptide of the invention is a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form of a hGAA polypeptide, and more particularly of the hGAA polypeptide shown in SEQ ID NO:1 or SEQ ID NO:33, even more particularly in SEQ ID NO:1, or of a functional variant thereof comprising amino acid substitutions in the sequence shown in SEQ ID NO:1 or SEQ ID NO:33, in particular SEQ ID NO:1, and having at least 75, 80, 85, 90, 91, 92, 93, 94, 95, 96, 97, 98 or 99 percent identity to SEQ ID NO:1 SEQ ID NO:33, in particular SEQ ID NO:1. In a further particular embodiment, the truncated GAA polypeptide of the invention is a Δ6, Δ7, Δ8, Δ9, A10, Δ40, Δ41, Δ42, Δ43 or Δ44, in particular a Δ8 or Δ42 truncated form of a hGAA polypeptide, and more particularly of the hGAA polypeptide shown in SEQ ID NO:1 or SEQ ID NO:33, in particular in SEQ ID NO:1, or of a functional variant thereof comprising amino acid substitutions in the sequence shown in SEQ ID NO:1 or SEQ ID NO:33, in particular in SEQ ID NO:1, and having at least 80, 85, 90, 95, 96, 97, 98 or 99 percent identity to SEQ ID NO:1 or SEQ ID NO:33, in particular in SEQ ID NO:1. In a specific embodiment, the truncated hGAA polypeptide of the invention has an amino acid sequence consisting of the sequence shown in SEQ ID NO:27 or SEQ ID NO:28.

The truncated GAA polypeptide according to the invention may further comprise a signal peptide, such as the natural signal peptide of GAA, or an alternative signal peptide derived from another secreted protein. Non-limiting examples of such signal peptides include those shown in SEQ ID NO:3 to 7. The inventors have surprisingly shown that fusing the truncated GAA polypeptide of the invention to an alternative signal peptide even further enhances its secretion. The invention thereby provides a chimeric GAA polypeptide comprising a signal moiety and a truncated GAA polypeptide moiety, the truncated GAA polypeptide moiety being a truncated GAA polypeptide as defined above. In a particular embodiment, the signal peptide is the natural signal peptide of a GAA, such as the signal peptide of hGAA shown in SEQ ID NO:4. In another embodiment, the signal peptide is an exogenous (or alternative) signal peptide, derived from a protein different from GAA. In a particular embodiment, the alternative signal peptide is selected in the group consisting of SEQ ID NO:3, 5, 6 and 7, or a functional derivative thereof as defined below.

The inventors have shown that the exogenous signal peptide fused to the remainder of the GAA protein increases the secretion of the resulting chimeric GAA polypeptide as compared to the corresponding GAA polypeptide comprising its natural signal peptide. In addition, the truncated GAA polypeptide moiety also increases the secretion of the chimeric GAA polypeptide (including both a signal peptide and a truncated GAA polypeptide) as compared to a chimeric GAA polypeptide comprising the same signal peptide fused to the parent GAA polypeptide.

Particular exogenous signal peptides workable in the present invention include amino acids 1-20 from chymotrypsinogen B2 (SEQ ID NO:3), the signal peptide of human alpha-1-antitrypsin (SEQ ID NO:5), amino acids 1-25 from iduronate-2-sulphatase (SEQ ID NO:6), and amino acids 1-23 from protease C1 inhibitor (SEQ ID NO:7). The signal peptides of SEQ ID NO:3 and SEQ ID NO:5 to SEQ ID NO:7, allow higher secretion of the chimeric GAA protein both *in vitro* and *in vivo* when compared to the GAA comprising its natural signal peptide. In a particular embodiment, the signal peptide has the sequence shown in SEQ ID NO:3 to 7, or is a functional derivative thereof, i.e. a sequence comprising from 1 to 5, in particular from 1 to 4, in particular from 1 to 3, more particularly from 1 to 2, in particular 1 amino acid deletion(s), insertion(s) or substitution(s) as compared to the sequences shown in SEQ ID NO:3 to 7, as long as the resulting sequence corresponds to a functional signal peptide, i.e. a signal peptide that allows secretion of a GAA protein. In a particular embodiment, the signal peptide moiety sequence consists of a sequence selected in the group consisting of SEQ ID NO:3 to 7.

In particular embodiments, the GAA polypeptide of the invention is selected from:
- the combination of SEQ ID NO:3 to a Δ8 truncated form of GAA, such as the Δ8 truncated form of hGAA represented in SEQ ID NO:27;
- the combination of SEQ ID NO:4 to a Δ8 truncated form of GAA, such as the Δ8 truncated form of hGAA represented in SEQ ID NO:27;
- the combination of SEQ ID NO:5 to a Δ8 truncated form of GAA, such as the Δ8 truncated form of hGAA represented in SEQ ID NO:27;
   the combination of SEQ ID NO:6 to a Δ8 truncated form of GAA, such as the Δ8 truncated form of hGAA represented in SEQ ID NO:27;
- the combination of SEQ ID NO:7 to a Δ8 truncated form of GAA, such as the Δ8 truncated form of hGAA represented in SEQ ID NO:27;
- the combination of SEQ ID NO:3 to a Δ42 truncated form of GAA, such as the Δ42 truncated form of hGAA represented in SEQ ID NO:28;
- the combination of SEQ ID NO:4 to a Δ42 truncated form of GAA, such as the Δ42 truncated form of hGAA represented in SEQ ID NO:28;
- the combination of SEQ ID NO:5 to a Δ42 truncated form of GAA, such as the Δ42 truncated form of hGAA represented in SEQ ID NO:28;
   the combination of SEQ ID NO:6 to a Δ42 truncated form of GAA, such as the Δ42 truncated form of hGAA represented in SEQ ID NO:28; and
- the combination of SEQ ID NO:7 to a Δ42 truncated form of GAA, such as the Δ42 truncated form of hGAA represented in SEQ ID NO:28;
or is a functional derivative thereof having at least 90% identity, in particular at least 95%, at least 96%, at least 97%, at least 98%, or at least 99% identity to the resulting sequence combination. In these embodiments, as mentioned above, the signal peptide moiety may be a sequence comprising from 1 to 5, in particular from 1 to 4, in particular from 1 to 3, more particularly from 1 to 2, in particular 1 amino acid deletion(s), insertion(s) or substitution(s) as compared to the sequences shown in SEQ ID NO:3 to 7, as long as the resulting sequence corresponds to a functional signal peptide, i.e. a signal peptide that allows secretion of the resulting chimeric truncated GAA protein.

The relative proportion of newly-synthesized GAA that is secreted from the cell can be routinely determined by methods known in the art and as described in the examples. Secreted proteins can be detected by directly measuring the protein itself (e.g., by Western blot) or by protein activity assays (e.g., enzyme assays) in cell culture medium, serum, milk, etc.

Those skilled in the art will further understand that the truncated GAA polypeptide or the chimeric GAA polypeptide may contain additional amino acids, e. g., as a result of manipulations of the nucleic acid construct such as the addition of a restriction site, as long as these additional amino acids do not render the signal peptide or the GAA polypeptide non-functional. The additional amino acids can be cleaved or can be retained by the mature polypeptide as long as retention does not result in a non-functional polypeptide.

In another aspect, the invention relates to a nucleic acid molecule encoding the truncated GAA polypeptide of the invention or the chimeric GAA polypeptide of the invention.

The sequence of the nucleic acid molecule of the invention, encoding a truncated GAA, is optimized for expression of the GAA polypeptide *in vivo.* Sequence optimization may include a number of changes in a nucleic acid sequence, including codon optimization, increase of GC content, decrease of the number of CpG islands, decrease of the number of alternative open reading frames (ARFs) and decrease of the number of splice donor and splice acceptor sites. Because of the degeneracy of the genetic code, different nucleic acid molecules may encode the same protein. It is also well known that the genetic codes of different organisms are often biased towards using one of the several codons that encode the same amino acid over the others. Through codon optimization, changes are introduced in a nucleotide sequence that take advantage of the codon bias existing in a given cellular context so that the resulting codon optimized nucleotide sequence is more likely to be expressed in such given cellular context at a relatively high level compared to the non-codon optimised sequence. In a preferred embodiment of the invention, such sequence optimized nucleotide sequence encoding a truncated GAA is codon-optimized to improve its expression in human cells compared to non-codon optimized nucleotide sequences coding for the same truncated GAA protein, for example by taking advantage of the human specific codon usage bias.

In a particular embodiment, the optimized GAA coding sequence is codon optimized, and/or has an increased GC content and/or has a decreased number of alternative open reading frames, and/or has a decreased number of splice donor and/or splice acceptor sites, as compared to nucleotides 82-2859 of the wild-type hGAA coding sequence of SEQ ID NO:8. For example, nucleic acid sequence of the invention results in an at least 2, 3, 4, 5 or 10 % increase of GC content in the GAA sequence as compared to the sequence of the wild-type GAA sequence. In a particular embodiment, the nucleic acid sequence of the invention results in a 2, 3, 4 or, more particularly, 5% or 10% (particularly 5%) increase of GC content in the GAA sequence as compared to the sequence of the wild-type GAA nucleotide sequence. In a particular embodiment, the nucleic acid sequence of the invention encoding a functional GAA polypeptide is "substantially identical", that is, about 70% identical, more preferably about 80% identical, even more preferably about 90% identical, even more preferably about 95% identical, even more preferably about 97%, 98% or even 99% identical to nucleotides 82-2859 of the sequence shown in SEQ ID NO: 8. As mentioned above, in addition to the GC content and/or number of ARFs, sequence optimization may also comprise a decrease in the number of CpG islands in the sequence and/or a decrease in the number of splice donor and acceptor sites. Of course, as is well known to those skilled in the art, sequence optimization is a balance between all these parameters, meaning that a sequence may be considered optimized if at least one of the above parameters is improved while one or more of the other parameters is not, as long as the optimized sequence leads to an improvement of the transgene, such as an improved expression and/or a decreased immune response to the transgene *in vivo.*

In addition, the adaptiveness of a nucleotide sequence encoding a functional GAA to the codon usage of human cells may be expressed as codon adaptation index (CAI). A codon adaptation index is herein defined as a measurement of the relative adaptiveness of the codon usage of a gene towards the codon usage of highly expressed human genes. The relative adaptiveness (w) of each codon is the ratio of the usage of each codon, to that of the most abundant codon for the same amino acid. The CAI is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li, 1987, Nucleic Acids Research 15: 1281-1295; also see: Kim et al, Gene. 1997, 199:293-301; zur Megede et al, Journal of Virology, 2000, 74: 2628-2635). Preferably, a nucleic acid molecule encoding a GAA has a CAI of at least 0.75 (in particular 0.77), 0.8, 0.85, 0.90, 0.92 or 0.94.

The term "nucleic acid sequence" (or nucleic acid molecule) refers to a DNA or RNA molecule in single or double stranded form, particularly a DNA encoding a GAA protein according to the invention.

The inventors have found that the above described truncated GAA polypeptide, when expressed from a nucleic acid molecule encoding the same, causes surprisingly high levels of expression of functional GAA protein both *in vitro* and *in vivo* compared to the wild-type GAA cDNA. Furthermore, as also shown by the inventors, the truncated GAA protein produced from liver and muscle cells expressing the nucleic acid molecule of the invention induces no immune response. This means that this nucleic acid molecule may be used to produce high levels of GAA protein, and provides therapeutic benefits such as avoiding to resort to immunosuppressive treatments, allowing low dose immunosuppressive treatment, and allowing repeated administration of the nucleic acid molecule of the invention to a subject in need thereof. Therefore, the truncated GAA polypeptide of the invention and the nucleic acid molecule of the invention are of special interest in contexts where GAA expression and/or activity is deficient or where high levels of expression of GAA can ameliorate a disease, such as for a glycogen storage disease. In a particular, the glycogen storage disease may be GSDI (von Gierke's disease), GSDII (Pompe disease), GSDIII (Cori disease), GSDIV, GSDV, GSDVI, GSDVII, GSDVIII or lethal congenital glycogen storage disease of the heart. More particularly, the glycogen storage disease is selected in the group consisting of GSDI, GSDII and GSDIII, even more particularly in the group consisting of GSDII and GSDIII. In an even more particular embodiment, the glycogen storage disease is GSDII.. In particular, the nucleic acid molecules of the invention may be useful in gene therapy to treat GAA-deficient conditions or other conditions associated by accumulation of glycogen such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII. In an even more particular embodiment, the nucleic acid molecules of the invention may be useful in gene therapy to treat GSDII.

In another embodiment of the invention, the part of the nucleic acid molecule of the invention encoding the truncated GAA polypeptide moiety has at least 75 percent (such as 77,7%), or at least 80 percent or at least 82 percent (such as 83.1%) identity to the corresponding part of the nucleotide sequence encoding SEQ ID NO:1 or SEQ ID NO:33, in particular SEQ ID NO:1, which are sequences of wild-type hGAA polypeptides devoid of a signal peptide.

The truncated GAA moiety of the nucleic acid molecule of the invention preferably has at least 85 percent, more preferably at least 90 percent, and even more preferably at least 92 percent identity, in particular at least 95 percent identity, for example at least 98, 99 or 100 percent identity to the nucleotide sequence of SEQ ID NO: 10 or 11, which are sequence-optimized sequences.

The term "identical" and declinations thereof refers to the sequence identity between two nucleic acid molecules. When a position in both of the two compared sequences is occupied by the same base e.g., if a position in each of two DNA molecules is occupied by adenine, then the molecules are identical at that position. The percent of identity between two sequences is a function of the number of matching positions shared by the two sequences divided by the number of positions compared X 100. For example, if 6 of 10 of the positions in two sequences are matched then the two sequences are 60% identical. Generally, a comparison is made when two sequences are aligned to give maximum identity. Various bioinformatic tools known to the one skilled in the art might be used to align nucleic acid sequences such as BLAST or FASTA.

Furthermore, the nucleic acid molecule of the invention encodes a functional GAA protein, i.e. it encodes for a human GAA protein that, when expressed, has the functionality of wild-type GAA protein. As defined above, the functionality of wild-type GAA is to hydrolyse both α-1,4 and α-1,6 linkages of oligosaccharides and polysaccharides, more particularly of glycogen, to liberate glucose. The functional GAA protein encoded by the nucleic acid of the invention may have a hydrolysing activity on glycogen of at least 50 %, 60 %, 70 %, 80 %, 90 %, 95 %, 99 %, or at least 100 % as compared to the wild-type GAA protein of SEQ ID NO: 1, 2, 30 or 33. The activity of the GAA protein encoded by the nucleic acid of the invention may even be of more than 100 %, such as of more than 110 %, 120 %, 130 %, 140 %, or even more than 150 % of the activity of the wild-type GAA protein of SEQ ID NO:1, 2, 30 or 33.

A skilled person is readily able to determine whether a nucleic acid according to the invention expresses a functional GAA protein. Suitable methods would be apparent to those skilled in the art. For example, one suitable in vitro method involves inserting the nucleic acid into a vector, such as a plasmid or viral vector, transfecting or transducing host cells, such as 293T or HeLa cells, or other cells such as Huh7, with the vector, and assaying for GAA activity. Alternatively, a suitable *in vivo* method involves transducing a vector containing the nucleic acid into a mouse model of Pompe disease or another glycogen storage disorder and assaying for functional GAA in the plasma of the mouse and presence of GAA in tissues. Suitable methods are described in more details in the experimental part below.

In a particular embodiment, the nucleic acid molecule of the invention comprises the sequence shown in SEQ ID NO:12 or SEQ ID NO:13, encoding the polypeptide having the amino acid sequence shown in SEQ ID NO:27.

The invention also relates to a nucleic acid construct comprising a nucleic acid molecule of the invention. The nucleic acid construct may correspond to an expression cassette comprising the nucleic acid sequence of the invention, operably linked to one or more expression control sequences and/or other sequences improving the expression of a transgene and/or sequences enhancing the secretion of the encoded protein and/or sequences enhancing the uptake of the encode protein. As used herein, the term "operably linked" refers to a linkage of polynucleotide elements in a functional relationship. A nucleic acid is "operably linked" when it is placed into a functional relationship with another nucleic acid sequence. For instance, a promoter, or another transcription regulatory sequence, is operably linked to a coding sequence if it affects the transcription of the coding sequence. Such expression control sequences are known in the art, such as promoters, enhancers (such as cis-regulatory modules (CRMs)), introns, polyA signals, etc.
In particular, the expression cassette may include a promoter. The promoter may be an ubiquitous or tissue-specific promoter, in particular a promoter able to promote expression in cells or tissues in which expression of GAA is desirable such as in cells or tissues in which GAA expression is desirable in GAA-deficient patients. In a particular embodiment, the promoter is a liver-specific promoter such as the alpha-1 antitrypsin promoter (hAAT) (SEQ ID NO:14), the transthyretin promoter, the albumin promoter, the thyroxine-binding globulin (TBG) promoter, the LSP promoter (comprising a thyroid hormone-binding globulin promoter sequence, two copies of an alpha1-microglobulin/bikunin enhancer sequence, and a leader sequence - 34.Ill, C. R., et al. (1997). Optimization of the human factor VIII complementary DNA expression plasmid for gene therapy of hemophilia A. Blood Coag. Fibrinol. 8: S23-S30.), etc. Other useful liver-specific promoters are known in the art, for example those listed in the Liver Specific Gene Promoter Database compiled the Cold Spring Harbor Laboratory (http://rulai.cshl.edu/LSPD/). A preferred promoter in the context of the invention is the hAAT promoter. In another embodiment, the promoter is a promoter directing expression in one tissue or cell of interest (such as in muscle cells), and in liver cells. For example, to some extent, promoters specific of muscle cells such as the desmin, Spc5-12 and MCK promoters may present some leakage of expression into liver cells, which can be advantageous to induce immune tolerance of the subject to the GAA polypeptide expressed from the nucleic acid of the invention.
Other tissue-specific or non-tissue-specific promoters may be useful in the practice of the invention. For example, the expression cassette may include a tissue-specific promoter which is a promoter different from a liver specific promoter. For example the promoter may be muscle-specific, such as the desmin promoter (and a desmin promoter variant such as a desmin promoter including natural or artificial enhancers), the SPc5-12 or the MCK promoter. In another embodiment, the promoter is a promoter specific of other cell lineage, such as the erythropoietin promoter, for the expression of the GAA polypeptide from cells of the erythroid lineage.
In another embodiment, the promoter is a ubiquitous promoter. Representative ubiquitous promoters include the cytomegalovirus enhancer/chicken beta actin (CAG) promoter, the cytomegalovirus enhancer/promoter (CMV), the PGK promoter, the SV40 early promoter, etc.
In addition, the promoter may also be an endogenous promoter such as the albumin promoter or the GAA promoter.

In a particular embodiment, the promoter is associated to an enhancer sequence, such as cis-regulatory modules (CRMs) or an artificial enhancer sequence. For example, the promoter may be associated to an enhancer sequence such as the human ApoE control region (or Human apolipoprotein E/C-I gene locus, hepatic control region HCR-1 - Genbank accession No. U32510, shown in SEQ ID NO:15). In a particular embodiment, an enhancer sequence such as the ApoE sequence is associated to a liver-specific promoter such as those listed above, and in particular such as the hAAT promoter. Other CRMs useful in the practice of the present invention include those described in Rincon et al., Mol Ther. 2015 Jan;23(1):43-52, Chuah et al., Mol Ther. 2014 Sep;22(9):1605-13 or Nair et al., Blood. 2014 May 15;123(20):3195-9.

In another particular embodiment, the nucleic acid construct comprises an intron, in particular an intron placed between the promoter and the GAA coding sequence. An intron may be introduced to increase mRNA stability and the production of the protein. In a further embodiment, the nucleic acid construct comprises a human beta globin b2 (or HBB2) intron, a coagulation factor IX (FIX) intron, a SV40 intron or a chicken beta-globin intron. In another further embodiment, the nucleic acid construct of the invention contains a modified intron (in particular a modified HBB2 or FIX intron) designed to decrease the number of, or even totally remove, alternative open reading frames (ARFs) found in said intron. Preferably, ARFs are removed whose length spans over 50 bp and have a stop codon in frame with a start codon. ARFs may be removed by modifying the sequence of the intron. For example, modification may be carried out by way of nucleotide substitution, insertion or deletion, preferably by nucleotide substitution. As an illustration, one or more nucleotides, in particular one nucleotide, in an ATG or GTG start codon present in the sequence of the intron of interest may be replaced resulting in a non-start codon. For example, an ATG or a GTG may be replaced by a CTG, which is not a start codon, within the sequence of the intron of interest.

The classical HBB2 intron used in nucleic acid constructs is shown in SEQ ID NO:16. For example, this HBB2 intron may be modified by eliminating start codons (ATG and GTG codons) within said intron. In a particular embodiment, the modified HBB2 intron comprised in the construct has the sequence shown in SEQ ID NO:17. The classical FIX intron used in nucleic acid constructs is derived from the first intron of human FIX and is shown in SEQ ID NO:18. FIX intron may be modified by eliminating start codons (ATG and GTG codons) within said intron. In a particular embodiment, the modified FIX intron comprised in the construct of the invention has the sequence shown in SEQ ID NO:19. The classical chicken-beta globin intron used in nucleic acid constructs is shown in SEQ ID NO:210. Chicken-beta globin intron may be modified by eliminating start codons (ATG and GTG codons) within said intron. In a particular embodiment, the modified chicken-beta globin intron comprised in the construct of the invention has the sequence shown in SEQ ID NO:21.

The inventors have previously shown in WO2015/162302 that such a modified intron, in particular a modified HBB2 or FIX intron, has advantageous properties and can significantly improve the expression of a transgene.

In a particular embodiment, the nucleic acid construct of the invention is an expression cassette comprising, in the 5' to 3' orientation, a promoter optionally preceded by an enhancer, the coding sequence of the invention (i.e. the optimized truncated GAA coding sequence of the invention, the chimeric GAA coding sequence of the invention, or the chimeric and sequence optimized GAA coding sequence of the invention), and a polyadenylation signal (such as the bovine growth hormone polyadenylation signal, the SV40 polyadenylation signal, or another naturally occurring or artificial polyadenylation signal). In a particular embodiment, the nucleic acid construct of the invention is an expression cassette comprising, in the 5' to 3' orientation, a promoter optionally preceded by an enhancer, (such as the ApoE control region), an intron (in particular an intron as defined above), the coding sequence of the invention, and a polyadenylation signal. In a further particular embodiment, the nucleic acid construct of the invention is an expression cassette comprising, in the 5' to 3' orientation, an enhancer such as the ApoE control region, a promoter, an intron (in particular an intron as defined above), the coding sequence of the invention, and a polyadenylation signal. In a further particular embodiment of the invention the expression cassette comprising, in the 5' to 3' orientation, an ApoE control region, the hAAT-liver specific promoter, a HBB2 intron (in particular a modified HBB2 intron as defined above), the coding sequence of the invention, and the bovine growth hormone polyadenylation signal, such as the construct shown in:
- SEQ ID NO: 22, including a non-optimized nucleotide sequence encoding a Δ8 truncated form of GAA derived from the parent hGAA of SEQ ID NO:1 and encoding a signal peptide of SEQ ID NO:5;
- SEQ ID NO:23, including an optimized sequence encoding a Δ8 truncated form of GAA derived from the parent hGAA of SEQ ID NO:1 (nucleotide sequence derived from the optimized sequence of SEQ ID NO:12) and encoding a signal peptide of SEQ ID NO:5;
- SEQ ID NO: 24, including another optimized sequence encoding a Δ8 truncated form of GAA derived from the parent hGAA of SEQ ID NO:1 (nucleotide sequence derived from the optimized sequence of SEQ ID NO:13) and encoding a signal peptide of SEQ ID NO:5;
- SEQ ID NO:25, including an optimized sequence encoding a Δ8 truncated form of GAA derived from the parent hGAA of SEQ ID NO:1 (nucleotide sequence derived from the optimized sequence of SEQ ID NO:12) and a signal peptide of SEQ ID NO:3; and
- SEQ ID NO:26, including an optimized sequence encoding a Δ42 truncated form of GAA derived from the parent hGAA of SEQ ID NO:1 (nucleotide sequence derived from the optimized sequence of SEQ ID NO:12) and a signal peptide of SEQ ID NO:3.
Other expression cassettes of the invention may include the following nucleic acid sequences:
- a non-optimized nucleotide sequence encoding a Δ8 truncated form of GAA derived from the parent hGAA of SEQ ID NO:1 and encoding a signal peptide of SEQ ID NO:4, 6 or 7;
- a non-optimized nucleotide sequence encoding a Δ42 truncated form of GAA derived from the parent hGAA of SEQ ID NO:1 and encoding a signal peptide of SEQ ID NO:4, 6 or 7;
- an optimized nucleotide sequence encoding a Δ8 truncated form of GAA derived from the parent hGAA of SEQ ID NO:1 (nucleotide sequence derived from the optimized sequence of SEQ ID NO:12) and encoding a signal peptide of SEQ ID NO:4, 6 or 7;
- an optimized nucleotide sequence encoding a Δ8 truncated form of GAA derived from the parent hGAA of SEQ ID NO:1 (nucleotide sequence derived from the optimized sequence of SEQ ID NO:13) and encoding a signal peptide of SEQ ID NO:4, 6 or 7;
- an optimized nucleotide sequence encoding a Δ42 truncated form of GAA derived from the parent hGAA of SEQ ID NO:1 (nucleotide sequence derived from the optimized sequence of SEQ ID NO:12) and encoding a signal peptide of SEQ ID NO:4, 6 or 7;
- an optimized nucleotide sequence encoding a Δ42 truncated form of GAA derived from the parent hGAA of SEQ ID NO:1 (nucleotide sequence derived from the optimized sequence of SEQ ID NO:13) and encoding a signal peptide of SEQ ID NO:4, 6 or 7.
In alternative embodiments of these specific constructs, the sequence coding SEQ ID NO:1 is replaced by a sequence coding SEQ ID NO:33.

In a particular embodiment, the expression cassette comprises the ApoE control region, the hAAT-liver specific promoter, a codon-optimized HBB2 intron, the coding sequence of the invention and the bovine growth hormone polyadenylation signal.

In designing the nucleic acid construct of the invention, one skilled in the art will take care of respecting the size limit of the vector used for delivering said construct to a cell or organ. In particular, one skilled in the art knows that a major limitation of AAV vector is its cargo capacity which may vary from one AAV serotype to another but is thought to be limited to around the size of parental viral genome. For example, 5 kb, is the maximum size usually thought to be packaged into an AAV8 capsid (Wu Z. et al., Mol Ther., 2010, 18(1): 80-86; Lai Y. et al., Mol Ther., 2010, 18(1): 75-79; Wang Y. et al., Hum Gene Ther Methods, 2012, 23(4): 225-33). Accordingly, those skilled in the art will take care in practicing the present invention to select the components of the nucleic acid construct of the invention so that the resulting nucleic acid sequence, including sequences coding AAV 5'- and 3'-ITRs to preferably not exceed 110 % of the cargo capacity of the AAV vector implemented, in particular to preferably not exceed 5.5 kb.

The invention also relates to a vector comprising a nucleic acid molecule or construct as disclosed herein. In particular, the vector of the invention is a vector suitable for protein expression, preferably for use in gene therapy. In one embodiment, the vector is a plasmid vector. In another embodiment, the vector is a nanoparticle containing a nucleic acid molecule of the invention, in particular a messenger RNA encoding the GAA polypeptide of the invention. In another embodiment, the vector is a system based on transposons, allowing integration of the nucleic acid molecule or construct of the invention in the genome of the target cell, such as the hyperactive Sleeping Beauty (SB100X) transposon system (Mates et al. 2009). In another embodiment, the vector is a viral vector suitable for gene therapy, targeting any cell of interest such as liver tissue or cells, muscle cell, CNS cells (such as brain cells), or hematopoietic stem cells such as cells of the erythroid lineage (such as erythrocytes). In this case, the nucleic acid construct of the invention also contains sequences suitable for producing an efficient viral vector, as is well known in the art. In a particular embodiment, the viral vector is derived from an integrating virus. In particular, the viral vector may be derived from a retrovirus or a lentivirus. In a further particular embodiment, the viral vector is an AAV vector, such as an AAV vector suitable for transducing liver tissues or cells, more particularly an AAV-1, -2 and AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p.16026), -7, -8, -9, -10 such as - cy10 and -rh10, -rh74, -dj, Anc80, LK03, AAV2i8, porcine AAV serotypes such as AAVpo4 and AAVpo6, etc., vector or a retroviral vector such as a lentiviral vector and an alpha-retrovirus. As is known in the art, depending on the specific viral vector considered for use, additional suitable sequences will be introduced in the nucleic acid construct of the invention for obtaining a functional viral vector. Suitable sequences include AAV ITRs for an AAV vector, or LTRs for lentiviral vectors. As such, the invention also relates to an expression cassette as described above, flanked by an ITR or an LTR on each side.

Advantages of viral vectors are discussed in the following part of this disclosure. Viral vectors are preferred for delivering the nucleic acid molecule or construct of the invention, such as a retroviral vector, for example a lentiviral vector, or a non-pathogenic parvovirus, more preferably an AAV vector. The human parvovirus Adeno-Associated Virus (AAV) is a dependovirus that is naturally defective for replication which is able to integrate into the genome of the infected cell to establish a latent infection. The last property appears to be unique among mammalian viruses because the integration occurs at a specific site in the human genome, called AAVS1, located on chromosome 19 (19q13.3-qter).
Therefore, AAV vectors have arisen considerable interest as a potential vectors for human gene therapy. Among the favorable properties of the virus are its lack of association with any human disease, its ability to infect both dividing and non-dividing cells, and the wide range of cell lines derived from different tissues that can be infected.
Among the serotypes of AAVs isolated from human or non-human primates (NHP) and well characterized, human serotype 2 is the first AAV that was developed as a gene transfer vector. Other currently used AAV serotypes include AAV-1, AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p.16026), -7, -8, -9, -10 such as cy10 and -rh10, -rh74, -dj, Anc80, LK03, AAV2i8, porcine AAV serotypes such as AAVpo4 and AAVpo6, and tyrosine, lysine and serine capsid mutants of the AAV serotypes, etc.. In addition, other non-natural engineered variants and chimeric AAV can also be useful.
AAV viruses may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus.
Desirable AAV fragments for assembly into vectors include the cap proteins, including the vp1, vp2, vp3 and hypervariable regions, the rep proteins, including rep 78, rep 68, rep 52, and rep 40, and the sequences encoding these proteins. These fragments may be readily utilized in a variety of vector systems and host cells.
AAV-based recombinant vectors lacking the Rep protein integrate with low efficacy into the host's genome and are mainly present as stable circular episomes that can persist for years in the target cells. Alternatively to using AAV natural serotypes, artificial AAV serotypes may be used in the context of the present invention, including, without limitation, AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid, a recombinant AAV capsid, or a "humanized" AAV capsid.
Accordingly, the present invention relates to an AAV vector comprising the nucleic acid molecule or construct of the invention. In the context of the present invention, the AAV vector comprises an AAV capsid able to transduce the target cells of interest, in particular hepatocytes. According to a particular embodiment, the AAV vector is of the AAV-1, -2, AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p.16026), -7, -8, -9, -10 such as -cy10 and -rh10, -rh74, -dj, Anc80, LK03, AAV2i8, porcine AAV such as AAVpo4 and AAVpo6, and tyrosine, lysine and serine capsid mutants of a AAV serotypes, etc., serotype. In a particular embodiment, the AAV vector is of the AAV8, AAV9, AAVrh74 or AAV2i8 serotype (i.e. the AAV vector has a capsid of the AAV8, AAV9, AAVrh74 or AAV2i8 serotype). In a further particular embodiment, the AAV vector is a pseudotyped vector, i.e. its genome and capsid are derived from AAVs of different serotypes. For example, the pseudotyped AAV vector may be a vector whose genome is derived from one of the above mentioned AAV serotypes, and whose capsid is derived from another serotype. For example, the genome of the pseudotyped vector may have a capsid derived from the AAV8, AAV9, AAVrh74 or AAV2i8 serotype, and its genome may be derived from and different serotype. In a particular embodiment, the AAV vector has a capsid of the AAV8, AAV9 or AAVrh74 serotype, in particular of the AAV8 or AAV9 serotype, more particularly of the AAV8 serotype.
In a specific embodiment, wherein the vector is for use in delivering the transgene to muscle cells, the AAV vector may be selected, among others, in the group consisting of AAV8, AAV9 and AAVrh74. In another specific embodiment, wherein the vector is for use in delivering the transgene to liver cells, the AAV vector may be selected, among others, in the group consisting of AAV5, AAV8, AAV9, AAV-LK03, AAV-Anc80 and AAV3B.
In another embodiment, the capsid is a modified capsid. In the context of the present invention, a "modified capsid" may be a chimeric capsid or capsid comprising one or more variant VP capsid proteins derived from one or more wild-type AAV VP capsid proteins.
In a particular embodiment, the AAV vector is a chimeric vector, i.e. its capsid comprises VP capsid proteins derived from at least two different AAV serotypes, or comprises at least one chimeric VP protein combining VP protein regions or domains derived from at least two AAV serotypes. Examples of such chimeric AAV vectors useful to transduce liver cells are described in Shen et al., Molecular Therapy, 2007 and in Tenney et al., Virology, 2014. For example a chimeric AAV vector can derive from the combination of an AAV8 capsid sequence with a sequence of an AAV serotype different from the AAV8 serotype, such as any of those specifically mentioned above. In another embodiment, the capsid of the AAV vector comprises one or more variant VP capsid proteins such as those described in WO2015013313, in particular the RHM4-1, RHM15-1, RHM15-2, RHM15-3/RHM15-5, RHM15-4 and RHM15-6 capsid variants, which present a high liver tropism.
In another embodiment, the modified capsid can be derived also from capsid modifications inserted by error prone PCR and/or peptide insertion (e.g. as described in Bartel et al., 2011). In addition, capsid variants may include single amino acid changes such as tyrosine mutants (e.g. as described in Zhong et al., 2008)
In addition, the genome of the AAV vector may either be a single stranded or self-complementary double-stranded genome (McCarty et al., Gene Therapy, 2003). Self-complementary double-stranded AAV vectors are generated by deleting the terminal resolution site (trs) from one of the AAV terminal repeats. These modified vectors, whose replicating genome is half the length of the wild type AAV genome have the tendency to package DNA dimers. In a preferred embodiment, the AAV vector implemented in the practice of the present invention has a single stranded genome, and further preferably comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid.

In a particularly preferred embodiment, the invention relates to an AAV vector comprising, in a single-stranded or double-stranded, self-complementary genome (e.g. a single-stranded genome), the nucleic acid acid construct of the invention. In one embodiment, the AAV vector comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid. In a further particular embodiment, said nucleic acid is operably linked to a promoter, especially a ubiquitous or liver-specific promoter. According to a specific variant embodiment, the promoter is a ubiquitous promoter such as the cytomegalovirus enhancer/chicken beta actin (CAG) promoter, the cytomegalovirus enhancer/promoter (CMV), the PGK promoter and the SV40 early promoter. In a specific variant, the ubiquitous promoter is the CAG promoter. According to another variant, the promoter is a liver-specific promoter such as the alpha-1 antitrypsin promoter (hAAT), the transthyretin promoter, the albumin promoter and the thyroxine-binding globulin (TBG) promoter. In a specific variant, the liver-specific promoter is the hAAT liver-specific promoter of SEQ ID NO:14. In a further particular embodiment, the nucleic acid construct comprised into the genome of the AAV vector of the invention further comprises an intron as described above, such as an intron placed between the promoter and the nucleic acid sequence encoding the GAA coding sequence (i.e. the optimized GAA coding sequence of the invention, the chimeric GAA coding sequence of the invention, or the chimeric and optimized GAA coding sequence of the invention). Representative introns that may be included within the nucleic acid construct introduced within the AAV vector genome include, without limitation, the human beta globin b2 (or HBB2) intron, the FIX intron and the chicken beta-globin intron. Said intron within the genome of the AAV vector may be a classical (or unmodified) intron or a modified intron designed to decrease the number of, or even totally remove, alternative open reading frames (ARFs) within said intron. Modified and unmodified introns that may be used in the practice of this embodiment where the nucleic acid of the invention is introduced within an AAV vector are thoroughly described above. In a particular embodiment, the AAV vector, in particular an AAV vector comprising an AAV8, AAV9, AAVrh74 or AAV2i8 capsid, in particular an AAV8, AAV9 or AAVrh74 capsid, such as an AAV8 or AAV9 capsid, more particularly an AAV8 capsid, of the invention includes within its genome a modified (or optimized) intron such as the modified HBB2 intron of SEQ ID NO:17, the modified FIX intron of SEQ ID NO:19 and the modified chicken beta-globin intron of SEQ ID NO:21. In a further particular embodiment, the vector of the invention is an AAV vector comprising comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid, comprising a genome containing, in the 5' to 3' orientation: an AAV 5'-ITR (such as an AAV2 5'-ITR); an ApoE control region; the hAAT-liver specific promoter; a HBB2 intron (in particular a modified HBB2 intron as defined above); the GAA coding sequence of the invention; the bovine growth hormone polyadenylation signal; and an AAV 3'-ITR (such as an AAV2 3'-ITR), such as a genome comprising a the nucleic acid construct shown in SEQ ID NO:22 to 26 flanked by an AAV 5'-ITR (such as an AAV2 5'-ITR) and an AAV 3'-ITR (such as an AAV2 3'-ITR). Other nucleic acid constructs useful in the practice of the present invention comprise those described above, including:
- a non-optimized nucleotide sequence encoding a Δ8 truncated form of GAA derived from the parent hGAA of SEQ ID NO:1 and encoding a signal peptide of SEQ ID NO:4, 6 or 7;
- a non-optimized nucleotide sequence encoding a Δ42 truncated form of GAA derived from the parent hGAA of SEQ ID NO:1 and encoding a signal peptide of SEQ ID NO:4, 6 or 7;
- an optimized nucleotide sequence encoding a Δ8 truncated form of GAA derived from the parent hGAA of SEQ ID NO:1 (nucleotide sequence derived from the optimized sequence of SEQ ID NO:12) and encoding a signal peptide of SEQ ID NO:4 or 6;
- an optimized nucleotide sequence encoding a Δ8 truncated form of GAA derived from the parent hGAA of SEQ ID NO:1 (nucleotide sequence derived from the optimized sequence of SEQ ID NO:13) and encoding a signal peptide of SEQ ID NO:4, 6 or 7;
- an optimized nucleotide sequence encoding a Δ42 truncated form of GAA derived from the parent hGAA of SEQ ID NO:1 (nucleotide sequence derived from the optimized sequence of SEQ ID NO:12) and encoding a signal peptide of SEQ ID NO:4 or 6;
- an optimized nucleotide sequence encoding a Δ42 truncated form of GAA derived from the parent hGAA of SEQ ID NO:1 (nucleotide sequence derived from the optimized sequence of SEQ ID NO:13) and encoding a signal peptide of SEQ ID NO:4, 6 or 7.In alternative embodiments of these specific constructs, the sequence coding SEQ ID NO:1 is replaced by a sequence coding SEQ ID NO:33.
In a particular embodiment of the invention, the nucleic acid construct of the invention comprises a liver-specific promoter as described above, and the vector is a viral vector capable of transducing liver tissue or cells as described above. The inventors present below data showing that the protolerogenic and metabolic properties of the liver are advantageously implemented thanks to this embodiment to develop highly efficient and optimized vectors to express secretable forms of GAA in hepatocytes and to induce immune tolerance to the protein.

In addition, in a further particular embodiment, the invention provides the combination of two vectors, such as two viral vectors, in particular two AAV vectors, for improving gene delivery and treatment efficacy in the cells of interest. For example, the two vectors may carry the nucleic acid molecule of the invention coding for the GAA protein of the invention, under the control of one different promoter in each of these two vectors. In a particular embodiment, one vector comprises a promoter which is a liver-specific promoter (as one of those described above), and the other vector comprises a promoter which is specific of another tissue of interest for the treatment of a glycogen storage disorder, such as a muscle-specific promoter, for example the desmin promoter. In a particular variant of this embodiment, this combination of vectors corresponds to multiple co-packaged AAV vectors produced as described in WO2015196179.

The invention also relates to a cell, for example a liver cell, that is transformed with a nucleic acid molecule or construct of the invention as is the case for *ex vivo* gene therapy. Cells of the invention may be delivered to the subject in need thereof, such as GAA-deficient patient, by any appropriate administration route such as via injection in the liver or in the bloodstream of said subject. In a particular embodiment, the invention involves introducing the nucleic acid molecule, the nucleic acid construct or the vector, particularly a lentiviral vector, of the invention into liver cells, in particular into liver cells of the subject to be treated, and administering said transformed liver cells into which the nucleic acid has been introduced to the subject. Advantageously, this embodiment is useful for secreting GAA from said cells. In a particular embodiment, the liver cells are liver cells from the patient to be treated, or are liver stem cells that are further transformed, and differentiated *in vitro* into liver cells, for subsequent administration to the patient.

The present invention further relates to a transgenic, nonhuman animal comprising in its genome the nucleic acid molecule or construct encoding a GAA polypeptide according to the invention. In a particular embodiment, the animal is a mouse.

Apart from the specific delivery systems embodied below in the examples, various delivery systems are known and can be used to administer the nucleic acid molecule or construct of the invention, e.g., encapsulation in liposomes, microparticles, microcapsules, recombinant cells capable of expressing the coding sequence of the invention, receptor-mediated endocytosis, construction of a therapeutic nucleic acid as part of a retroviral or other vector, etc.

According to an embodiment, it may be desirable to introduce the GAA polypeptide, nucleic acid molecule, nucleic acid construct or cell of the invention into the liver of the subject by any suitable route. In addition naked DNA such as minicircles and transposons can be used for delivery or lentiviral vectors. Additionally, gene editing technologies such as zinc finger nucleases, meganucleases, TALENs, and CRISPR can also be used to deliver the coding sequence of the invention.

The present invention also provides pharmaceutical compositions comprising the nucleic acid molecule, the nucleic acid construct, the vector, the GAA polypeptide, or the cell of the invention. Such compositions comprise a therapeutically effective amount of the therapeutic (the nucleic acid molecule, the nucleic acid construct, the vector, the GAA polypeptide or the cell of the invention), and a pharmaceutically acceptable carrier. In a specific embodiment, the term "pharmaceutically acceptable" means approved by a regulatory agency of the Federal or a state government or listed in the U.S. or European Pharmacopeia or other generally recognized pharmacopeia for use in animals, and humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsions, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the subject. In a particular embodiment, the nucleic acid, vector or cell of the invention is formulated in a composition comprising phosphate-buffered saline and supplemented with 0.25% human serum albumin. In another particular embodiment, the nucleic acid, vector or cell of the invention is formulated in a composition comprising ringer lactate and a non-ionic surfactant, such as pluronic F68 at a final concentration of 0.01-0.0001%, such as at a concentration of 0.001%, by weight of the total composition. The formulation may further comprise serum albumin, in particular human serum albumin, such as human serum albumin at 0.25%. Other appropriate formulations for either storage or administration are known in the art, in particular from WO 2005/118792 or Allay et al., 2011.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection.

In an embodiment, the nucleic acid molecule, the nucleic acid construct, the vector, the GAA polypeptide or the cell of the invention can be delivered in a vesicle, in particular a liposome. In yet another embodiment, the nucleic acid molecule, the nucleic acid construct, the vector, the GAA polypeptide or the cell of the invention can be delivered in a controlled release system.

Methods of administration of the nucleic acid molecule, the nucleic acid construct, the vector, the GAA polypeptide or the cell of the invention include but are not limited to intradermal, intramuscular, intraperitoneal, intravenous, subcutaneous, intranasal, epidural, and oral routes. In a particular embodiment, the administration is via the intravenous or intramuscular route. The nucleic acid molecule, the nucleic acid construct, the vector, the GAA polypeptide or the cell of the invention, whether vectorized or not, may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

In a specific embodiment, it may be desirable to administer the pharmaceutical compositions of the invention locally to the area in need of treatment, e.g. the liver. This may be achieved, for example, by means of an implant, said implant being of a porous, nonporous, or gelatinous material, including membranes, such as sialastic membranes, or fibers.

The amount of the therapeutic (i.e. the nucleic acid molecule, the nucleic acid construct, the vector, the GAA polypeptide or the cell of the invention) of the invention which will be effective in the treatment of a glycogen storage disease can be determined by standard clinical techniques. In addition, in vivo and/or in vitro assays may optionally be employed to help predict optimal dosage ranges. The precise dose to be employed in the formulation will also depend on the route of administration, and the seriousness of the disease, and should be decided according to the judgment of the practitioner and each patient's circumstances. The dosage of the nucleic acid molecule, the nucleic acid construct, the vector, the GAA polypeptide or the cell of the invention administered to the subject in need thereof will vary based on several factors including, without limitation, the route of administration, the specific disease treated, the subject's age or the level of expression necessary to achieve the therapeutic effect. One skilled in the art can readily determine, based on its knowledge in this field, the dosage range required based on these factors and others. In case of a treatment comprising administering a viral vector, such as an AAV vector, to the subject, typical doses of the vector are of at least 1x10⁸ vector genomes per kilogram body weight (vg/kg), such as at least 1x10⁹ vg/kg, at least 1x10¹⁰ vg/kg, at least 1x10¹¹ vg/kg, at least 1x10¹² vg/kg at least 1x10¹³ vg/kg, or at least 1x10¹⁴ vg/kg.

The invention also relates to a method for treating a glycogen storage disease, which comprises a step of delivering a therapeutic effective amount of the nucleic acid, the vector, the GAA polypeptide, the pharmaceutical composition or the cell of the invention to a subject in need thereof.

The invention also relates to a method for treating a glycogen storage disease, said method inducing no immune response to the transgene (i.e. to the GAA polypeptide of the invention), or inducing a reduced immune response to the transgene, comprising a step of delivering a therapeutic effective amount of the nucleic acid molecule, nucleic acid construct, vector, pharmaceutical composition or cell of the invention to a subject in need thereof. The invention also relates to a method for treating a glycogen storage disease, said method comprising repeated administration of a therapeutic effective amount of the nucleic acid molecule, nucleic acid construct, vector, pharmaceutical composition or cell of the invention to a subject in need thereof. In this aspect, the nucleic acid molecule or the nucleic acid construct of the invention comprises a promoter which is functional in liver cells, thereby allowing immune tolerance to the expressed GAA polypeptide produced therefrom. As well, in this aspect, the pharmaceutical composition used in this aspect comprises a nucleic acid molecule or nucleic acid construct comprising a promoter which is functional in liver cells. In case of delivery of liver cells, said cells may be cells previously collected from the subject in need of the treatment and that were engineered by introducing therein the nucleic acid molecule or the nucleic acid construct of the invention to thereby make them able to produce the GAA polypeptide of the invention. According to an embodiment, in the aspect comprising a repeated administration, said administration may be repeated at least once or more, and may even be considered to be done according to a periodic schedule, such as once per week, per month or per year. The periodic schedule may also comprise an administration once every 2, 3, 4, 5, 6, 7, 8, 9 or 10 year, or more than 10 years. In another particular embodiment, administration of each administration of a viral vector of the invention is done using a different virus for each successive administration, thereby avoiding a reduction of efficacy because of a possible immune response against a previously administered viral vector. For example, a first administration of a viral vector comprising an AAV8 capsid may be done, followed by the administration of a vector comprising an AAV9 capsid, or even by the administration of a virus unrelated to AAVs, such as a retroviral or lentiviral vector.

According to the present invention, a treatment may include curative, alleviation or prophylactic effects. Accordingly, therapeutic and prophylactic treatment includes amelioration of the symptoms of a particular glycogen storage disease or preventing or otherwise reducing the risk of developing a particular glycogen storage disease. The term "prophylactic" may be considered as reducing the severity or the onset of a particular condition. "Prophylactic" also includes preventing reoccurrence of a particular condition in a patient previously diagnosed with the condition. "Therapeutic" may also reduce the severity of an existing condition. The term 'treatment' is used herein to refer to any regimen that can benefit an animal, in particular a mammal, more particularly a human subject.

The invention also relates to an *ex vivo* gene therapy method for the treatment of a glycogen storage disease comprising introducing the nucleic acid molecule or the nucleic acid construct of the invention into an isolated cell of a patient in need thereof, for example an isolated hematopoietic stem cell, and introducing said cell into said patient in need thereof. In a particular embodiment of this aspect, the nucleic acid molecule or construct is introduced into the cell with a vector as defined above. In a particular embodiment, the vector is an integrative viral vector. In a further particular embodiment, the viral vector is a retroviral vector, such as a lenviral vector. For example, a lentiviral vector as disclosed in van Til et al., 2010, Blood, 115(26), p. 5329, may be used in the practice in the method of the present invention.

The invention also relates to the nucleic acid molecule, the nucleic acid construct, the vector, the GAA polypeptide or the cell of the invention for use as a medicament.

The invention also relates to the nucleic acid molecule, the nucleic acid construct, the vector, the GAA polypeptide or the cell of the invention, for use in a method for treating a disease caused by a mutation in the GAA gene, in particular in a method for treating Pompe disease. The invention further relates to the nucleic acid molecule, the nucleic acid construct, the vector, the GAA polypeptide or the cell of the invention, for use in a method for treating a glycogen storage disease such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII. The truncated GAA polypeptide of the invention may be administered to a patient in need thereof, for use in enzyme replacement therapy (ERT), such as for use in enzyme replacement therapy a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII.

The invention further relates to the use of the nucleic acid molecule, the nucleic acid construct, the vector, the GAA polypeptide or the cell of the invention, in the manufacture of a medicament useful for treating a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII.

### EXAMPLES

The invention is further described in detail by reference to the following experimental examples and the attached figures. These examples are provided for purposes of illustration only, and are not intended to be limiting.

### MATERIAL AND METHODS

### GAA activity

GAA activity was measured following homogenization of frozen tissue samples in distilled water. 50-100 mg of tissue were weighed and homogenized, then centrifuged for 20 minutes at 10000 x g. The reaction was set up with 10 µl of supernatant and 20 µl of substrate - 4MUα-D-glucoside, in a 96 wells plate. The reaction mixture was incubated at 37°C for one hour, and then stopped by adding 150 µl of Sodium Carbonate buffer pH 10.5. A standard curve (0-2500 pmol/µl of 4MU) was used to measure released fluorescent 4MU from individual reaction mixture, using the EnSpire alpha plate reader (Perkin-Elmer) at 449 nm (Emission) and 360 nm (Excitation). The protein concentration of the clarified supernatant was quantified by BCA (Thermo Fisher Scientific). To calculate the GAA activity, released 4MU concentration was divided by the sample protein concentration and activity was reported as nmol/hour/mg protein.

### Mouse studies

Gaa -/- mouse was generated by targeted disruption of exon 6 and is maintained on the C57BL/6J/129X1/SvJ background (Raben N. et al 1998). Vectors were delivered via the tail vein in a volume of 0.2 ml. Serum samples were collected monthly to monitor levels of secreted hGAA. PBS-injected affected animals and wild type littermates were used as controls.

### NHP study

Male Cynomolgus macaques were housed in stainless steel cages and maintained on a 12-hour light/dark cycle. All macaques had neutralizing antibody titers of <1:5 before the start of the study. A dose of 2E12 vg/kg of AAV8-hAAT-sp7-Δ8-hGAAco1 was infused via the saphenous vein. Blood samples were taken 12 days before and 30 days after the injection via the femoral vein. Whole blood was collected in EDTA containing tubes and centrifuged to separate serum. Three months after vector administration all macaques were euthanized. The animals were first anesthetized with a mixture of ketamine/dexmedetomidine and then euthanized using sodium pentobarbital injected IV. Tissues were immediately collected and frozen in liquid nitrogen.

### Western blot analysis

Total homogenates were obtained from frozen muscles. Protein concentration was determined in the extracts by Pierce BCA Protein Assay (Thermo Fisher Scientific), following manufacturer's instructions. Western blot was performed with an anti hGAA antibody (Abcam). Anti-tubulin antibody (Sigma Aldrich) was used as loading controls.

### RESULTS

With the aim of designing new forms of GAA with improved secretion and reduced immunogenicity, we decided to produce truncated forms of GAA, optionally combining them with alternative signal peptides.

The human GAA shown in SEQ ID NO:2 served has the basis for designing these new forms. SEQ ID NO:1 corresponds to the sequence of SEQ ID NO:2, devoid of the corresponding natural signal peptide of GAA (amino acids 1-27 of SEQ ID NO:2). Nucleic acid constructs were designed to encode GAA polypeptides derived from SEQ ID NO:1 truncated at its N-terminal end. We started by designing a nucleic acid sequence based on the wild-type hGAA coding sequence (SEQ ID NO:9, corresponding to nucleotides 82-2859 of SEQ ID NO:8 that is the wild-type hGAA coding sequence including the signal peptide coding sequence) deleted for the codons corresponding to the first 8 amino acids of SEQ ID NO:1 (Δ8). In addition to the wild-type hGAA coding sequence, we designed optimized nucleic acid sequence encoding the Δ8 truncated hGAA polypeptide (SEQ ID NO:10 and SEQ ID NO:11 corresponds to the hGAAco1 and hGAAco2 optimized coding sequence, respectively), to exclude a possible sequence-specific effect.

**Table 1. Description of the optimized sequences. Table illustrating the characteristics of the two hGAA optimized sequences compared to the wild-type one. a) codon adaptation index and b) GC content calculated using a rare codon analysis tool (http://www.genscript.com). c) and d) are respectively the alternative open reading frames calculated on the 5' to 3' (aORF 5'→3')and 3' to 5' (aORF 3'→5')strands. e) and f) are respectively the acceptor (SA) and donor (SD) splicing sites calculated using a splicing site online prediction tool (http://www.fruitfly.org/seq_tools/splice.html). g) and h) are respectively the percentual identity calculated versus wild-type (wt) and optimized col sequence. i) CpG islands calculated using MethDB online tool (http://www.methdb.de/links.html). CpG islands are sequences longer than 100 bp, with GC content>60% and an observed/expected ratio>0.6.**

| sequence | WT | co1 | co2 |
|---|---|---|---|
| CAI^{a} | 0.84 | 0.94 | 0.77 |
| GC content^{b} | 64.7 | 61.9 | 54.4 |
| aORF 5'-3'^{c} | 2 | 3 | 0 |
| aORF 3'→5^{'d} | 5 | 4 | 0 |
| SA^{e} | 3 | 0 | 1 |
| SD^{f} | 3 | 0 | 0 |
| % identity vs wt^{g} | | 83.1 | 77.7 |
| % identity vs co1^{h} | | | 80.8 |
| CpG islandsⁱ | 4 | 5 | 1 |

Amino acids 1-27 of the hGAAs sequences (corresponding to the natural signal peptide of hGAA, here defined as sp1; whose sequence is shown in SEQ ID NO:4) have been replaced by amino acids 1-24 of the sequence of the human alpha-1-antitrypsin (NP_000286.3) here defined as sp2 (sequence shown in SEQ ID NO:5). We transfected truncated hGAA coding constructs in parallel with their full-size versions in human hepatoma cells (Huh-7) and we measured the quantity of hGAA released in the medium 48 hours after (figure 1A). The Δ8 deletion of hGAAs sequences led to a significant, 50% increase in the secretion level both for wild-type (hGAA) and codon optimized (hGAAco2) sequences. The same truncation performed on a different codon optimized sequence (hGAAco1) also improved the secretion of hGAA to the same extent.
To confirm that a change in the sequence following signal peptide may improve the secretion of hGAA, we further truncated the hGAA polypeptide. We eliminated the codons corresponding to the first 42 amino acids of hGAA from the hGAAco1 construct (Δ42) and we replaced them with a signal peptide derived from chymotrypsinogen B1 (sp7; sequence shown in SEQ ID NO:3). We then compared the efficacy of secretion obtained with this new deleted construct with its Δ8 version fused with sp7 signal peptide and the full size hGAAco1 with sp1 or with sp7. We transfected those constructs in Huh-7 cells and we measured the activity of hGAA in the medium 48 hours after. As expected, we could measure hGAA activity after the transfection of a full size hGAAco1 (p = 0.055 vs GFP) and its secretion is two-fold increased by substituting the wild-type signal peptide with the sp7 (p = 0.006 vs hGAAco1). Surprisingly, both the Δ8 and the Δ42 hGAA sequences fused with the sp7 signal peptide shown a two-fold increase in the secreted hGAA compared to the full-size sequence (p = 0.0002 and 0.0003 respectively vs sp7-hGAAco1, Figure 1B).
Taken together, these data demonstrate that the truncation of hGAA sequence coupled with an efficient signal peptide is able to increase the secretion of the protein in vitro. Additionally, the truncation has one important advantage compared to the mutagenesis of the native sequence as it does not create major neo-antigens, which is an advantage in the engineering of a therapeutic product.
We then verified those findings in vivo, in a Pompe disease mouse model. We injected GAA -/- mice (Raben et al J. Bio. Chem. 1998) with AAV8 vectors expressing hGAAco1 full size, Δ8, or Δ42 fused with sp7 signal peptide under the transcriptional control of a highly potent liver specific promoter derived from the fusion of the apolipoprotein B enhancer and the human alpha-1-antitrypsin promoter (hAAT). One month after the injection of 2E12 vg/kg of the vectors described above, mice were bled and the activity of hGAA was measured in serum. The treatment of mice with vectors expressing the full-length hGAAco1 fused with sp7 shown an increased level of hGAA in the bloodstream (p = 0.115 vs PBS). Surprisingly, both the truncated hGAA, Δ8 and Δ42, led to a significant increase in the level of hGAA in serum (p = 0.014 and 0.013 respectively).

These data indicate that the deletion of the first amino acids of the hGAA lead to a significant improvement in the level of hGAA secreted in the bloodstream.

Furthermore, another signal peptide was fused to the Δ8 truncated form of hGAA, corresponding to amino acids 1-25 from iduronate-2-sulphatase (sp6; SEQ ID NO:6). We transfected hepatoma cells (Huh-7) with plasmids expressing GFP or wild-type hGAA (hGAA; parent polypeptide corresponding to amino acid residues 28-952 of SEQ ID NO:30) in parallel with plasmids expressing optimized hGAA (hGAAco1) fused with sp1, sp2, sp6, sp7 or sp8. 48 hours after transfection the growth medium has been analyzed for the presence of hGAA. Notably these constructs led to the secretion of hGAA levels significantly higher than what observed in the negative control represented by GFP-transfected cells (Figure 3).

We then evaluated glycogen content in heart, diaphragm and quadriceps of GAA -/- mice treated as described above with a Δ8-hGAA. Notably, we observed high levels of hGAA in the tissues after treatment with Δ8-hGAAco expressing vectors (data not shown) that correlated with a significant reduction in glycogen content in all the tissues considered (figure 4B-D). In particular, in the heart (figure 4B) the level of glycogen measured after treatment with vectors bearing the high efficient signal peptides sp7 and 8 were undistinguishable from those observed in non-affected animals (p = 0.983 and 0.996 vs WT respectively). Importantly the level observed after treatment with both the sp7 and sp8 vectors were significantly reduced compared to GAA -/- animals PBS-injected or treated with hGAAco expressing vector fused with sp1 signal peptide.

We also tested if the liver transduction with our vectors induced a humoral response against the transgene. Mice were injected intravenously with AAV8 vectors expressing Δ8-hGAAco or Δ8-hGAAco fused with sp2 or sp7, under the transcriptional control of a liver specific promoter. The results are presented in Figure 5. Mice injected intramuscularly with an AAV9 expressing Δ8-hGAAco under the transcriptional control of a constitutive promoter showed very high level of total IgG, whereas in general vector expressing the same protein in the liver showed lower level of humoral response. Interestingly, three out of five mice injected with sp1 hGAAco (non-truncated) expressing vector showed detectable level of antibodies, whereas just one out of ten mice injected with the high secreted vectors has detectable IgG levels. These data indicate that the expression of a transgene in the liver is fundamental for the induction of peripheral tolerance, also they provide indications that high circulating levels of a hGAA, achieved by the fusion with an efficient signal peptide induce a reduction in the humoral response against the protein itself.

The best performing vector selected in the mouse study was injected in two non-human primates (NHP, Macaca Fascicularis sp.) to verify the efficacy of secretion of our vector and the uptake in muscles. We injected two monkeys with 2E12 vg/kg of AAV8-hAAT-sp7-Δ8-hGAAco1. One month after the injection we measured the levels of hGAA in the serum of the two animals by western blot using a specific anti-hGAA antibody. We observed a clear band with a size compatible with that of hGAA in the two monkeys. This band was not present in serum samples obtained 12 days before vector injection, thus confirming the specificity of our detection method (Figure 6A). Three months after the injection we sacrificed the animals and we obtained tissues to verify if hGAA secreted from the liver in the bloodstream were efficiently taken up by muscle. We performed a western blot using an antibody specific for hGAA on total lysates obtained from biceps and diaphragm of the two monkeys. Interestingly we were able to observe a clear band in animal number 2 which also showed the highest levels of hGAA in the bloodstream (Figure 6B). Also, in animal number 1 we could observe a fainter band with a molecular weight consistent with that of hGAA in both muscles analyzed. These data indicate that the AAV8-hAAT-sp7-Δ8-hGAAco1 vector efficiently transduces liver in NHP. They also demonstrate that the protein secreted in the bloodstream is efficiently taken up in muscle and that this uptake is correlated with the level of hGAA measured in blood.

## Claims

1. A truncated GAA polypeptide, comprising a deletion of at least one amino acid from the N-terminal end of a parent GAA polypeptide, wherein the parent polypeptide corresponds to a precursor form of a GAA polypeptide devoid of its signal peptide.

2. The truncated GAA polypeptide of claim 1, wherein said truncated GAA polypeptide has 1 to 75 consecutive amino acids deleted at its N-terminal end as compared to the parent GAA polypeptide, in particular 6, 7, 8, 9, 10, 40, 41, 42, 43 or 44 consecutive amino acids deleted at its N-terminal end as compared to a parent GAA polypeptide, even more particularly 8 or 42 consecutive amino acids truncated at its N-terminal end as compared to a parent GAA polypeptide.

3. The truncated GAA polypeptide of claim 1 or 2, wherein the parent polypeptide is a human GAA (hGAA), in particular a hGAA having the amino acid sequence shown in SEQ ID NO:1 or in SEQ ID NO:33, in particular in SEQ ID NO:1.

4. The truncated GAA polypeptide of any one of claims 1 to 3, wherein the truncated GAA polypeptide has the amino acid sequence shown in SEQ ID NO:27 or SEQ ID NO:28.

5. The truncated GAA polypeptide of any one of claims 1 to 4, further comprising a signal peptide fused to its N-terminal end, in particular a signal peptide selected in the group consisting of SEQ ID NO:3 to 7, in particular the signal peptide of SEQ ID NO:3.

6. A nucleic acid molecule encoding a truncated GAA polypeptide of any one of claims 1 to 5, said nucleic acid molecule being in particular a nucleotide sequence optimized to improve the expression of and/or improve immune tolerance to the truncated GAA polypeptide *in vivo,* such as the nucleic acid sequence shown in SEQ ID NO:12 or SEQ ID NO:13.

7. A nucleic acid construct, comprising the nucleic acid molecule of claim 6, comprising said nucleic acid molecule operably linked to a promoter, in particular a promoter that is a liver-specific promoter preferably selected in the group consisting of the alpha-1 antitrypsin promoter (hAAT), the transthyretin promoter, the albumin promoter and the thyroxine-binding globulin (TBG) promoter, wherein said nucleic acid construct further optionally comprises an intron, in particular an intron selected in the group consisting of a human beta globin b2 (or HBB2) intron, a FIX intron, a chicken beta-globin intron, and a SV40 intron, wherein said intron is optionally a modified intron such as a modified HBB2 intron of SEQ ID NO:17, a modified FIX intron of SEQ ID NO:19, or a modified chicken beta-globin intron of SEQ ID NO:21.

8. The nucleic acid construct of claim 7, comprising, preferably in this order,: an enhancer; an intron; a promoter, in particular a liver-specific promoter; the nucleic acid sequence encoding the GAA protein; and a polyadenylation signal, the construct comprising preferably, in this order: an ApoE control region; a HBB2 intron, in particular a modified HBB2 intron; a hAAT promoter; the nucleic acid sequence encoding the truncated GAA polypeptide; and a bovine growth hormone polyadenylation signal, said nucleic acid construct more particularly comprising the nucleotide sequence of any one of SEQ ID NO:22 to 26.

9. A vector comprising the nucleic acid molecule or the nucleic acid construct according to any one of claims 6 to 8, which is a viral vector, preferably a retroviral vector, such as a lentiviral vector, or an AAV vector.

10. The vector according to claim 9, which is a single-stranded or double-stranded self-complementary AAV vector, preferably an AAV vector with an AAV-derived capsid, such as an AAV1, AAV2, variant AAV2, AAV3, variant AAV3, AAV3B, variant AAV3B, AAV4, AAV5, AAV6, varient AAV6, AAV7, AAV8, AAV9, AAV10 such as AAVcy10 and AAVrh10, AAVrh74, AAVdj, AAV-Anc80, AAV-LK03, AAV2i8, and porcine AAV, such as AAVpo4 and AAVpo6 capsid or with a chimeric capsid.

11. The vector according to claim 10, wherein the AAV vector has an AAV8, AAV9, AAVrh74 or AAV2i8 capsid, in particular an AAV8, AAV9 or AAVrh74 capsid, more particularly an AAV8 capsid.

12. A cell transformed with the nucleic acid molecule of claim 6, the nucleic acid construct of any one of claims 7 to 8 or the vector of any one of claims 9 to 11, wherein the cell is in particular a liver cell or a muscle cell.

13. A pharmaceutical composition, comprising, in a pharmaceutically acceptable carrier, the truncated GAA polypeptide of any one of claims 1 to 5, the nucleic acid molecule of claim 6, the nucleic acid construct of any one of claims 7 to 8, the vector of any one of claims 9 to 11, or the cell according to claim 12.

14. The truncated GAA polypeptide of any one of claims 1 to 5, the nucleic acid molecule of claim 6, the nucleic acid construct of any one of claims 7 to 8, the vector of any one of claims 9 to 11, or the cell according to claim 12, for use as a medicament.

15. The truncated GAA polypeptide of any one of claims 1 to 5, the nucleic acid molecule of claim 6, the nucleic acid construct of any one of claims 7 to 8, the vector of any one of claims 9 to 11, or the cell according to claim 12, for use in a method for treating a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII.
